# EUROPEAN PATENT SPECIFICATION

(11) **EP 3 362 564 B1**
(45) Date of publication and mention of the grant of the patent: **01.07.2020**
(21) Application number: 16788347.9
(22) Date of filing: 14.10.2016
(51) Int. Cl.: C12N 15/113

(54) **NUCLEIC ACID BASED TIA-1 INHIBITORS**
NUKLEINSÄUREBASIERTE TIA-1-INHIBITOREN
INHIBITEURS DE TIA-1 À BASE D'ACIDE NUCLÉIQUE

(30) Priority: 14.10.2015 US 201562241535 P; 04.05.2016 US 201662331795 P
(43) Date of publication of application: 22.08.2018
(73) Proprietor: Aquinnah Pharmaceuticals, Inc., Cambridge, MA 02139 (US); Trustees of Boston University, Boston, MA 02215 (US); Larsen, Glenn R., Sudbury, MA 01776 (US); Wolozin, Benjamin, Newton, MA 02459 (US); Vanderweyde, Tara, Newton, MA 02460 (US); Apicco, Daniel J., Westford, MA 01886 (US)
(72) Inventor: LARSEN, Glenn, R., Sudbury, MA 01776 (US); WOLOZIN, Benjamin, Newton, MA 02459 (US); VANDERWEYDE, Tara, Newton, MA 02460 (US); APICCO, Daniel, J., Westford, MA 01886 (US)
(74) Representative: Cornish, Kristina Victoria Joy
(86) International application number: PCT/US2016/057164
(87) International publication number: WO 2017/066657

(56) References cited:
- WO-A1-95/09924
- WO-A1-2013/000064
- WO-A2-2004/045543
- WO-A2-2004/048511
- WO-A2-2011/005786
- A. ALBORNOZ ET AL: "The Stress Granule Component TIA-1 Binds Tick-Borne Encephalitis Virus RNA and Is Recruited to Perinuclear Sites of Viral Replication To Inhibit Viral Translation", JOURNAL OF VIROLOGY., vol. 88, no. 12, 15 June 2014 (2014-06-15) , pages 6611-6622, XP055335018, US ISSN: 0022-538X, DOI: 10.1128/JVI.03736-13
- O. APARICIO ET AL: "Adenovirus VA RNA-derived miRNAs target cellular genes involved in cell growth, gene expression and DNA repair", NUCLEIC ACIDS RESEARCH, vol. 38, no. 3, 1 January 2010 (2010-01-01), pages 750-763, XP055335191, ISSN: 0305-1048, DOI: 10.1093/nar/gkp1028
- BRUNELLO, C.A. ET AL.: "Abstract n°: ADPD5-0748 - Regulation of tau localization to stress granules.", NEURODEGENERATIVE DISEASES, vol. 15, no. Suppl.1, 19 March 2015 (2015-03-19), page 1508, XP002765903, 12th International Conference AD/PDTM Nice, France, March 18-22, 2015 ISSN: 1660-2854, DOI: 10.1159/000381736
- VANDERWEYDE TARA ET AL: "Role of stress granules and RNA-binding proteins in neurodegeneration: a mini-review.", GERONTOLOGY 2013, vol. 59, no. 6, 2013, pages 524-533, XP002765904, ISSN: 1423-0003
- TARA VANDERWEYDE ET AL: "Interaction of tau with the RNA-Binding Protein TIA1 Regulates tau Pathophysiology and Toxicity", CELL REPORTS, vol. 15, no. 7, 1 May 2016 (2016-05-01), pages 1455-1466, XP055335025, US ISSN: 2211-1247, DOI: 10.1016/j.celrep.2016.04.045

### SEQUENCE LISTING

<110> AQUINNAH PHARMACEUTICALS, INC.
   TRUSTEES OF BOSTON UNIVERSITY
<120> NUCLEIC ACID BASED TIA-1 INHIBITORS
<130> A2137-7013WO
<140>
   <141>
<150> 62/331,795
   <151> 2016-05-04
<150> 62/241,535
   <151> 2015-10-14
<160> 10
<170> PatentIn version 3.5
<210> 1
   <211> 4620
   <212> DNA
   <213> Homo sapiens
<400> 1
   gctcctaggc tcccggctcg ccgccatctt gtattggggt ttcattgttc ccgctgggcc      60
   gggcggttta gtgtaattgc cgccggagga ggaggcggag taacctctgg tcagccgaga     120
   aaccccacta tcctgtagcc ataaccgctt aaacgatttg ggaggtagtg aagggcaggg     180
   agctggacct ggaggcgccg ccgcgacagc agcagccatg gaggacgaga tgcccaagac     240
   tctatacgtc ggtaaccttt ccagagatgt gacagaagct ctaattctgc aactctttag     300
   ccagattgga ccttgtaaaa actgcaaaat gattatggat acagctggaa atgatcccta     360
   ttgttttgtg gagtttcatg agcatcgtca tgcagctgca gcattagctg ctatgaatgg     420
   acggaagata atgggtaagg aagtcaaagt gaattgggca acaaccccta gcagtcaaaa     480
   gaaagataca agcaatcatt tccatgtctt tgttggtgat ctcagcccag aaattacaac     540
   tgaagatata aaagctgctt ttgcaccatt tggaagaata tcagatgccc gagtggtaaa     600
   agacatggca acaggaaagt ctaagggata tggctttgtc tcctttttca acaaatggga     660
   tgctgaaaac gccattcaac agatgggtgg ccagtggctt ggtggaagac aaatcagaac     720
   taactgggca acccgaaagc ctcccgctcc aaagagtaca tatgagtcaa ataccaaaca     780
   gctatcatat gatgaggttg taaatcagtc tagtccaagc aactgtactg tatactgtgg     840
   aggtgttact tctgggctaa cagaacaact aatgcgtcag actttttcac catttggaca     900
   aataatggaa attcgagtct ttccagataa aggatattca tttgttcggt tcaattccca     960
   tgaaagtgca gcacatgcaa ttgtttctgt taatggtact accattgaag gtcatgttgt    1020
   gaaatgctat tggggcaaag aaactcttga tatgataaat cccgtgcaac agcagaatca    1080
   aattggatat ccccaacctt atggccagtg gggccagtgg tatggaaatg cacaacaaat 1140
   tggccagtat atgcctaatg gttggcaagt tcctgcatat ggaatgtatg gccaggcatg 1200
   gaaccagcaa ggatttaatc agacacagtc ttctgcacca tggatgggac caaattatgg 1260
   agtgcaaccg cctcaagggc aaaatggcag catgttgccc aatcagcctt ctgggtatcg 1320
   agtggcaggg tatgaaaccc agtgaataag gactccagaa tctaaagcca gtggcttgag 1380
   gctacaggga gtgtagtaaa gccgttgttt acttaaagat ttatcaaatc agtcagtgca 1440
   aatgtcagat acaatgtatt tatttaaaag attcattttt aatcatgaaa ttacttatca 1500
   tccacattgt tttaaaaaga aacaagatgc tggatgtctg ccaatttttg ccttcattac 1560
   cttttttgat aaagtttctc agatccttgt ttcaaacaca aatgcaggga ttgctgccac 1620
   tttttaacta ttaagaggca gaaaattgca caatattgaa cttttttcca ctgaagtagt 1680
   gtgcagttct agtttgcatt cctgatatga tttaaaacat gtaatataaa gatgttaaaa 1740
   aaaaaaacca aaactgtgca gagtctagaa gttgtttgtc atcttcagct tgtgcacaat 1800
   tctgttttag gttaaaaaaa ggcattgttt gagctgtccc atctccactg ttatcccttt 1860
   ggggtttttt aatataaatt attagtttac atcatttttg tatctacatc ttttttcaca 1920
   aatttgtctt gccttattaa agttctgtaa aatatactta aatggaaaaa atgatgttca 1980
   tttagattga aaacttttct cagatggatt gataattgca ttcatcttgt gttttatatg 2040
   agaaggtgcc tcaagaattt cctgttggat ttgtttaaaa ggatttttat ctttcgtgat 2100
   aaactttgct gtgtaccagg aactataaaa acaaaaactt gttactaaag aaaatatctg 2160
   aaatgtgata agttcttatg ccatgttaat ttcatgtgtc aacttcaaca tttacatgta 2220
   ttatttcatt atgtaaaatg ttttagcaat ttaatatttt gcacagttag caaactttgt 2280
   atgtcatttc cttcaaggca tcatgcagag ttgacatgag atttataagg ttttaagttg 2340
   tttgcatgtg aaaatcaaat acatactttg gtagtctttg aatacaaagt catctgctct 2400
   tgtttttcaa gaattttgag acacaaagtt gtatgtaaag gaatatatta atttgccgtt 2460
   ttctaggtag atttgctcaa aaagagtgaa tcaacttaat atgtacaaat gatagctgtg 2520
   aaactgtaga atatctttgt gtcaggcttg gagttcattg tgacctccaa attttgcctg 2580
   aaggaccagc tgggcaaagc atcttttaaa tgttcagagg ccaaaagata aacaaaaaaa 2640
   aaaccttaaa atcctacctc tttaaacagc cttcagataa gagaatcctc agtgcaatca 2700
   ttattttgat tcgtttggta cctgttttcc tggagttccc gattttatta ttttggggtg 2760
   gctccaagca ttaagaggtt taatctttga tggcattgtt ctagttttga aatttctagt 2820
   atatttcaga gtctcttaga agacttgtgt gggaagtttc actttgtttt cagtgaagat 2880
   cacaaacctc cttcttcctt tactcaagag gaaaggtccc agtatacata tttgaatggt 2940
   tgatggtttt caagaccttc agggagctcc ctgcatttta cctagaaaca gaaaaggccc     3000
   gcaaaatctt aagtttcctg gcctgcattt cccgggtagg ggcaaatgac tccaagctgg     3060
   tctctaagcc aataccctta taaaccagag cccaggaaag acagctcgag tgtataattc     3120
   tctggagctc aattctatgc agttgtgctg atatttcatt aagtcactgt gtatttttaa     3180
   gtgttgatac attaaaagtc gctttatgga agatgagtaa attttttaaa tacttggaaa     3240
   ttttatttcc ttgttaactt ctacagatca gggcatgcaa ccaaaagcag cttaaatgaa     3300
   atattttaaa ataaaatatc aggaagctat ttttagattt cttctggctt atgtttctac     3360
   tttaggaccc tcattgttct cttattaaaa aaaattattt cctgtgcatc tcatggactg     3420
   cagggtaaat tatttgggca taaataattt aaatagtttt ctttcatttt gactatctcc     3480
   agtaataaca gtttttatta tccagcatat tggcttattg cacaaatctt aaaatgtaca     3540
   ttgactactt tctgagaaga aagtggtatc agtactcatg atgaaaaggt tactactgaa     3600
   caaattcaca tttcaggaac acctctatct ttggtttaaa tcttactctt agtttttccg     3660
   tctaaaaatc atactggtat tagtatcagg taaggaaatt aaagttttta aaatggtttc     3720
   attctctgca atatgcaaaa tttagatttt actttctggt actgtaaaga acctgaagtg     3780
   atttacactt aatgggtgat taatccagta ttctttaccc tgaatgtttg gatattaaag     3840
   ttcctttatg ttttctataa cctgtgggat cttcttgcag tgattattgt gtgtgagatt     3900
   tttttctttt ttggtctatc catattgtta tattcactca ggtatttttt ttttaatctt     3960
   attccagaat cagtggttta tattgggtta ctgtttaaca ccaaatggaa ttggcattct     4020
   gcagatttaa ttaattatga aaccagggtc tcattttcct tgctgatact tgttgaaaat     4080
   gagattcaca ttctagtctt tattttcctc ctgttttgtc cctgtgcttg tacatcttcc     4140
   ttttatttgt gtgttatagt tctattccat ttgagaaggc agttggtaag aactagattg     4200
   catgtacaaa gacaggttta ctaagtgctg tacagtggtc ctgaggttac agttgaatta     4260
   gaaaaacgaa atgtacttac aggaaataag aaagcaaacc tttcaaatga gagtgatgat     4320
   ttctttaaaa aaaatcagtt tttttctctc aaataatgtt ctttatttca cgaaatcgtc     4380
   aatcttaagc atgagcaggg ataaacaact cctagaagga actcaattca ttcttcctgg     4440
   attttctctg ttgttaaatc acaaaaatga tagtccccaa tcgtttcttt ataggaggtt     4500
   attacatttc attacagtca ctgcattttg actgttgtgt ttagaatttg aatgtacatc     4560
   caaaatgatg agtttcaatt taagagcctt aataaaatgt gtgagtgtgt ctcaattgaa     4620
<210> 2
   <211> 20
   <212> DNA
   <213> Homo sapiens
<400> 2
   attggcagac atccagcatc 20
<210> 3
   <211> 20
   <212> DNA
   <213> Homo sapiens
<400> 3
   gatgctggat gtctgccaat 20
<210> 4
   <211> 58
   <212> DNA
   <213> Homo sapiens
<400> 4
   ccggcgatgc tggatgtctg ccaatctcga gattggcaga catccagcat cgtttttg 58
<210> 5
   <211> 386
   <212> PRT
   <213> Homo sapiens
<400> 5
<210> 6
   <211> 375
   <212> PRT
   <213> Homo sapiens
<400> 6
   Met Glu Asp Glu Met Pro Lys Thr Leu Tyr Val Gly Asn Leu Ser Arg
   1               5                   10                  15
   Asp Val Thr Glu Ala Leu Ile Leu Gln Leu Phe Ser Gln Ile Gly Pro
               20                  25                  30
   Cys Lys Asn Cys Lys Met Ile Met Asp Thr Ala Gly Asn Asp Pro Tyr
           35                  40                  45
   Cys Phe Val Glu Phe His Glu His Arg His Ala Ala Ala Ala Leu Ala
       50                  55                  60
   Ala Met Asn Gly Arg Lys Ile Met Gly Lys Glu Val Lys Val Asn Trp
   65                  70                  75                  80
   Ala Thr Thr Pro Ser Ser Gln Lys Lys Asp Thr Ser Asn His Phe His
                   85                  90                  95
   Val Phe Val Gly Asp Leu Ser Pro Glu Ile Thr Thr Glu Asp Ile Lys
               100                 105                 110
   Ala Ala Phe Ala Pro Phe Gly Arg Ile Ser Asp Ala Arg Val Val Lys
           115                 120                 125
   Asp Met Ala Thr Gly Lys Ser Lys Gly Tyr Gly Phe Val Ser Phe Phe
       130                 135                 140
   Asn Lys Trp Asp Ala Glu Asn Ala Ile Gln Gln Met Gly Gly Gln Trp
   145                 150                 155                 160
   Leu Gly Gly Arg Gln Ile Arg Thr Asn Trp Ala Thr Arg Lys Pro Pro
                   165                 170                 175
   Ala Pro Lys Ser Thr Tyr Glu Ser Asn Thr Lys Gln Leu Ser Tyr Asp
               180                 185                 190
   Glu Val Val Asn Gln Ser Ser Pro Ser Asn Cys Thr Val Tyr Cys Gly
           195                 200                 205
   Gly Val Thr Ser Gly Leu Thr Glu Gln Leu Met Arg Gln Thr Phe Ser
       210                 215                 220
   Pro Phe Gly Gln Ile Met Glu Ile Arg Val Phe Pro Asp Lys Gly Tyr
   225                 230                 235                 240
   Ser Phe Val Arg Phe Asn Ser His Glu Ser Ala Ala His Ala Ile Val
                   245                 250                 255
   Ser Val Asn Gly Thr Thr Ile Glu Gly His Val Val Lys Cys Tyr Trp
               260                 265                 270
   Gly Lys Glu Thr Leu Asp Met Ile Asn Pro Val Gln Gln Gln Asn Gln
           275                 280                 285
   Ile Gly Tyr Pro Gln Pro Tyr Gly Gln Trp Gly Gln Trp Tyr Gly Asn
       290                 295                 300
   Ala Gln Gln Ile Gly Gln Tyr Met Pro Asn Gly Trp Gln Val Pro Ala
   305                 310                 315                 320
   Tyr Gly Met Tyr Gly Gln Ala Trp Asn Gln Gln Gly Phe Asn Gln Thr
                   325                 330                 335
   Gln Ser Ser Ala Pro Trp Met Gly Pro Asn Tyr Gly Val Gln Pro Pro
               340                 345                 350
   Gln Gly Gln Asn Gly Ser Met Leu Pro Asn Gln Pro Ser Gly Tyr Arg
           355                 360                 365
   Val Ala Gly Tyr Glu Thr Gln
       370                 375
<210> 7
   <211> 214
   <212> PRT
   <213> Homo sapiens
<400> 7
   Met Glu Asp Glu Met Pro Lys Thr Leu Tyr Val Gly Asn Leu Ser Arg
   1               5                   10                  15
   Asp Val Thr Glu Ala Leu Ile Leu Gln Leu Phe Ser Gln Ile Gly Pro
               20                  25                  30
   Cys Lys Asn Cys Lys Met Ile Met Asp Thr Ala Gly Asn Asp Pro Tyr
           35                  40                  45
   Cys Phe Val Glu Phe His Glu His Arg His Ala Ala Ala Ala Leu Ala
       50                  55                  60
   Ala Met Asn Gly Arg Lys Ile Met Gly Lys Glu Val Lys Val Asn Trp
   65                  70                  75                  80
   Ala Thr Thr Pro Ser Ser Gln Lys Lys Asp Thr Ser Ser Ser Thr Val
                   85                  90                  95
   Val Ser Thr Gln Arg Ser Gln Asp His Phe His Val Phe Val Gly Asp
               100                 105                 110
   Leu Ser Pro Glu Ile Thr Thr Glu Asp Ile Lys Ala Ala Phe Ala Pro
           115                 120                 125
   Phe Gly Arg Ile Ser Asp Ala Arg Val Val Lys Asp Met Ala Thr Gly
       130                 135                 140
   Lys Ser Lys Gly Tyr Gly Phe Val Ser Phe Phe Asn Lys Trp Asp Ala
   145                 150                 155                 160
   Glu Asn Ala Ile Gln Gln Met Gly Gly Gln Trp Leu Gly Gly Arg Gln
                   165                 170                 175
   Ile Arg Thr Asn Trp Ala Thr Arg Lys Pro Pro Ala Pro Lys Ser Thr
               180                 185                 190
   Tyr Glu Cys Arg Cys Ile Gly Glu Glu Lys Glu Met Trp Asn Phe Gly
           195                 200                 205
   Glu Lys Tyr Ala Arg Phe
       210
<210> 8
   <211> 58
   <212> DNA
   <213> Homo sapiens
<400> 8
   ccggcgatgg tggatgtttg ccaatctcga gattggcaaa catccaccat cgtttttg 58
<210> 9
   <211> 20
   <212> DNA
   <213> Homo sapiens
<400> 9
   attggcaaac atccaccatc 20
<210> 10
   <211> 20
   <212> DNA
   <213> Homo sapiens
<400> 10
   gatggtggat gtttgccaat 20

## Description

This application claims priority to U.S. Serial No. 62/241,535 filed October 14, 2015 and U.S. Serial No. 62/331,795 filed May 4, 2016.

### SEQUENCE LISTING

The instant application contains a Sequence Listing which has been submitted electronically in ASCII format and is hereby incorporated by reference in its entirety. Said ASCII copy, created on October 11, 2016, is named A2137-7013WO_SL.txt and is 15,743 bytes in size.

### STATEMENT REGARDING FEDERALLY SPONSORED RESEARCH OR DEVELOPMENT

This invention was made with government support under grant numbers F31AG042213, R01AG050471, R01NS089544, and 1R43NS095481-01 awarded by the National Institutes of Health. The government has certain rights in the invention.

### BACKGROUND

One of the hallmarks of many neurodegenerative diseases is the accumulation of protein inclusions in the brain and central nervous system. These inclusions are insoluble aggregates of proteins and other cellular components that cause damage to cells and result in impaired function. Proteins such as tau, α-synuclein, huntingtin and β-amyloid have all been found to form inclusions in the brain and are linked to the development of a number of neurodegenerative diseases, including Alzheimer's disease and Huntington's disease. Neurodegenerative diseases are also associated with stress granules, which contain RNAs and aggregated RNA binding proteins.

TIA-1 is an RNA binding protein and a core nucleating stress granule protein. Nucleation is followed by recruitment of secondary RNA-binding proteins to form a mature stress granule, which is a key component of stress-induced translational suppression. TIA1 co-localizes with neuropathology in the brain tissue of subjects with neurodegenerative disorders.

There is a need in the art for compositions and methods that can disaggregate stress granules or inhibit their formation.

### SUMMARY OF THE INVENTION

The invention is as set out in the claims.

The present disclosure provides, among other things, nucleic acid based inhibitors of TIA-1. Neurodegenerative disorders are associated with the formation of stress granules, which can contain TIA-1 and tau. As Example 1 herein shows, in cells prone to formation of stress granules, knockdown of TIA-1 reduces the formation of stress granules. This disclosure provides, e.g., methods of treating neurodegenerative disorders by treating a subject with a nucleic acid based TIA-1 inhibitor.

The present disclosure provides, in certain aspects, a nucleic acid based inhibitor capable of reducing the level or translation of an mRNA encoding TIA-1.

The present disclosure also provides, in certain aspects, a method of treating a subject having a neurodegenerative disorder, comprising administering to the subject a therapeutically effective amount of a nucleic acid based inhibitor, e.g., a nucleic acid based TIA-1 inhibitor described herein, which targets mRNA encoding TIA-1, thereby treating the subject. In a related aspect, the present disclosure provides a nucleic acid based TIA-1 inhibitor described herein, for the manufacture of a medicament for treating a neurodegenerative disorder. In a related aspect, the present disclosure provides a nucleic acid based TIA-1 inhibitor described herein, for treating a neurodegenerative disorder.

The present disclosure also provides, in certain aspects, a method of reducing a number or size of stress granules, inhibiting stress granule formation, or reducing the number of cells that are positive for stress granules (e.g., compared to an untreated sample or subject, or compared to the expected course of disease without treatment), comprising contacting a cell with a nucleic acid based inhibitor, e.g., a nucleic acid based TIA-1 inhibitor described herein, which targets mRNA encoding TIA-1.

The present disclosure also provides, in certain aspects, a method of prolonging cell survival, comprising contacting the cell with a nucleic acid based inhibitor, e.g., a nucleic acid based TIA-1 inhibitor described herein, which targets mRNA encoding TIA-1.

The present disclosure also provides, in certain aspects, a method of inhibiting tau misfolding, comprising contacting a cell having misfolded tau with a nucleic acid based inhibitor, e.g., a nucleic acid based TIA-1 inhibitor described herein, which targets mRNA encoding TIA-1.

The present disclosure also provides, in certain aspects, a kit comprising a nucleic acid based TIA-1 inhibitor described herein, and instructions for using the nucleic acid based inhibitor for treating a neurodegenerative disease.

The present disclosure also provides, in certain aspects, a vector encoding a nucleic acid based TIA-1 inhibitor described herein, and optionally further comprising one or more of (e.g., two or three of) a promoter, selectable marker, polyadenylation site. In embodiments, the vector is a viral vector, e.g., an AAV vector. In embodiments, the vector comprises or encodes a plurality of nucleic acid based inhibitors described herein.

The present disclosure also provides, in certain aspects, a pharmaceutical composition comprising a nucleic acid based TIA-1 inhibitor described herein and one or more pharmaceutically acceptable excipient.

The present disclosure also describes, in certain aspects, a method of producing a nucleic acid based TIA-1 inhibitor described herein, comprising performing solid phase synthesis to polymerize a plurality of nucleotides. The nucleotides may be polymerized in a predefined order, thereby producing the nucleic acid based inhibitor. The present disclosure also provides, in certain aspects, a method of producing a nucleic acid based TIA-1 inhibitor described herein, comprising culturing a cell comprising a vector described herein, under conditions that allow expression of a nucleic acid based TIA-1 inhibitor.

The present disclosure also describes, in certain aspects, a method of screening for modulators of stress granules, comprising contacting a nucleic acid based TIA-1 inhibitor with a cell that develops stress granules, e.g., a cell treated with a physicochemical stressor described herein, wherein decreased formation of stress granules indicates that the TIA-1 inhibitor is an inhibitor of stress granule formation.

In any of the aspects herein, e.g., in any of the nucleic acid based inhibitors, kits, compositions, compositions for use, and methods herein, the nucleic acid based inhibitor may have one or more of the properties below:
In some embodiments, the nucleic acid based inhibitor comprises at least one chemical modification.

In some embodiments, the nucleic acid based inhibitor is capable of hybridizing with a first region of a TIA-1 nucleic acid sequence of SEQ ID NO: 1 that overlaps by at least 1, 2, 3, 4, 5, 6, 7, 8, 9, 10, 11, 12, 13, 14, 15, 16, 17, 18, 19, or 20 nucleotides with a second region comprising nucleotides 1526-1545 of SEQ ID NO: 1. In some embodiments, the nucleic acid based inhibitor is capable of hybridizing with a first region of a TIA-1 nucleic acid sequence of SEQ ID NO: 1 that is within at least 0, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 200, 300, 400, 500, 600, 700, 800, 900, or 1000 nucleotides of a second region comprising nucleotides 1526-1545 of SEQ ID NO: 1. In some embodiments, the nucleic acid based inhibitor is capable of competing for binding to TIA-1 RNA with the shRNA of SEQ ID NO: 4. In some embodiments, the nucleic acid based inhibitor is capable of competing for binding to TIA-1 RNA with a nucleic acid of SEQ ID NO: 2, which is the guide sequence from the hairpin of SEQ ID NO: 4.

In some embodiments, the nucleic acid based inhibitor comprises a sequence of SEQ ID NO: 2, or a sequence having no more than 1, 2, 3, 4, 5 substitutions, insertions, or deletions relative to SEQ ID NO: 2. In some embodiments, the nucleic acid based inhibitor further comprises a sequence capable of hybridizing to SEQ ID NO: 2. In some embodiments, the sequence capable of hybridizing to SEQ ID NO: 2 is a sequence having no more than 1, 2, 3, 4, or 5 substitutions, insertions, or deletions relative to SEQ ID NO: 3. In some embodiments, the nucleic acid based TIA-1 inhibitor comprises a region (e.g., of at least 5, 6, 7, 8, 9, 10, 11, 12, 13, 14, 15, 16, 17, 18, 19, or 20 nucleotides) capable of hybridizing with no mismatches, or with no more than one mismatch, to a human TIA-1 nucleic acid, e.g., the nucleic acid of SEQ ID NO: 1.

In some embodiments, the nucleic acid based inhibitor comprises a sequence of SEQ ID NO: 4, or a sequence having no more than 1, 2, 3, 4, 5, 6, 7, 8, 9, 10 substitutions, insertions, or deletions relative to SEQ ID NO: 4.

In some embodiments, the nucleic acid based inhibitor comprises a double stranded RNA (dsRNA) (e.g., a siRNA or shRNA), an antisense RNA, a microRNA (miRNA), long interfering dsRNA (liRNA), an aptamer, or a ribozyme. In some embodiments, the nucleic acid based inhibitor is not a shRNA. In some embodiments, the nucleic acid based inhibitor comprises a double stranded region. In some embodiments, the double stranded region is about 30 base pairs in length. In embodiments, the double stranded region is about 16, 17, 18, 19, 20, 21, 22, 23, 24, 25, 26, 27, 28, or 29 base pairs in length. In some embodiments, the nucleic acid based inhibitor comprises one or two overhanging ends of about 1 to about 3 (*e.g.,* about 1, 2, or 3) nucleotides. In some embodiments, the nucleic acid based inhibitor comprises one or two blunt ends. In some embodiments, the nucleic acid based inhibitor is a shRNA with a blunt end, a 5' overhang, or a 3' overhang, e.g., of about 1 to about 3 (*e.g.,* about 1, 2, or 3) nucleotides. In some embodiments, the nucleic acid based inhibitor is single stranded.

In some embodiments, the nucleic acid based inhibitor comprises one or more chemical modifications selected from: phosphorothioate internucleotide linkages, 2'-deoxyribonucleotides, 2'-O-methyl ribonucleotides, 2'-O-methoxyethyl ribonucleotides, 2'-deoxy-2'-fluoro ribonucleotides, "universal base" nucleotides, "acyclic" nucleotides, 5-C-methyl nucleotides, and terminal glyceryl and inverted deoxy abasic residues. In some embodiments, the nucleic acid based inhibitor comprises at least 1, 2, 3, 4,5 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 chemically modified nucleotides. In embodiments, every nucleotide of the nucleic acid based inhibitor is a chemically modified nucleotide. In some embodiments, the nucleic acid based inhibitor comprises one or more conjugate moieties. In some embodiments, the conjugate moiety is attached to a nucleic acid via one or more cleavable bonds, e.g., phosphodiester linkage.

In some embodiments, the nucleic acid based inhibitor is capable of promoting cleavage of a target RNA, e.g., a target mRNA. In some embodiments, the nucleic acid based inhibitor is capable of promoting degradation of a target RNA, e.g., a target mRNA.

Any of the methods or compositions for use described herein may involve one or more of the following steps or characteristics.

In some embodiments, the invention further comprises administering a second anti-neurodegenerative agent or therapy to the subject. In some embodiments, e.g., of the compositions for use herein, the patient has been treated, is being treated, or will be treated with a second anti-neurodegenerative agent or therapy.

In some embodiments, the subject is a mammal, e.g., a human. In some embodiments, the neurodegenerative disorder is Alzheimer's disease. In some embodiments, the neurodegenerative disorder is a tauopathy. In some embodiments, the neurodegenerative disorder is a motor neuron disease, e.g., amyotrophic lateral sclerosis (ALS). In some embodiments, the neurodegenerative disease is selected from: frontotemporal dementia (FTD), frontotemporal dementia with parkinsonism (FTDP-17), frontotemporal lobar dementia (FTLD-TDP), Huntington's disease, Creutzfeld-Jacob disease, and spinomuscular atrophy.

In some embodiments, total tau levels do not decrease, e.g., tau levels increase. In some embodiments, the stress granules comprise tau protein, e.g., misfolded tau protein, e.g., tau protein comprising an epitope recognized by the antibody MC1.

In some embodiments, the method comprises decreasing the stability or increasing the solubility of stress granules.

In some embodiments, the cell is a human cell. In some embodiments, the cell is ex vivo or in situ. In some embodiments, the cell is a neuron.

In some embodiments, the tau protein is wild-type or mutant e.g., P301L tau, unphosphorylated, or phosphorylated e.g. at S396, S404 or both S396 and S404.

In some embodiments, a nucleic acid based TIA-1 inhibitor described herein inhibits the formation of a stress granule. The nucleic acid based TIA-1 inhibitor can inhibit the formation of a stress granule by at least 10%, at least 20%, at least 30%, at least 40%, at least 50%, at least 60%, at least 70%, at least 80%, at least 90%, at least 95%, or 100% (i.e., complete inhibition) relative to a control.

In some embodiments, a nucleic acid based TIA-1 inhibitor disaggregates a stress granule. The nucleic acid based TIA-1 inhibitor can disperse or disaggregate a stress granule by at least 10%, at least 20%, at least 30%, at least 40%, at least 50%, at least 60%, at least 70%, at least 80%, at least 90%, at least 95%, or 100% (i.e., complete dispersal) relative to a control.

In some disclosure, the methods further comprise the step of diagnosing the subject with a neurodegenerative disease or disorder, a musculoskeletal disease or disorder, a retinal disease, or a viral infection prior to administration of a nucleic acid based TIA-1 inhibitor. In some disclosure, the methods further comprise the step of diagnosing the subject with a neurodegenerative disease or disorder (e.g., Alzheimer's disease, a tauopathy, a motor neuron disease such as ALS, FTD, FTDP-17, FTLD-TDP, Huntington's disease, Creutzfeld-Jacob disease, and spinomuscular atrophy) prior to administration of nucleic acid based TIA-1 inhibitor.

In some embodiments, pathology of the disease or disorder comprises stress granules. By comprising stress granules is meant that number of stress granules in a cell in the subject is changed relative to a control and/or healthy subject or relative to before onset of said disease or disorder. Exemplary diseases and disorders pathology of which incorporate stress granules include, but are not limited to, neurodegenerative diseases, musculoskeletal diseases, cancers, retinal diseases, and viral infections.

In some embodiments, the cell is treated with a physiochemical stressor. In some embodiments, the physicochemical stressor is selected from arsenite, nutrient deprivation, heat shock, osmotic shock, a virus, genotoxic stress, radiation, oxidative stress, oxidative stress, a mitochondrial inhibitor, and an endoplasmic reticular stressor. In some embodiments, the physicochemical stressor is ultraviolet or x-ray radiation. In some embodiments, the physicochemical stressor is oxidative stress induced by FeCl₂ or CuCl₂ and a peroxide.

Still other objects and advantages of the invention will become apparent to those of skill in the art from the disclosure herein, which is simply illustrative and not restrictive. Thus, other embodiments will be recognized by the skilled artisan.

### BRIEF DESCRIPTION OF THE DRAWINGS

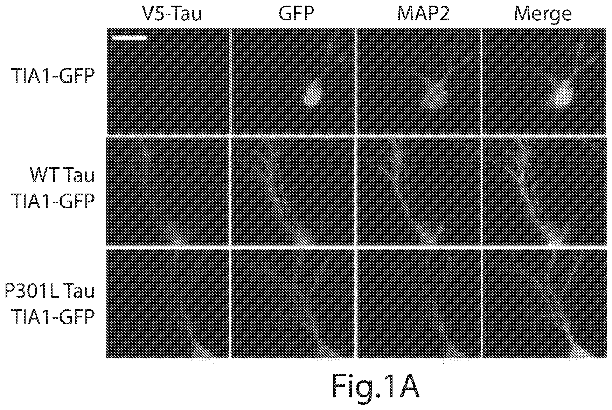

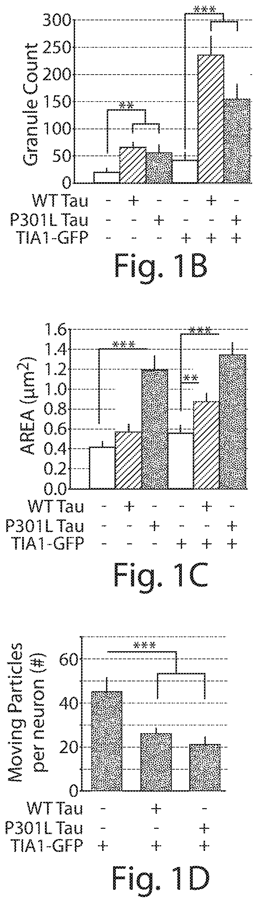

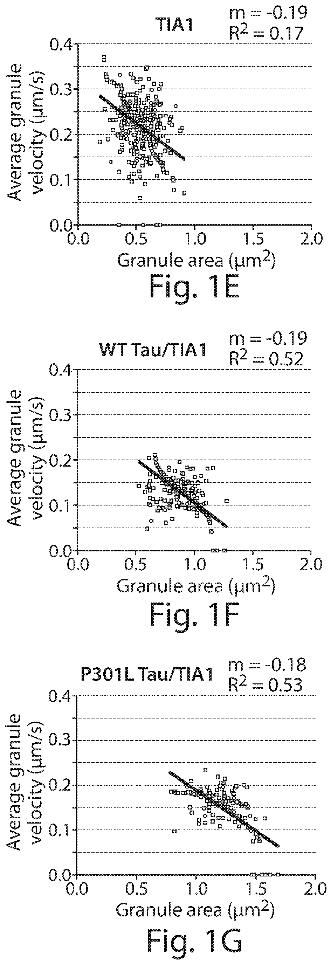

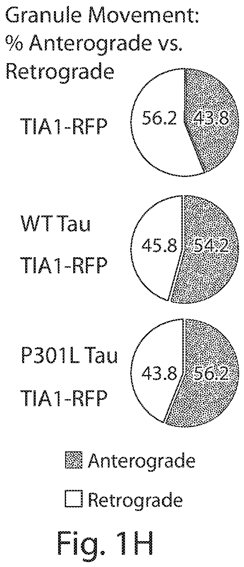

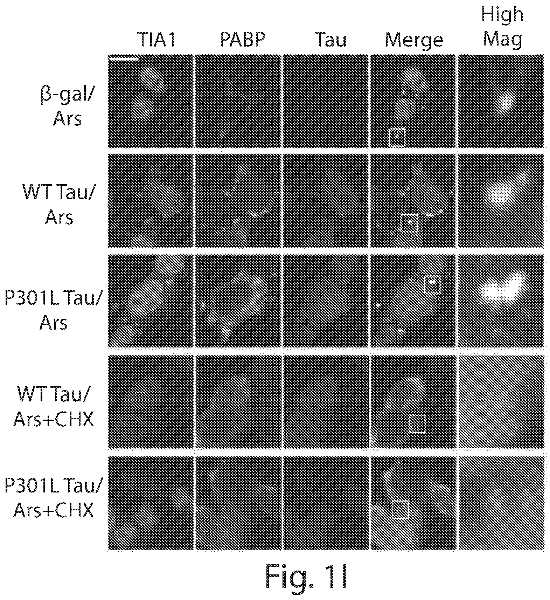

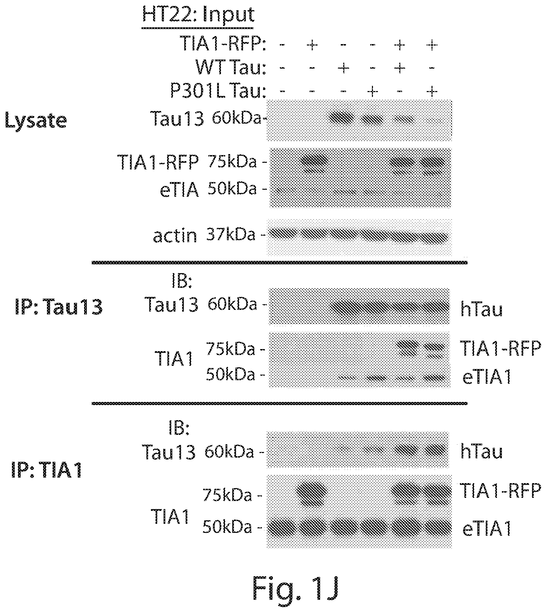

**Figures 1A-1J****:** Tau increases the somatodendritic localization of TIA1 and potentiates stress granules. **A)** Imaging for V5 (Tau), GFP (TIA1) and MAP2 in primary hippocampal Tau knockout neurons transduced with TIA1-GFP lentivirus ± AAV1-WT Tau-V5 or AAV1-P301L Tau-V5. Images indicate tau increases TIA1 movement into processes and SG formation (N=100/condition). **Figures 1B and 1C****)** WT or P301L Tau increases TIA1 granules in tau^{-/-} neurons. Granule density **(****Figure 1B****)** and area **(****Figure 1C****)** was determined using ImageJ to quantify TIA1 puncta per neuron for both endogenous TIA1 staining and exogenous TIA1-GFP fluorescence (N=100/condition). **Figure 1D****)** Live cell imaging was done on tau^{-/-} primary hippocampal neurons transduced with AAV1-TIA1-mRFP lentivirus ± AAV9-WT or P301L Tau. The number of moving particles per neuron was determined with BitPlane (Imaris) (N=20/condition). **Figures 1E-1G****)** Scatter plots of TIA1⁺ granule area vs. distance from soma in tau^{-/-} neurons. TIA1 average granule velocity vs. granule area for neurons transduced with: **Figure 1E****)** TIA1-RFP, **Figure 1F****)** WT Tau/TIA1-RFP, **Figure 1G****)** P301L Tau/TIA1-RFP (N=20/condition). **Figure 1H****)** Quantification of the net displacement of TIA1-positive granules in both anterograde (+) and retrograde (-) directions (N=20/condition). **Figure 1I****)** HT22 immortalized hippocampal cells over-expressing TIA1 and WT or P301L tau (or β-gal as a control for transfection) followed by arsenite treatment (0.5 mM, 30 min) to induce formation of SGs positive for TIA1, PABP and tau (identified with the Tau13 antibody). Formation of tau⁺ SGs is reversed with cycloheximide treatment (10µg/mL, CHX). **Figure 1J****)** Immunoblots and immunoprecipitations showing levels of Tau13, TIA1, and actin in lysates and immunoprecipitates from HT22 cells transfected with EGFP, WT Tau, or P301L ± TIA1-RFP.

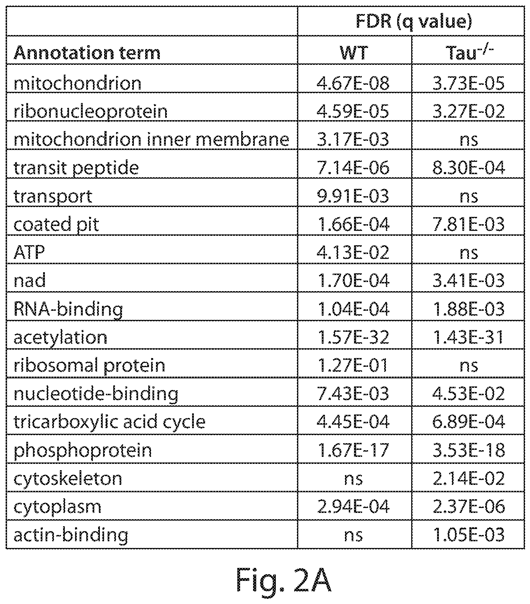

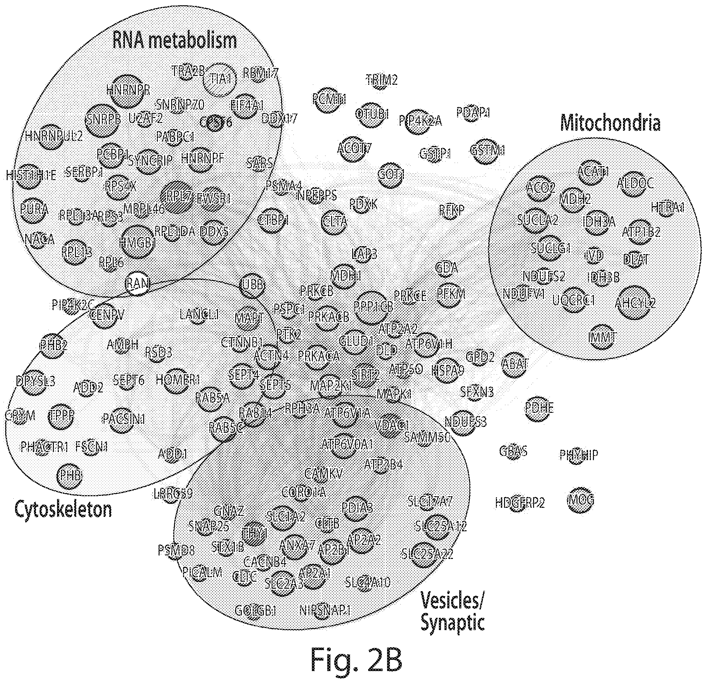

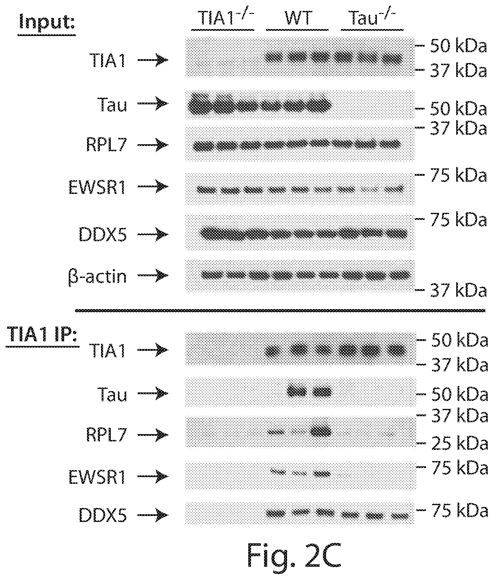

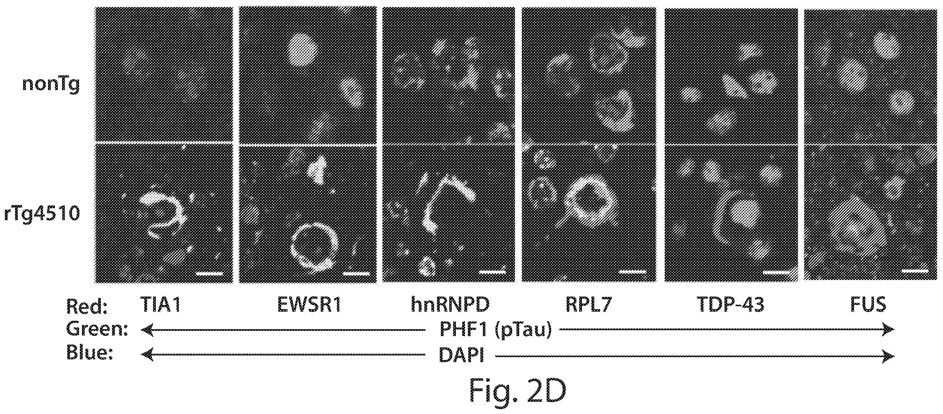

**Figures 2A-2D****: Tau regulates the TIA1 binding proteome in the brain.** Endogenous TIA1 was immunoprecipitated from the adult (10 months) cortex of WT C57BL/6J and tau^{-/-} mice (N=3), and associated proteins identified by mass spectrometry and analyzed using the DAVID bioinformatics resource (NIH). **Figure 2A****)** Functional annotation terms showing statistically significant enrichment (FDR <0.20) in either the WT or tau^{-/-} samples determined using the Keywords database. Terms highlighted in red represent annotation terms with q values more than 100-fold more significant in the WT versus tau^{-/-} cortex (ns=not significant). Only edges containing more than 3 shared annotation clusters are shown. **Figure 2B****)** Network diagram depicting the TIA1 binding proteome in the WT mouse cortex. Edges connecting nodes denote shared functional annotation terms (>3) between the connected proteins, as determined by DAVID functional clustering analysis. Proteins highlighted in red were not detected in any of the 3 tau^{-/-} samples analyzed, indicating that their interaction with TIA1 is tau-dependent. Node size is proportional to the degree of replication in the WT samples (N=3). Background circles indicate protein clusters in the TIA1 network including RNA metabolism (orange), Cytoskeleton (yellow), Vesicles/Synaptic Function (grey), and Mitochondrial (green). **Figure 2C****)** Immunoprecipitation of TIA1 from the TIA -/-, C57BL/6 and Tau -/- mouse cortical lysates used for the proteomic studies. TIA1 was evident in the C57BL/6 and Tau -/- lysates, but absent from the TIA1 -/- lysates. Excluding from the proteomic analysis any proteins that were present in the TIA -/- IP eliminates proteins IP'd non-specifically. Binding of RPL7 and EWSR1 to TIA1 was greatly reduced in the Tau -/- cortices, as suggested by the mass spectrometry studies. In contrast, binding of DDX5 to TIA1 was not affected by the absence of tau. **Figure 2D****)** Immunohistochemistry for TIA1, EWSR1, hnRNPD, RPL7, TDP-43 and FUS (red), PHF1 (Green) and DAPI (blue) in frontal cortex of 11 month old rTg4510 mice. Scale bar: 20 µM.

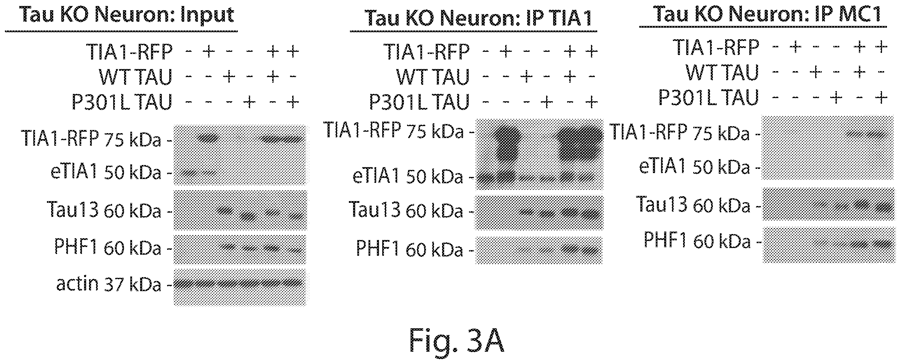

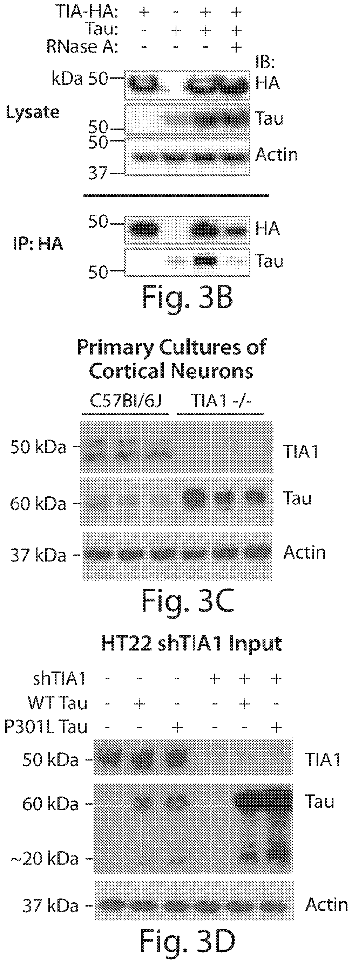

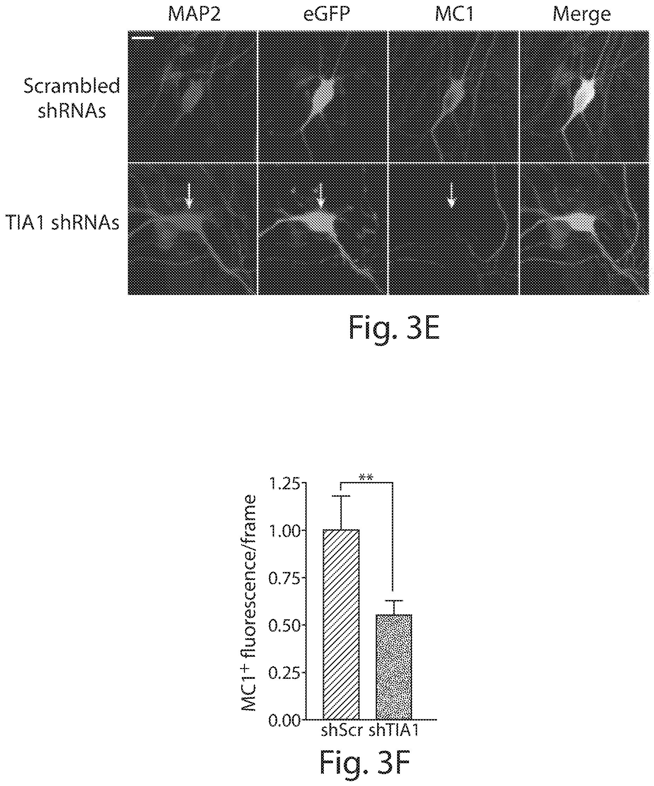

**Figures 3A-3F****:** Association of TIA1 with tau. **Figure 3A****)** Tau immunoprecipitates with TIA1, and TIA1 is found bound to MC1⁺ misfolded tau in primary hippocampal Tau knockout neurons. **Left panel:** Immunoblots showing levels of total tau (Tau13), phosphorylated tau (PHF1), TIA1, and actin in lysates from primary hippocampal tau^{-/-} neurons transduced AAV1-TIA1-RFP ± AAV9-WT or P301L Tau. **Middle panel:** Immunoprecipitation of TIA1, followed by immunoblotting with Tau13, PHF1, and TIA1. **Right panel:** Immunoprecipitation of MC1 tau, followed by immunoblotting with Tau13, PHF1, and TIA1. **Figure 3B****)** Immunoprecipitation with HA-antibody in lysates from HEK293 cells transfected with TIA1-HA ± WT tau, showing loss of binding after treatment with RNaseA. **Figure 3C****)** Immunoblots showing higher levels of total tau (Tau5 antibody) in primary cortical neurons from TIA1^{-/-} vs C57B1/6J (control) mice. **Figure 3D****)** Immunoblots and immunoprecipitations showing higher levels of total tau (Tau13 antibody) in lysates from HT22 cells transfected with EGFP, WT Tau, P301L Tau co-transfected with shControl or shTIA1. Immunoprecipitation of tau with the MC1 antibody from above lysates, followed by immunoblotting with the Tau13 antibody showed an increase in MC1 tau with TIA1 knockdown. **Figure 3E****)** Immunocytochemistry of hippocampal neurons transfected with TIA1 or control shRNA (+GPF), and stained for misfolded tau (MC1) and MAP2. Neurons transfected with TIA1 shRNA show reduced MC1 reactivity. **Figure 3F****)** Quantification of MC1 levels in neurons transfected with TIA1 or control shRNA (N=50). *p<0.05, **p<0.01, ***p<0.001. Scale bars, 10µm.

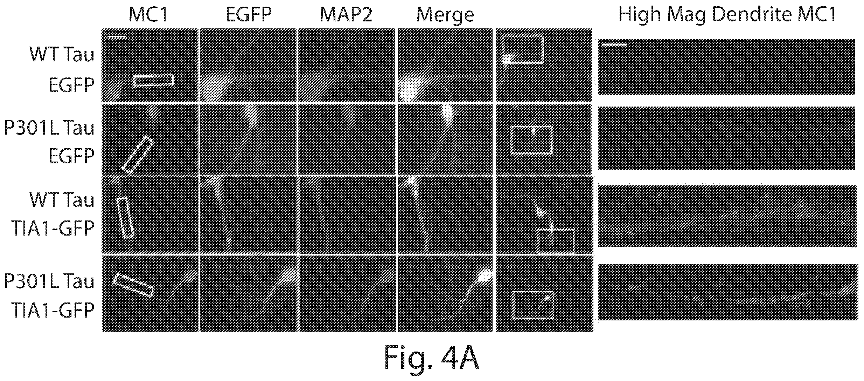

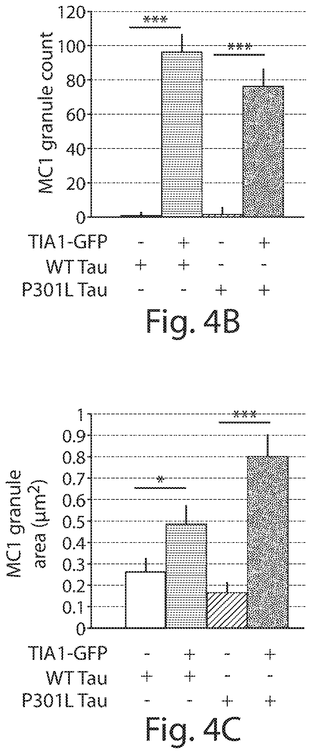

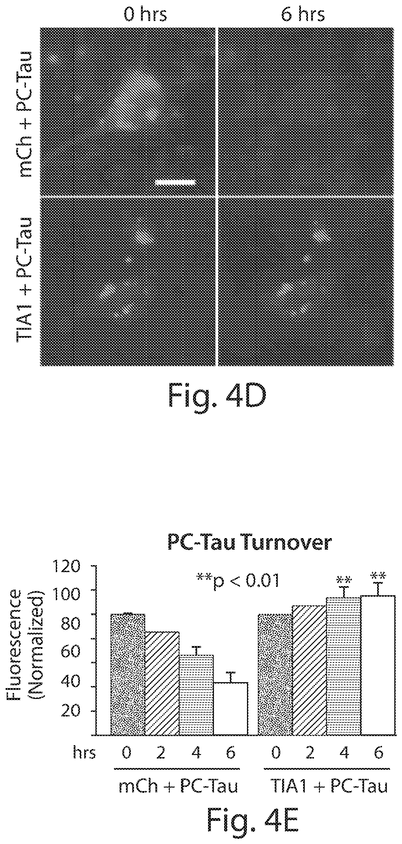

**Figures 4A-4E****:** TIA1 promotes consolidation of misfolded tau into SGs. **Figure 4A****)** TIA1 promotes MC1⁺ granules in hippocampal neurons. Immunocytochemistry for MC1 tau and MAP2 in primary tau^{-/-} hippocampal neurons transduced with AAV9-WT or P301L Tau co-transduced with EGFP or TIA1-GFP lentivirus. Scale bar, 10 µm. High magnification inset of dendritic process shows MC1 staining, scale bar 4µm. **Figure 4B****)** Quantification of MC1 granule count per neuron (N=100/condition). **Figure 4C****)** Quantification of average MC1 granule area (N=100/condition). **Figure 4D****)** Live cell imaging of photoconvertable WT tau (PC-Tau). Following photo-conversion of PC-Tau, neurons were imaged for up to 6 hrs. Representative images are shown for the 0 and 6 hr time points showing stabilization of tau in granules in cells co-expressing TIA1. **Figure 4E****)** Quantification of PC-Tau from neurons at varying time points after photo-conversion (N=20 per condition). Scale bar, 10 µm. *p<0.05, *** p<0.001.

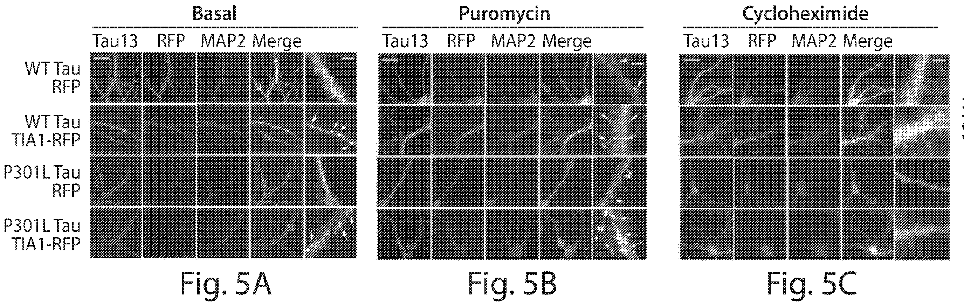

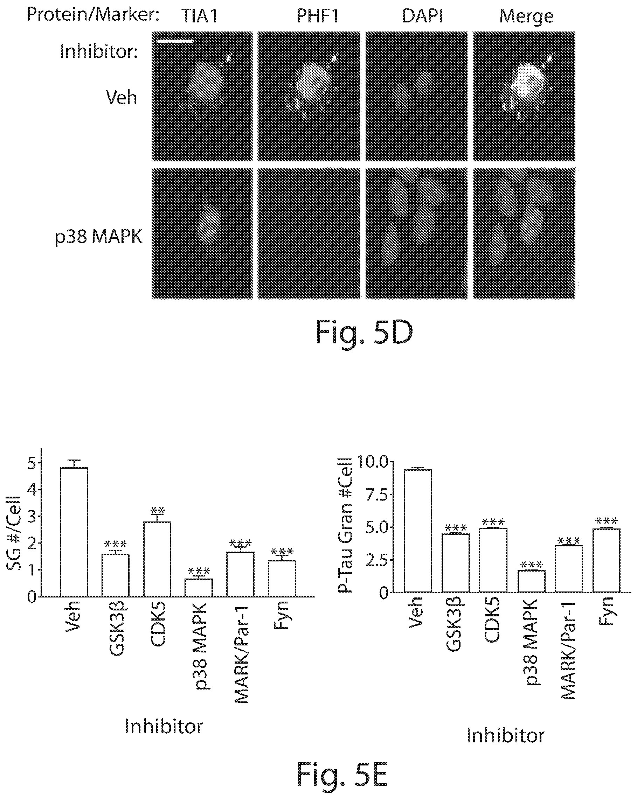

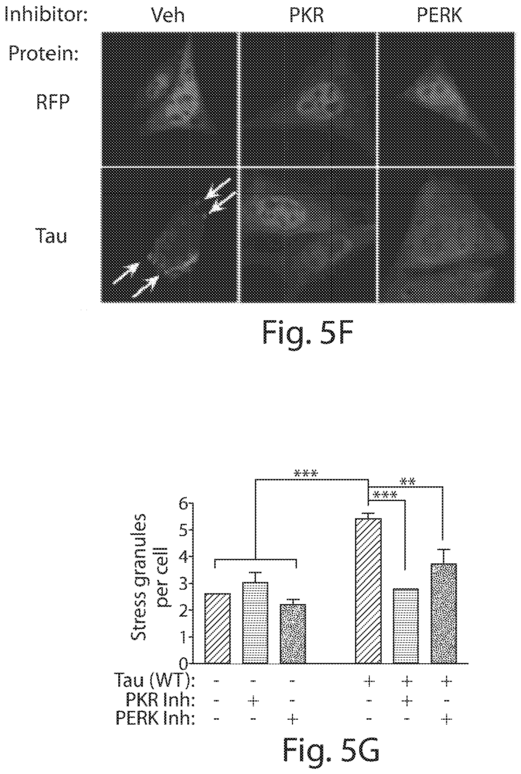

**Figures 5A-5G****:** Immunocytochemistry for Tau (Tau13 antibody) and MAP2 in primary cultures of tau^{-/-} hippocampal neurons transduced with AAV1 RFP or AAV1-TIA1-mRFP ± AAV9-WT or P301L Tau. The columns on the right show images at high magnification (arrows identify tau granules). **Figure 5A****)** No treatment, **Figure 5B****)** Puromycin **Figure 5C****)** Cycloheximide. **Figure 5D****)** Inhibition of tau-induced TIA1⁺ SGs and PHF1⁺ granules in HT22 cells following transfection with TIA1 and WT tau and treatment with the p38 kinase inhibitor, (SB203580, 20 µM). **Figure 5E****)** Quantification of inhibition of SGs and tau granules in HT22 cells following treatment for 24 hrs with one of 5 different kinase inhibitors: GSK3β (XXVI, 20 µM), CDK2/5 (alkylbenzyldimethyammonium chloride, 5 µM), p38 MAPK (SB203580, 20 µM), MARK/Par-1 (39621, 20µM) and Fyn (PP2, 20 nM). **Figure 5F****)** Inhibition of tau-induced TIA1⁺ SGs in HT22 cells following transfection with TIA1 and WT tau and treatment for 24 hrs with the PKR inhibitor (C16, 1 µM) or PERK inhibitor (GSK2606414, 50 nM). **Figure 5G****)** Quantification of granule count in cells treated with kinase inhibitors. *p<0.05, **p<0.01, *** p<0.001. Low magnification: 10 µm, Inset: 2 µm.

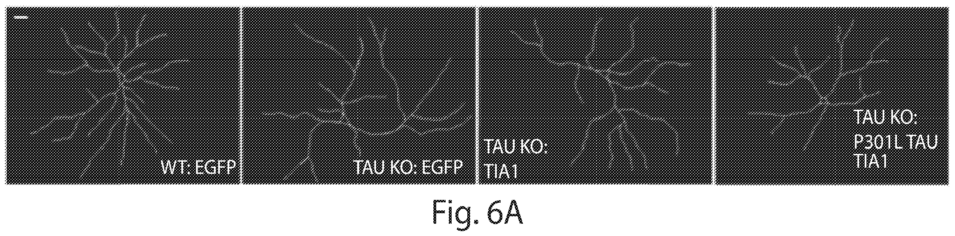

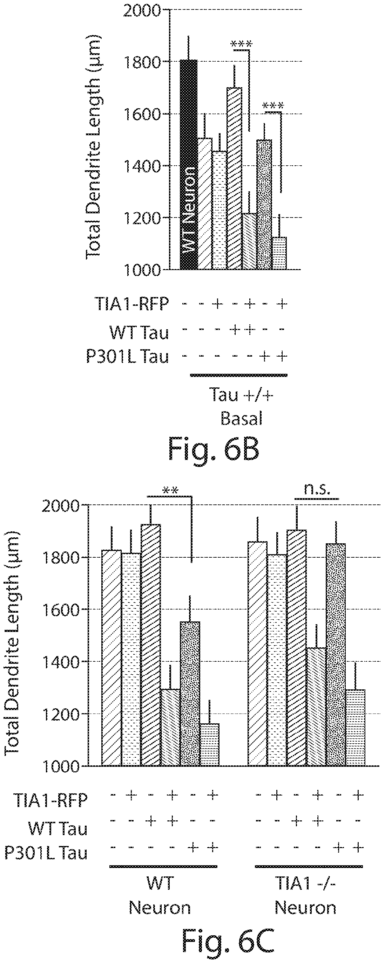

**Figures 6A-6H****:** Expression of TIA1 with tau reduces dendrite lengths in hippocampal neurons. **Figure 6A****)** Representative dendrite traces of hippocampal neurons (primary culture, DIV21) using MAP2 labeling from WT or tau^{-/-} mice, transduced with AAV1-TIA1-mRFP or mRFP ± AAV9-WT or P301L tau. **Figure 6B****)** Quantification of dendrite lengths (N=30/condition).

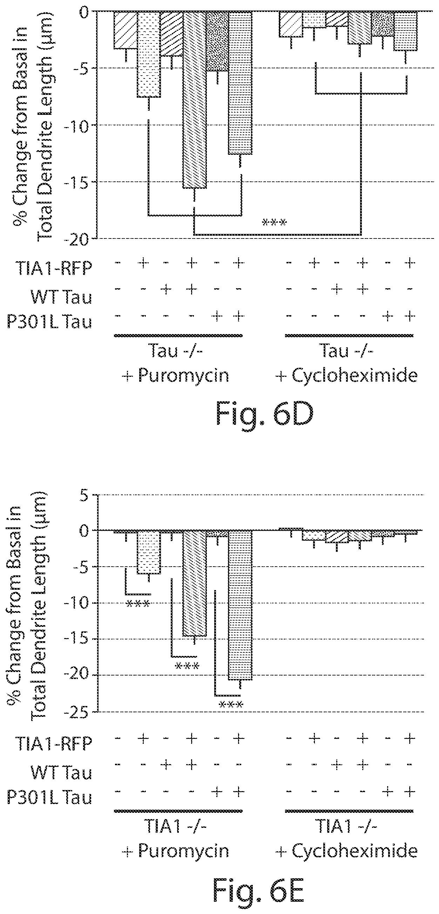

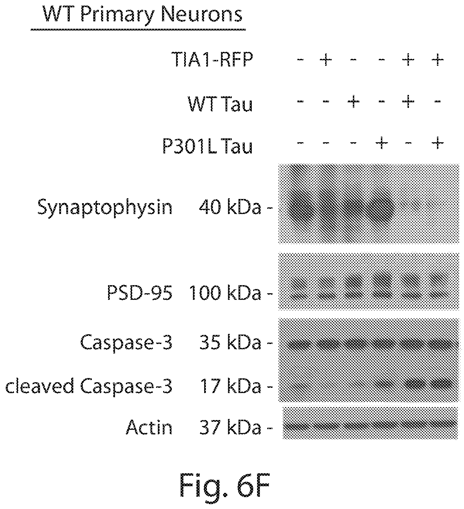

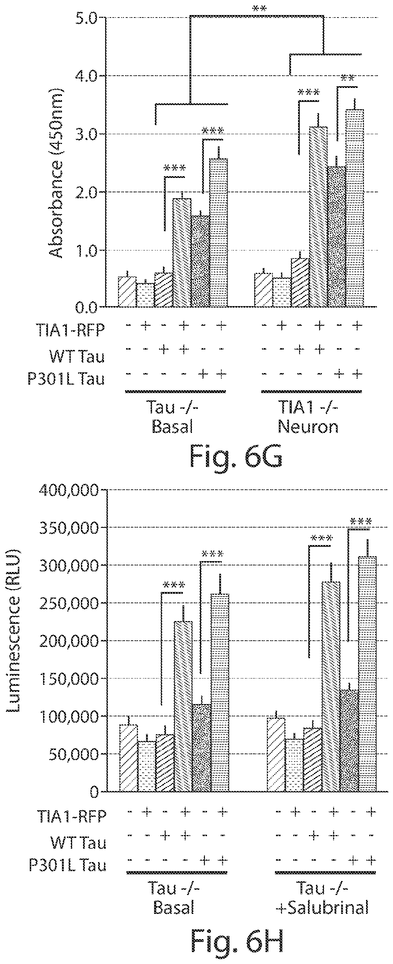

**Figure 6C**) Quantification of dendrite lengths in C57B1/6J and TIA1^{-/-} primary hippocampal neurons transduced with AAV1-TIA1-mRFP or mRFP ± AAV9-WT or P301L tau (N=30/condition). **Figure 6D****)** Quantification of the % change in dendrite lengths in hippocampal neurons (primary culture, DIV21) from tau^{-/-} mice transduced with AAV1-TIA1-mRFP or mRFP ± AAV9-WT or P301L tau and treated with translation inhibitors puromycin (SG-promoting) or cycloheximide (SG inhibiting). Comparison is to neurons from C57B1/6J (control) mice. (N=30/condition). **Figure 6E****)** The same experiment as in D, but done in TIA^{-/-} neurons (N=30/condition). **Figure 6F****)** Immunoblots showing levels of synaptophysin, PSD-95, caspase-3, and cleaved caspase-3 in WT primary cortical neurons transduced with AAV1-TIA1-mRFP or mRFP ± AAV9-WT or P301L Tau. **Figure 6G****)** Colorimetric quantification of DNA fragmentation to measure apoptosis (TiterTACS Colorimetric Apoptosis Detection kit, Trevigen). Tau^{-/-} primary hippocampal neurons (DIV21) transduced with AAV1-TIA1-mRFP or mRFP ± AAV9-WT or P301L tau under basal conditions and treatments with 25 µM salubrinal (N=6 wells/condition). **Figure 6H****)** Luminescent quantification of caspase cleavage (Caspase-Glo 3/7 Assay kit, Promega). Comparison of the amount of caspase cleavage in tau^{-/-} primary hippocampal neurons (DIV21) transduced with AAV1-TIA1-mRFP or mRFP ± AAV9-WT or P301L treated with 25 µM salubrinal (N=6 wells/condition). Scale bar, 10 µm. **p<0.01, ***p<0.001.

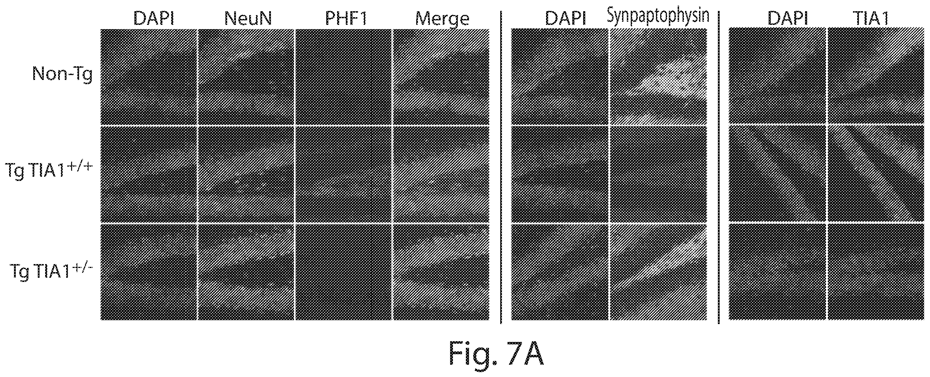

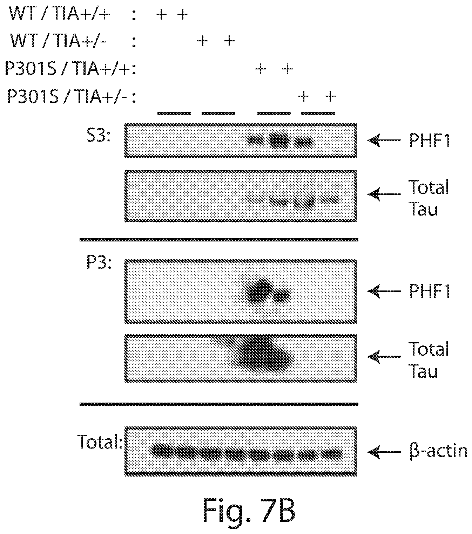

**Figures 7A-7B****:** TIA1 haplo-insufficiency inhibits tauopathy. **Figure 7A****)** Phospho-tau (PHF1), synaptic loss (synaptophysin) and TIA1 levels were quantified in 2.5 month old mice expressing P301S Tau, P301S Tau/TIA1 +/- or wild type control. Levels are shown for dentate gyrus and CA3 fields of the hippocampus. Similar results were evident in the cortex. **Figure 7B****)** Immunoblot of soluble (S3) and sarkosyl insoluble (P3) fractions from cortices of the 2.5 month WT, TIA1 +/-, P301S Tau or P301S Tau/TIA1 +/- crosses. Haplo-insufficiency lead reduced levels of total and phosphorylated tau in the insoluble fractions.

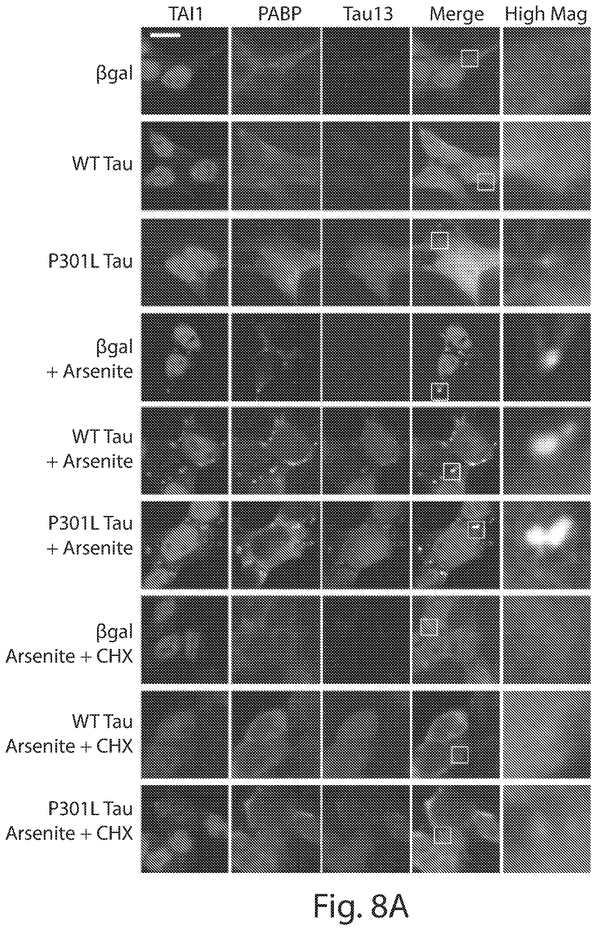

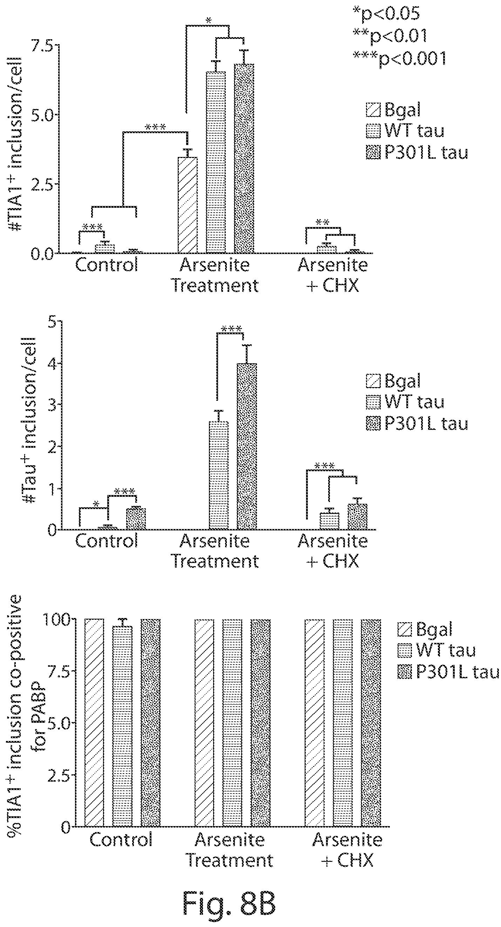

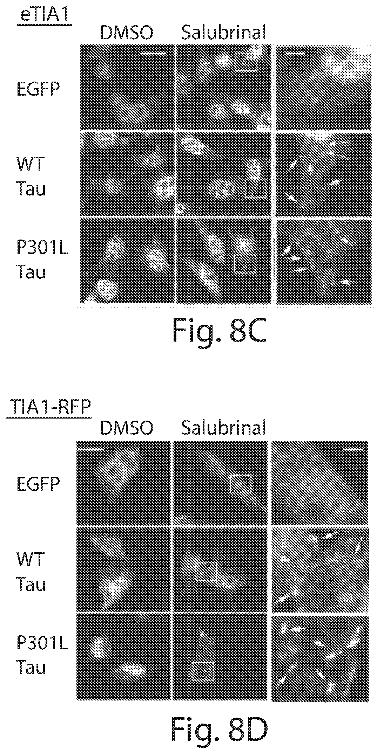

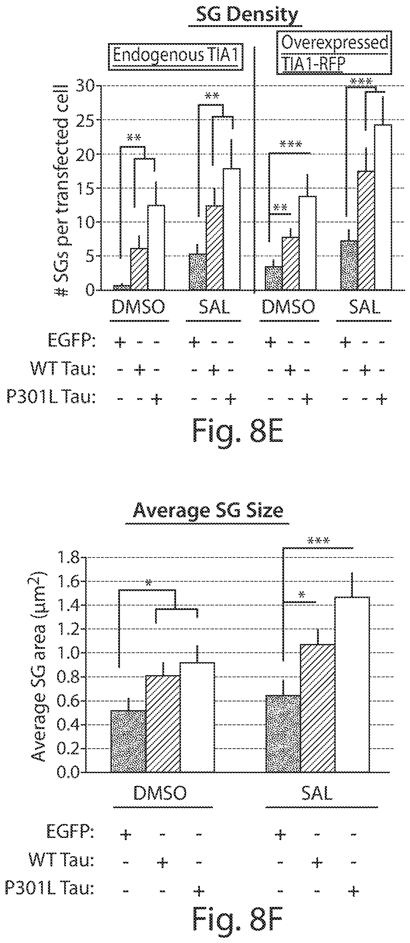

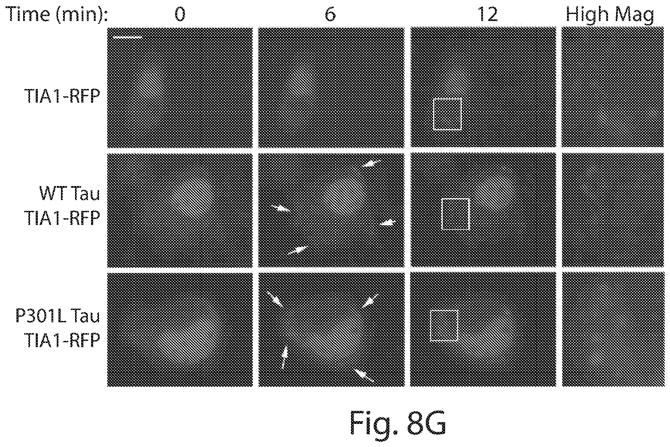

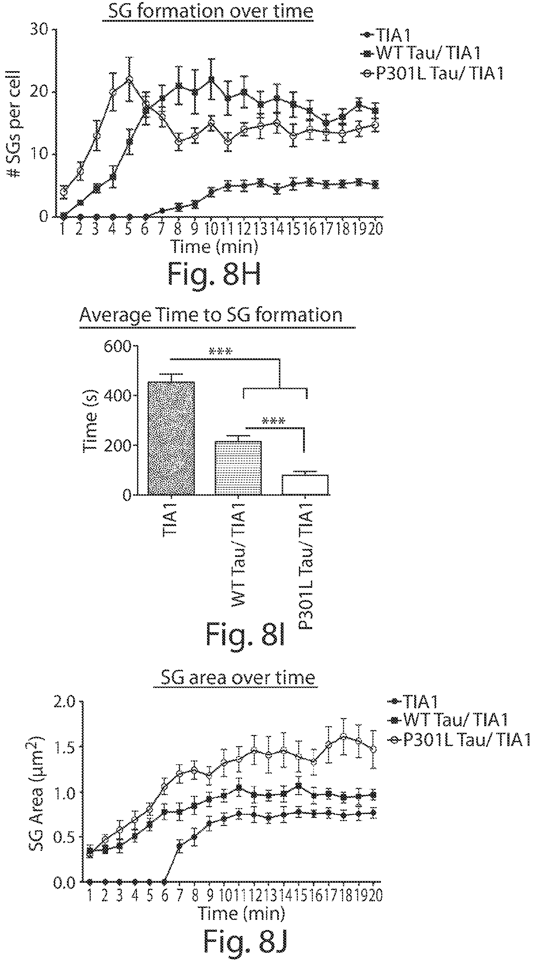

**Figures 8A-8J****:** **Figure 8A****)** Expanded data from Figures 1A-1J showing immunocytochemistry for TIA1, PABP and tau (Tau13 antibody) in HT22 immortalized hippocampal cells with tau positive granules (identified with the Tau13 antibody) formed by co-expressing TIA1 ± WT or P301 tau followed by arsenite treatment (15 µM, 6 hrs) or arsenite + cycloheximide (10 µg/mL). Scale bar: 10 µm. **Figure 8B****)** Quantification of tau (Tau13), TIA1 and PABP positive inclusions in cells from Figure 8A. N=50. **p<0.01, ***p<0.001. **Figure 8C****)** Immunocytochemistry for endogenous TIA1 in HT22 cells transfected with EGFP, WT Tau, or P301L Tau treated with DMSO vehicle or 25 µM salubrinal. Inset shows higher magnification, with arrows pointing to TIA1 granules. **Figure 8D****)** Fluorescence imaging of TIA1-RFP transfected in HT22 cells co-transfected with EGFP, WT Tau, or P301L Tau treated with DMSO vehicle or 25µM salubrinal. Inset shows higher magnification with arrows pointing to TIA1 granules. **Figure 8E****)** Quantification of TIA1 positive granule density for both endogenous TIA1 labeling and exogenous TIA1-RFP fluorescence (N=50). Note that the increase in SG count in HT22 cells is greater for P301L than WT, while in primary hippocampal neurons (see Figures 1C and 1D) the increase in SG count is less for P301L than WT. This difference between the behavior of primary neurons and neuronal cell lines is observed consistently and might reflect physiological differences between highly differentiated, non-dividing hippocampal neurons and immortalized neuronal cell lines. **Figure 8F****)** Quantification of TIA1 positive granule size for both endogenous TIA1 labeling and exogenous TIA1-RFP fluorescence (N=50). **Figure 8G****)** Live cell imaging of TIA1-RFP was done in HT22 cells 24 hrs after transfection with EGFP, WT Tau-EGFP, or P301L Tau-EGFP and co-transfection with TIA1-mRFP. Imaging was done using a Zeiss AxioObserver Z1 microscope illuminated by Colibri LEDs with a heated stage and CO2 chamber. 500µM Arsenite was added to the cells to stimulate SG formation and images were taken every 15s for 30min. Representative images shown for 0, 6, and 12 min, with arrows at 6 min indicating the SG formation. High magnification insets shown. **Figure 8H****)** Quantification of the average number of SGs per cell over time per condition (N=12 per condition) was done using the "spots" feature in the Imaris software identifying all granules >1 µm2 (Bitplane), **Figure 8I****)** Quantification of the mean time to SG formation, as defined by the time to detection of the first SG in each imaged cell (N=12). **Figure 8J****)** Graph depicting the average area of SGs over time per condition (N=12/condition). Quantification was done using the "spots" feature in the Imaris software (Bitplane). Scale bars, 10 µm. * p<0.05, **p<0.01, ***p<0.001.

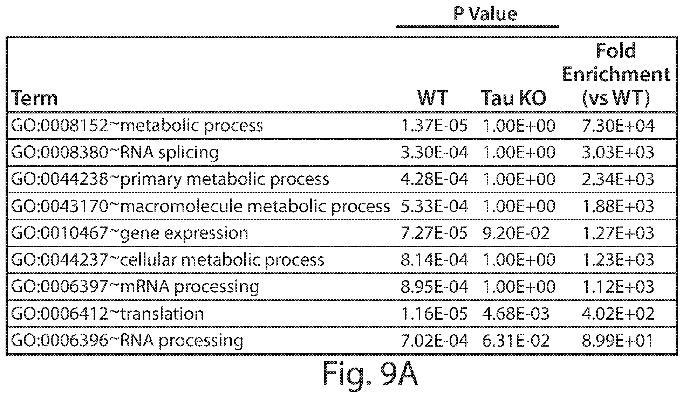

**Figures 9A-9B****:** **Figure 9A****)** Comparison of the functional categories showing statistically significant enrichment (Benjamini corrected p values <0.05) in the TIA1 associated proteome for either WT or Tau KO cortex as determined using the DAVID bioinformatics resource (Huang da et al., 2009). Listing of the enriched functional categories by GO gene ontology annotation.

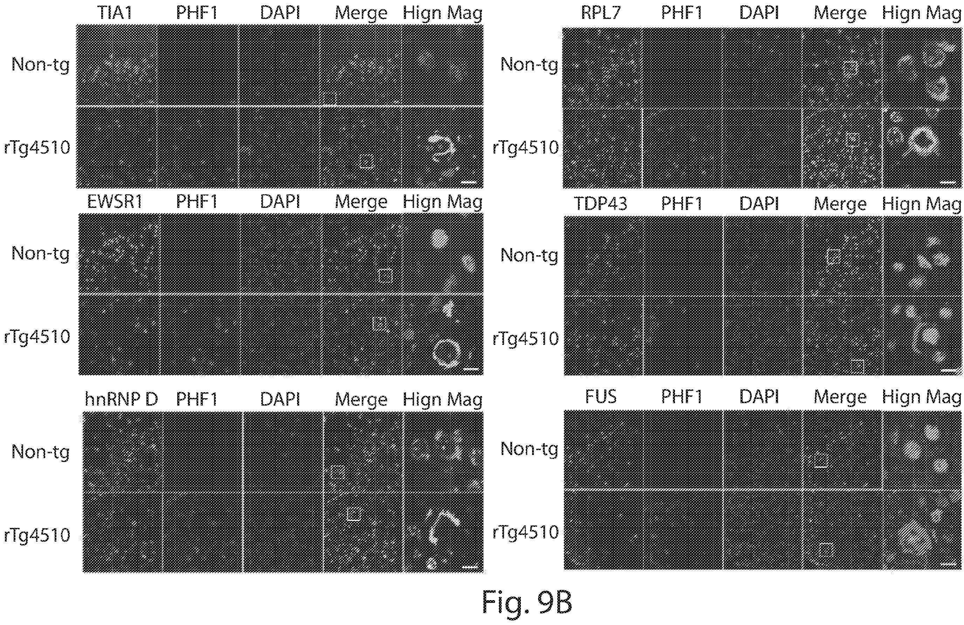

**Figure 9B****)** Immunohistochemistry of 8 month old cortices from P301L rTg4510 mice using antibodies directed against TIA1, EWSR1, HNRNPD, RPL7, TDP-43 and FUS. Scale bars: 10 µm.

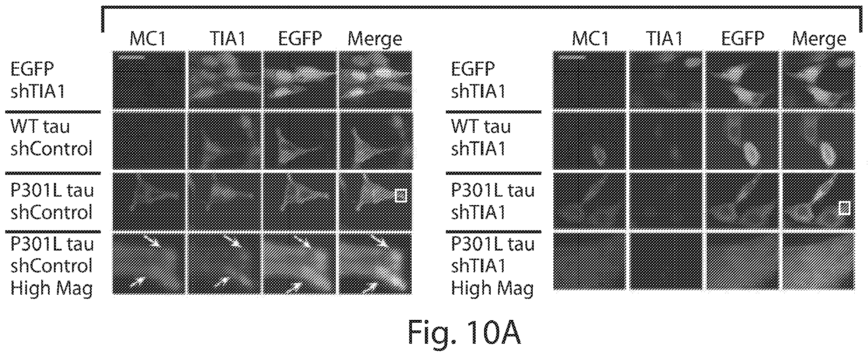

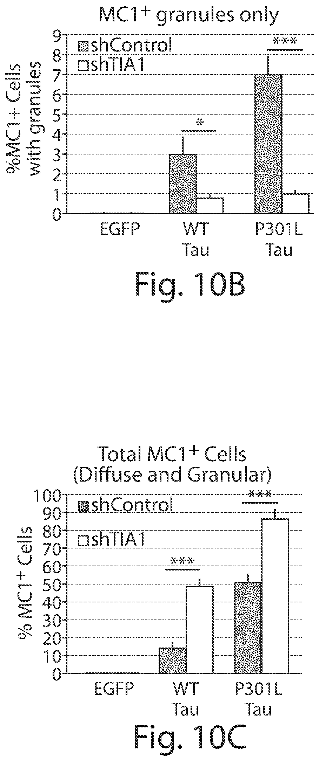

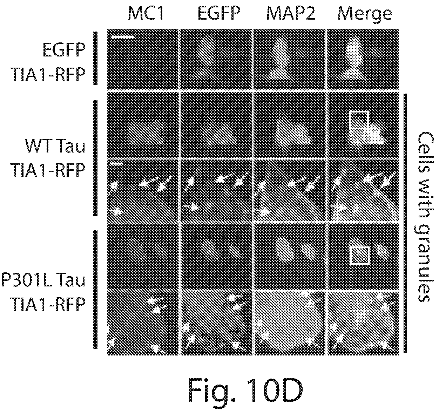

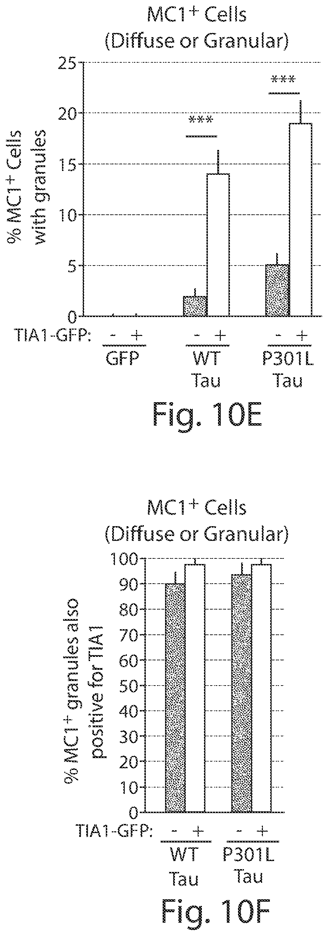

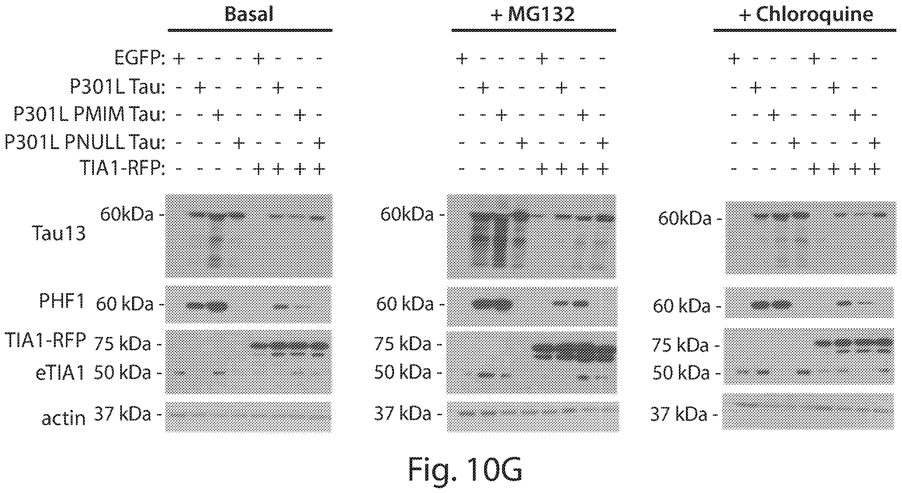

**Figures 10A-10G****:** TIA1 knockdown also reduced formation of MC1 tau granules in HT22 cells **Figure 10A****)** Immunocytochemistry for MC1 tau and eTIA1 in HT22 cells transfected with EGFP, WT Tau, or P301L Tau (visualized via EGFP) and co-transfected with shControl (scrambled, left panel) and shTIA1 (right panel). **Figure 10B****)** Quantification of the percentage of MC1⁺ cells that have granules (N=100/condition). **Figure 10C****)** Quantification of the percentage of MC1⁺ transfected cells per condition (N=100/condition). **Figure 10D****)** Immunocytochemistry for MC1 (misfolded) tau in HT22 cells transfected with EGFP, WT Tau, or P301L Tau, and co-transfected with TIA1-RFP (visualized via RFP), scale bar 10µm. Bottom panels show cells with granules, with higher magnification (arrows point to MC1⁺/TIA1⁺ granules, scale bar 10 µm). **Figure 10E****)** Quantification of the percentage of MC1⁺ cells that have granules (N=100/condition). **Figure 10F****)** Quantification of the percentage of MC1⁺ granules that are also positive for TIA1 **Figure 10G****)** Immunoblots showing levels of total tau (Tau13), phosphorylated tau (PHF1), TIA1-RFP, endogenous TIA1 (eTIA1) and actin in lysates from HT22 cells transfected with β-gal, P301L Tau, P301L Tau-PMIM, or P301L Tau-NULL, then treated overnight with 5 µM MG132 or 50 µM chloroquine. (N=100/condition). * p<0.05, ***p<0.001.

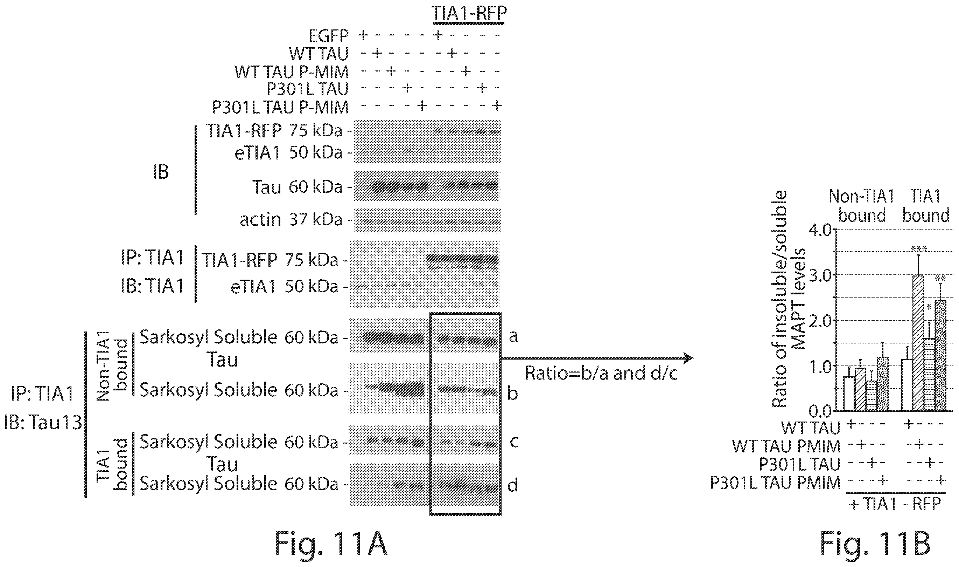

**Figures 11A-11B****:** TIA1 promotes consolidation of misfolded tau into SGs. **Figure 11A****)** Immunoblots of total lysates from HT22 cells transfected with TIA1-RFP ± EGFP, WT Tau, WT Tau-PMIM, P301L Tau, or P301L Tau-PMIM. The WT Tau-PMIM construct has had 14 sites exhibiting increased phosphorylation in AD replaced with aspartate (Hoover et al., 2010). This was followed by TIA1 immunoprecipitation/immunoblot, followed by fractionation (1% sarkosyl) and immunoblotting of the TIA1-bound and non-TIAl bound protein complexes. **Figure 11B****)** Quantification of the ratio of insoluble to soluble tau levels showing increased ratio of insoluble to soluble tau in the TIA1 bound fraction. Binding of P301L tau to TIA1 was not strongly affected by the PMIM modification, suggesting that the P301L mutation exerts effects on aggregation similar to that of phosphorylation, rendering the two changes non-additive (N=3).

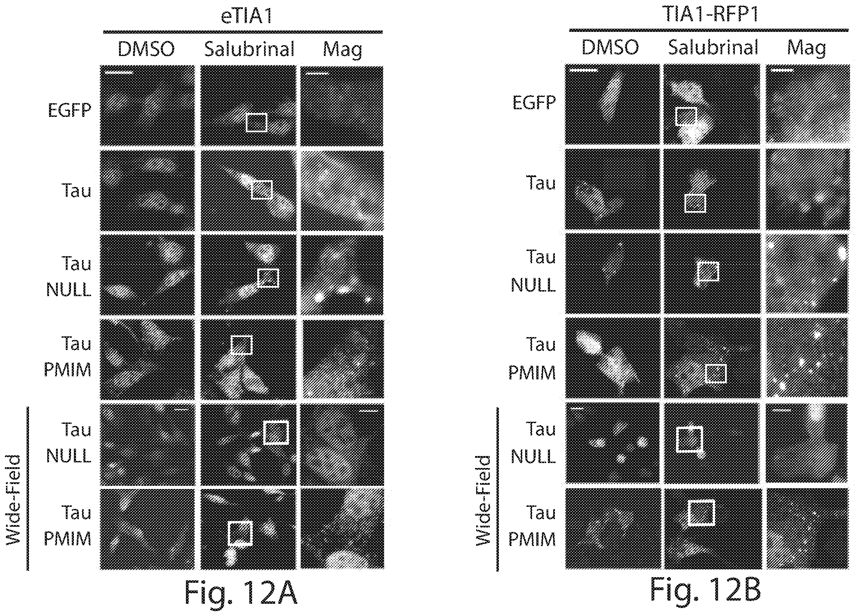

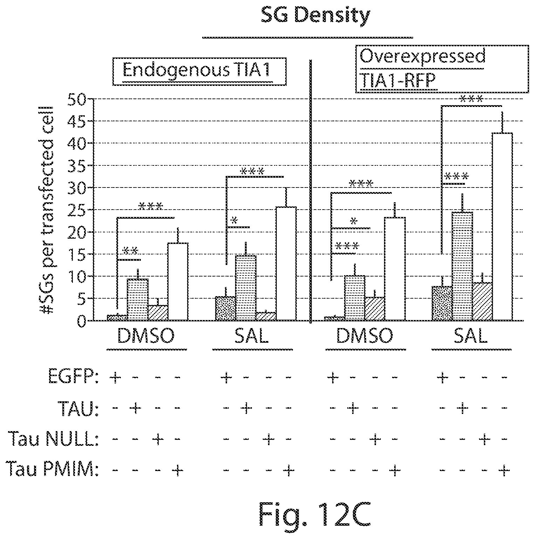

**Figures 12A-12C****:** Phospho-mimetic tau increases TIA1 granule formation. **Figure 12A****)** Phosphorylation of tau increases TIA1+ granules endogenously **(****Figure 12A****,** images; **Figure 12C****,** quantification) and with over-expressed TIA1 **(****Figure 12B****,** images; **Figure 12C****,** quantification) (N=50). Immunocytochemistry for endogenous TIA1 in HT22 cells transfected with EGFP, or Tau (P301L, P301L PMIM or P301L NULL) treated with vehicle (DMSO) or 25 µM salubrinal. The WT Tau-NULL construct has had 14 sites exhibiting increased phosphorylation in AD replaced with alanine (Hoover et al., 2010). The Tau NULL construct exhibited fewer cells with granules. The reduction is evident in wide-field images of cells shown in the second to last row of panels (labeled "Wide Field"). However, rare cells expressing tau NULL exhibited granules, that tended to be larger; the image shown for NULL tau presents a cell containing a granule in order to allow the reader to evaluate the small number of cells in which granules were present. Scale bar 10 µm. Inset = higher magnification, scale bar 2 µm. * p<0.05, **p<0.01, ***p<0.001.

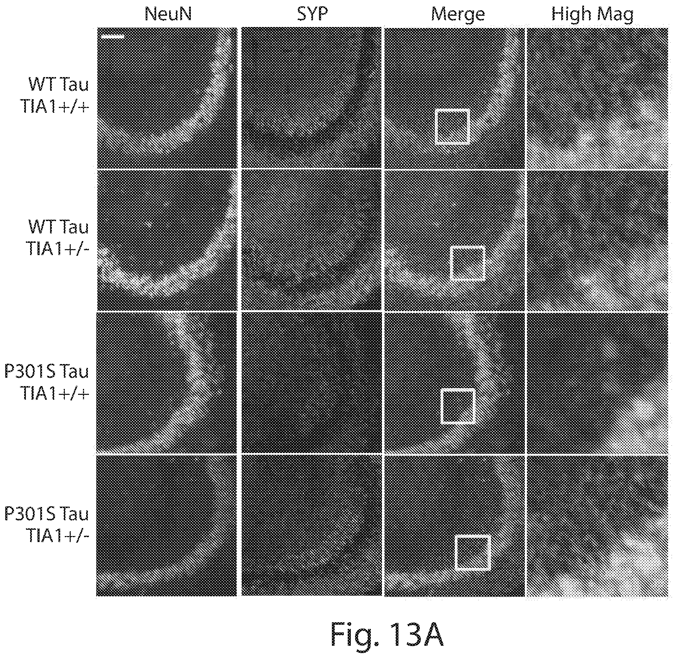

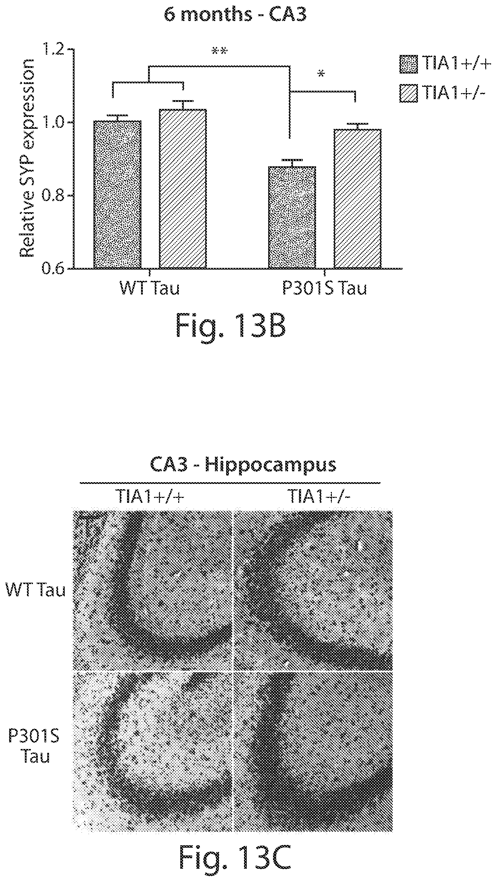

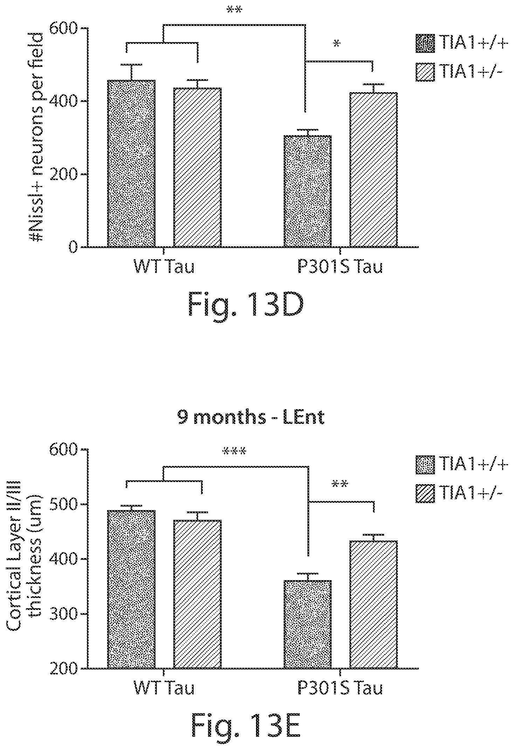

**Figures 13A-13E****. TIA1 reduction rescues synaptic and neuronal loss in PS19 mice.** **Figure 13A****.** IHC of NeuN and synaptophysin (SYP) in the CA3 region of 6 month non-transgenic (WT Tau) and PS19 (P301S Tau) hippocampus. Scale bar = 20 um. **Figure 13B****.** Quantification of relative SYP expression from A. *p<0.05 **p<0.01 by 2-way ANOVA with Tukey's post-hoc test (n=6/group). **Figure 13C****.** Representative Cresyl Violet staining of CA3 in 9 month non-transgenic and PS19 mice. Scale bar = 20 um. **Figure 13D****.** Quantification of number of Nissl+ neurons per field in C. *p<0.05 **p<0.01 by 2-way ANOVA with Tukey's post-hoc test (n=6/group). **Figure 13E****.** Quantification of average cortical thickness from E. **p<0.01 ***p<0.001 by 2-way ANOVA with Tukey's post-hoc tests (n=4-6/group).

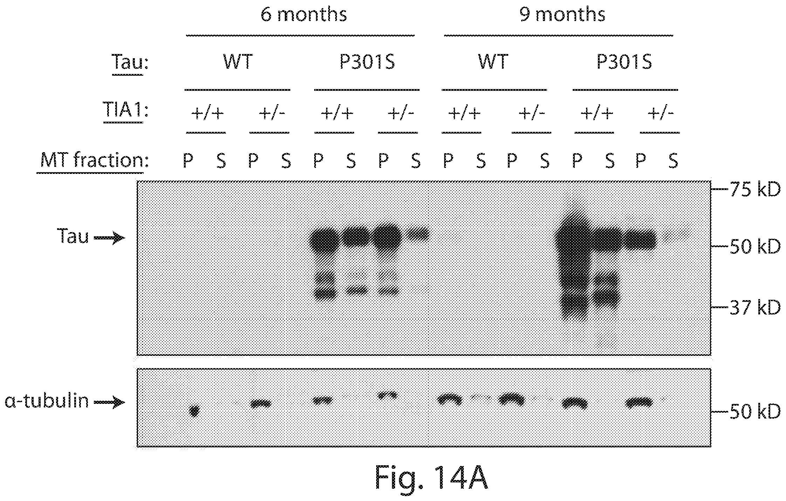

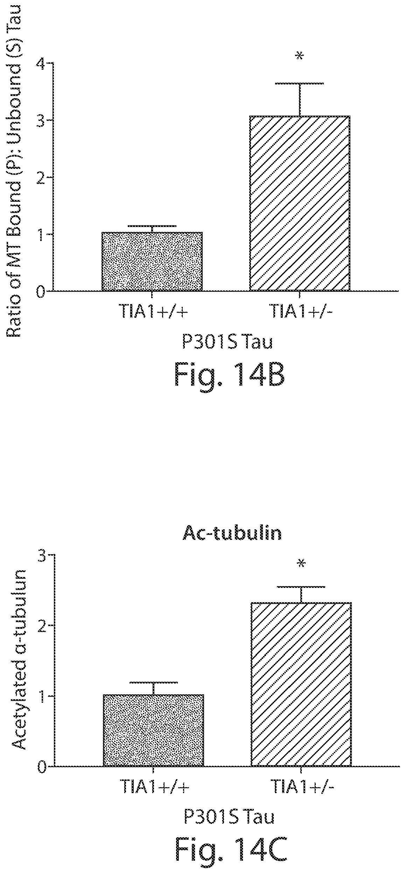

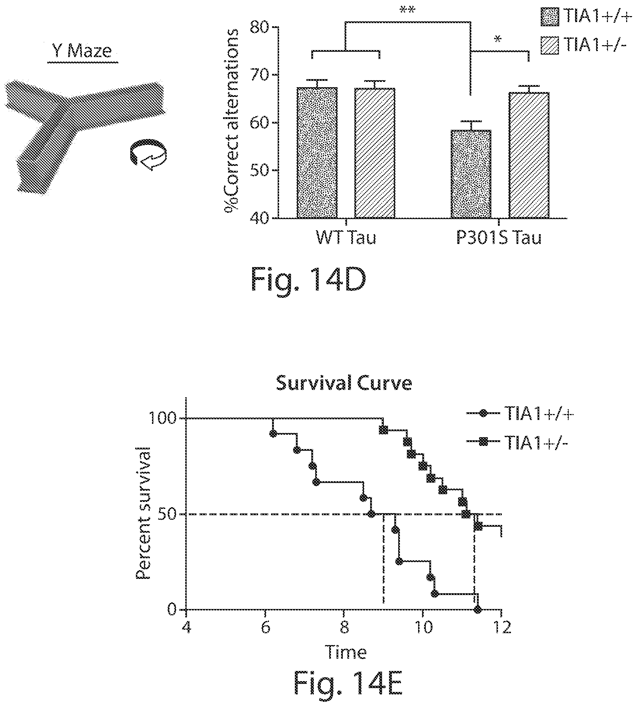

**Figures 14A-14E****. TIA1 reduction confers functional and behavioral protection in PS19 mice.** **Figure 14A****.** Immunoblot of total tau detected in microtubule (MT)-bound (pellet, P) and unbound (supernatant, S) fractions in 6 and 9 month WT and PS19 mice. **Figure 14B****.** Quantification of ratio of MT-bound to MT-unbound tau in A. *p=0.0138 by Student's t-test (n=4/group). **Figure 14C****.** Quantification of the ratio of acetylated to total a-tubulin in 6 month PS19 cortex. *p=0.0138 by Student's t-test (n=4/group). **Figure 14D****.** Percent correct alternations in the Y maze spontaneous alternation task for 6 month old non-transgenic and PS19 mice. *p=0.0213 **p=0.0072 by 2-way ANOVA with Tukey's post-hoc test (n=16-20 mice/group). **Figure 14E****.** Kaplan-Meier survival plot of P301S TIA1+/+ (n=12) compared to P301S TIA1+/- (n=16) mice. ***p<0.001 by the Gehan-Breslow-Wilcoxon test.

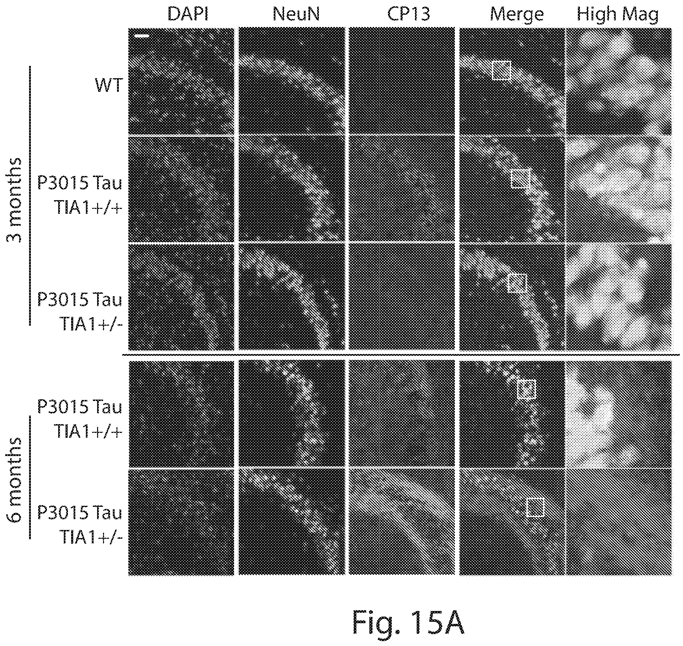

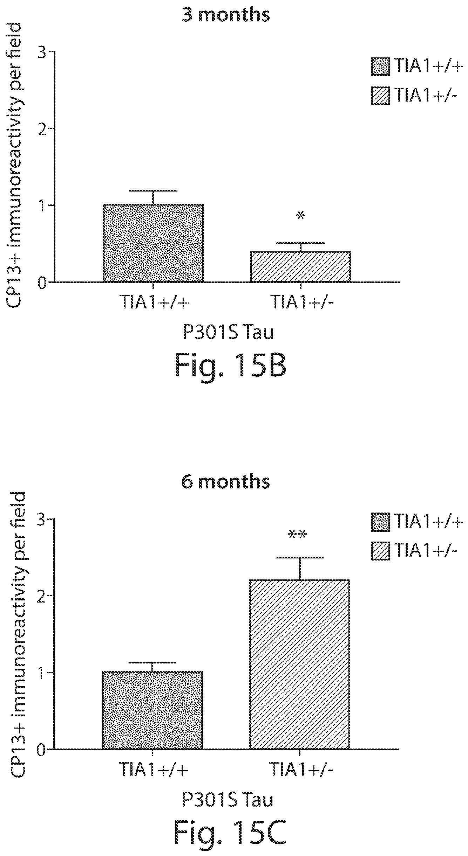

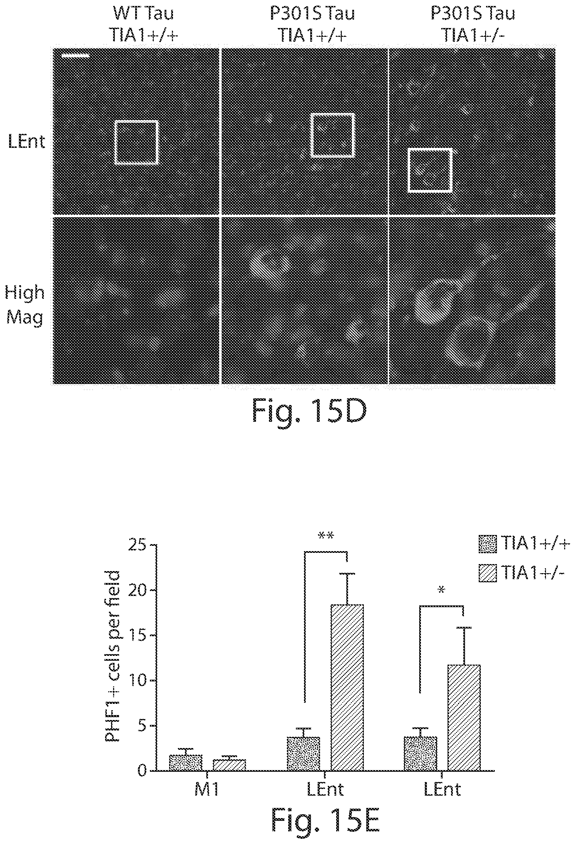

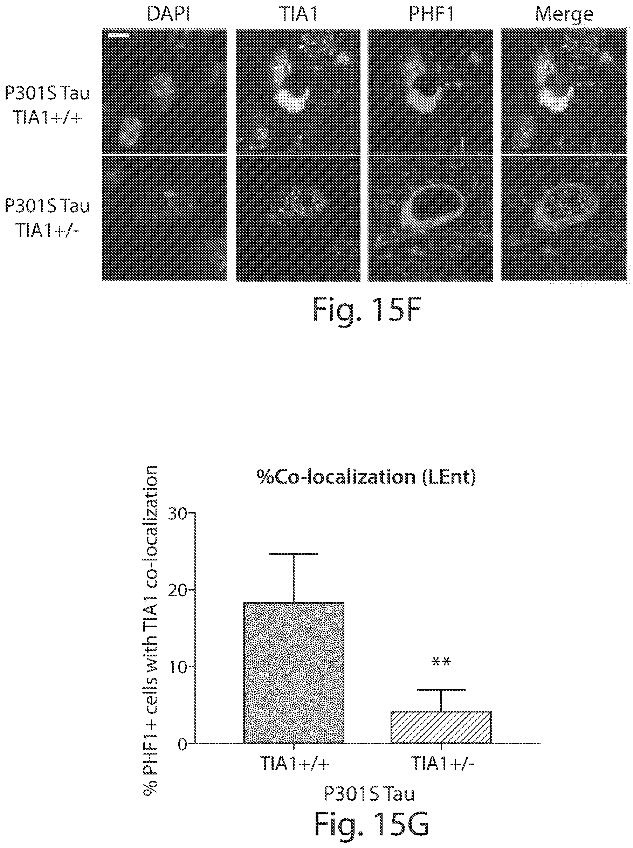

**Figures 15A-15G****. TIA1 reduction elicits a biphasic change in tau phosphorylation.** **Figure 15A****.** IHC of DAPI, NeuN, and CP13 (S202) phospho-tau in the CA3 region of 3 (top panels) and 6 (bottom panels) month hippocampus of P301S TIA1+/+ vs P301S TIA1+/- mice. Scale bar = 20 um. **Figures 15B and 15C****.** Quantification of relative CP13 immunofluorescence per field for 3 **(****Figure 15B****)** and 6 **(****Figure 15C****)** month PS19 mice. *p=0.0266 **p=0.0044 by Student's t-test (n=6/group). **Figure 15D****.** IHC of DAPI and PHF1 (S396/S404) phospho-tau in the lateral entorhinal cortex (LEnt) of 9 month old P301S TIA1+/+ and P301S TIA1+/- mice. Scale bar = 10 um. **Figure 15E****.** Quantification of number of PHF1+ cells in **Figure 15D****.** *p=0.0483 **p=0.0028 by 3-way ANOVA with Bonferroni post-hoc tests (n=5/group). **Figure 15F****.** 63x magnification images of PHF1+ cells in P301S TIA1+/+ and P301S TIA1+/- LEnt. Scale bar = 2 um. **Figure 15G****.** Quantification of percent of PHF1+ cells with TIA1 co-localization in F. **p=0.0074 by Student's t-test (n=5/group).

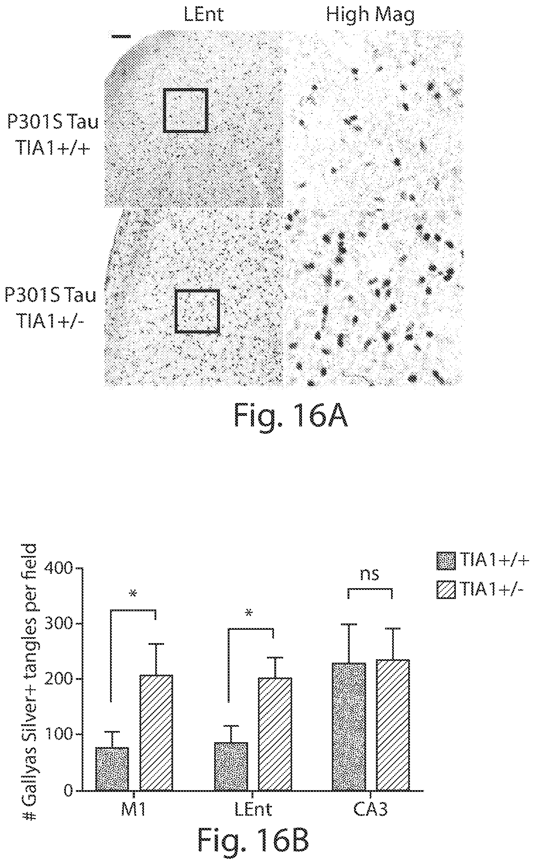

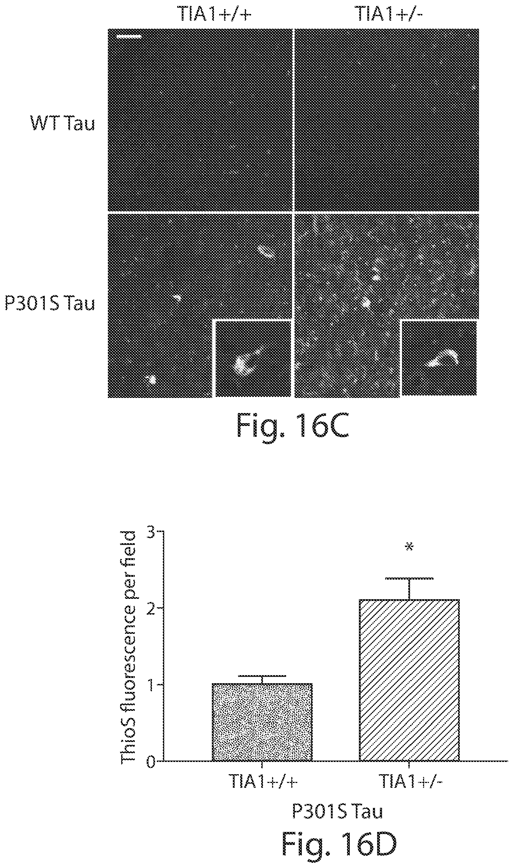

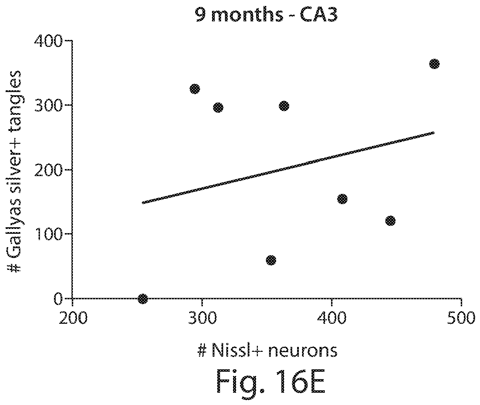

**Figures 16A-16E****. TIA1 reduction accelerates neurofibrillary tangle formation in PS19 mice.** **Figure 16A****.** Representative images of Gallyas Silver stained neurofibrillary tangles in 9 month P301S TIA1+/+ and P301S TIA1+/- mice. Scale bar = 40 um. **Figure 16B****.** Quantification of number of Gallyas Silver+ tangles in the frontal cortex (primary motor area, M1), lateral entorhinal cortex (LEnt), and CA3. *p<0.05 by 3-way ANOVA with Bonferroni post-hoc tests (n=5/group). Fig. 16C. Representative images of LEnt from 9 month non-transgenic and PS19 mice stained with Thioflavin S (ThioS). Scale bar = 20 um. Inserts denote high magnification images of ThioS+ tangles in P301S TIA1+/+ and P301S TIA1+/- LEnt. **Figure 16D****.** Quantification of ThioS fluorescence in C. *p=0.0213 by Student's t-test (n=5/group) **Figure 16E****.** Number of Gallyas Silver+ tangles plotted against the number of Nissl+ neurons in CA3 of the same animal for 9 month P301S TIA1+/+ and P301S TIA1+/- mice (r2 = 0.07).

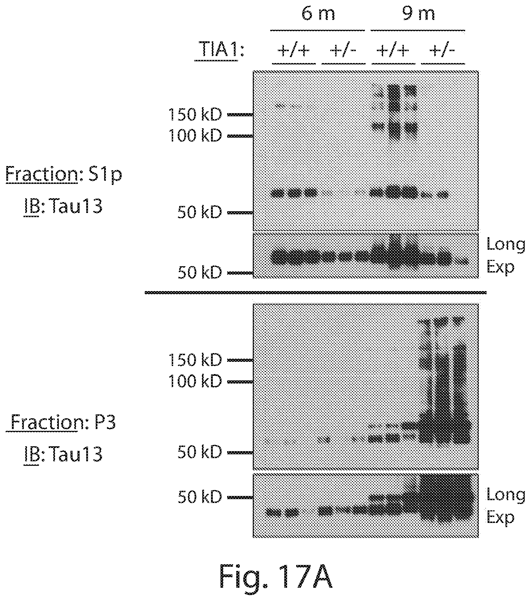

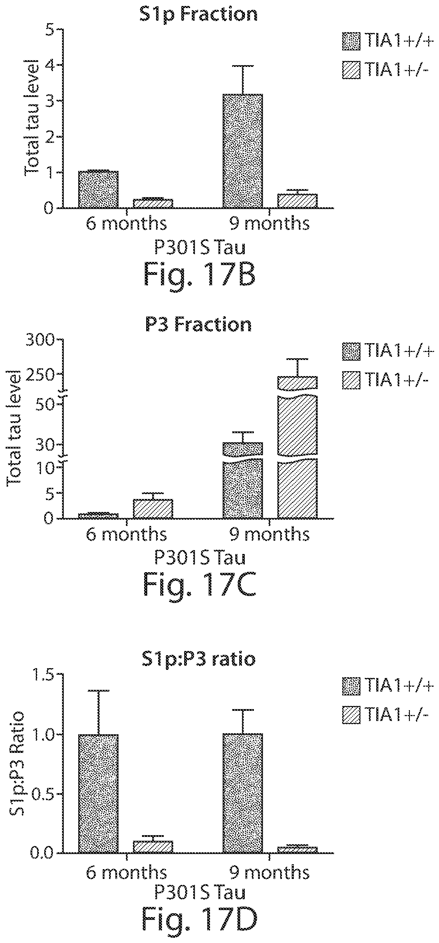

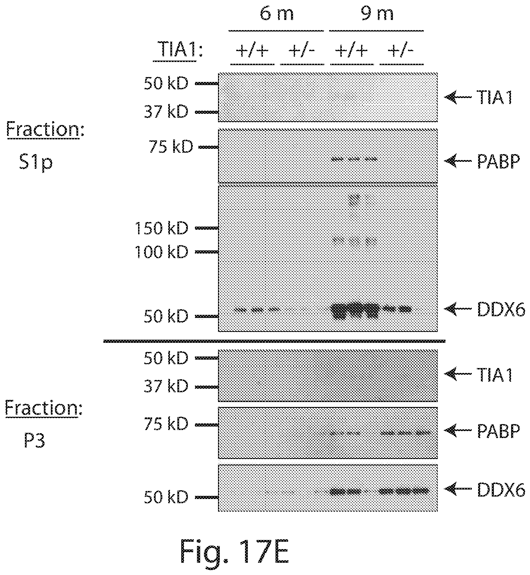

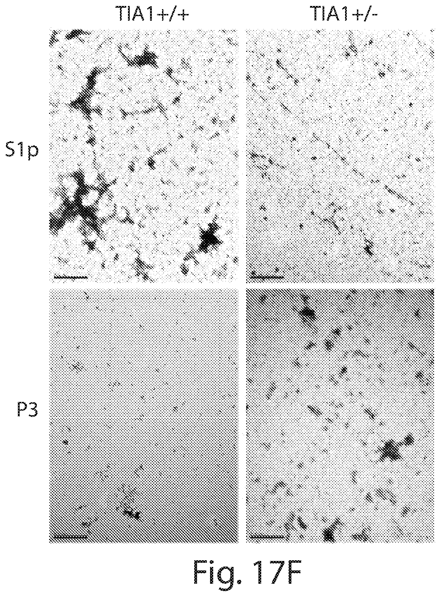

**Figures 17A-17F****. TIA1 reduction shifts the biochemical and structural properties of tau aggregation.** **Figure 17A****.** Immunoblot for total tau (Tau13) in the S1p (top) and P3 (bottom) fractions of 6 and 9 month P301S TIA1+/+ and P301S TIA1+/- cortex. **Figures 17B and 17C****.** Quantification of tau accumulation in the S1p (Fig. 17B) and P3 (**Figure 17C**) fractions from Fig. 17A. *p<0.05 by 2-way ANOVA with Bonferroni post-hoc tests (n=4/group). **Figure 17D****.** Ratio of tau in the S1p versus P3 fraction from A. **p<0.01 by 2-way ANOVA with Bonferroni post-hoc tests (n=4/group). **Figure 17E****.** Immunoblot of TIA1, PABP, and DDX5 in the S1p and P3 fractions of 6 and 9 month P301S TIA1+/+ and P301S TIA1+/- cortex. **Figure 17F****.** Representative transmission electron microscopy images of tau aggregate structure in 9 month P301S TIA1+/+ and P301S TIA1+/- hippocampus.

### DETAILED DESCRIPTION

### Definitions

Unless stated otherwise, or implicit from context, the following terms and phrases include the meanings provided below. Unless explicitly stated otherwise, or apparent from context, the terms and phrases below do not exclude the meaning that the term or phrase has acquired in the art to which it pertains. The definitions are provided to aid in describing particular embodiments, and are not intended to limit the claimed invention, because the scope of the invention is limited only by the claims. Further, unless otherwise required by context, singular terms shall include pluralities and plural terms shall include the singular.

As used herein the term "comprising" or "comprises" is used in reference to compositions, methods, and respective component(s) thereof that are essential to the invention, yet open to the inclusion of unspecified elements, whether essential or not.

As used herein the term "consisting essentially of' refers to those elements required for a given embodiment. The term permits the presence of additional elements that do not materially affect the basic and novel or functional characteristic(s) of that embodiment of the invention.

The term "consisting of' refers to compositions, methods, and respective components thereof as described herein, which are exclusive of any element not recited in that description of the embodiment.

Unless otherwise indicated, all numbers expressing quantities of ingredients or reaction conditions used herein should be understood as modified in all instances by the term "about." The term "about" when used in connection with percentages may mean ±1%.

The singular terms "a," "an," and "the" refer to one or to more than one, unless context clearly indicates otherwise. Similarly, the word "or" is intended to include "and" unless the context clearly indicates otherwise.

Although methods and materials similar or equivalent to those described herein can be used in the practice or testing of this disclosure, suitable methods and materials are described below. The term "comprises" means "includes." The abbreviation, "e.g." is derived from the Latin exempli gratia, and is used herein to indicate a non-limiting example. Thus, the abbreviation "e.g." is synonymous with the term "for example."

The terms "decrease", "reduced", "reduction" , "decrease" or "inhibit" are all used herein generally to mean a decrease by a significant amount. However, for avoidance of doubt, "reduced", "reduction", "decrease" or "inhibit" means a decrease by at least 10% as compared to a reference level, for example a decrease by at least about 20%, or at least about 30%, or at least about 40%, or at least about 50%, or at least about 60%, or at least about 70%, or at least about 80%, or at least about 90% or up to and including a 100% decrease (e.g. absent level as compared to a reference sample), or any decrease between 10-100% as compared to a reference level.

The terms "increased", "increase", "enhance" or "activate" are all used herein to generally mean an increase by a significant amount; for the avoidance of any doubt, the terms "increased", "increase", "enhance" or "activate" means an increase of at least 10% as compared to a reference level, for example an increase of at least about 20%, or at least about 30%, or at least about 40%, or at least about 50%, or at least about 60%, or at least about 70%, or at least about 80%, or at least about 90% or up to and including a 100% increase or any increase between 10-100% as compared to a reference level, or at least about a 2-fold, or at least about a 3-fold, or at least about a 4-fold, or at least about a 5-fold or at least about a 10-fold increase, or any increase between 2-fold and 10-fold or greater as compared to a reference level.

As used herein, the term "administer" refers to the placement of a composition into a subject by a method or route which results in at least partial localization of the composition at a desired site such that desired effect is produced. A nucleic acid based inhibitor or composition described herein can be administered by any appropriate route known in the art including, but not limited to, oral or parenteral routes, including intravenous, intramuscular, subcutaneous, transdermal, airway (aerosol), pulmonary, nasal, rectal, intrathecal, and topical (including buccal and sublingual) administration. Exemplary modes of administration include, but are not limited to, injection, infusion, instillation, inhalation, or ingestion. "Injection" includes, without limitation, intravenous, intramuscular, intraarterial, intrathecal, intraventricular, intracapsular, intraorbital, intracardiac, intradermal, intraperitoneal, transtracheal, subcutaneous, subcuticular, intraarticular, sub capsular, subarachnoid, intraspinal, intracerebro spinal, and intrasternal injection and infusion. In some embodiments, the compositions are administered by intravenous infusion or injection.

By "treatment", "prevention" or "amelioration" of a disease or disorder is meant delaying or preventing the onset of such a disease or disorder, reversing, alleviating, ameliorating, inhibiting, slowing down or stopping the progression, aggravation or deterioration the progression or severity of a condition associated with such a disease or disorder. In one embodiment, at least one symptom of a disease or disorder is alleviated by at least 5%, at least 10%, at least 20%, at least 30%, at least 40%, or at least 50%.

As used herein, the terms "effective" and "effectiveness" includes both pharmacological effectiveness and physiological safety. Pharmacological effectiveness refers to the ability of the treatment to result in a desired biological effect in the patient. Physiological safety refers to the level of toxicity, or other adverse physiological effects at the cellular, organ and/or organism level (often referred to as side-effects) resulting from administration of the treatment. "Less effective" means that the treatment results in a therapeutically significant lower level of pharmacological effectiveness and/or a therapeutically greater level of adverse physiological effects.

As used herein, an amount of a compound or combination effective to treat a disorder (*e.g.,* a disorder as described herein), "therapeutically effective amount", "effective amount" or "effective course" refers to an amount of the compound or combination which is effective, upon single or multiple dose administration(s) to a subject, in treating a subject, or in curing, alleviating, relieving or improving a subject with a disorder (*e.g.,* a disorder as described herein) beyond that expected in the absence of such treatment. Determination of a therapeutically effective amount is well within the capability of those skilled in the art. Generally, a therapeutically effective amount can vary with the subject's history, age, condition, sex, as well as the severity and type of the medical condition in the subject, and administration of other pharmaceutically active agents.

As used herein, a "subject" means a human or animal. Usually the animal is a vertebrate such as a primate, rodent, domestic animal or game animal. Primates include chimpanzees, cynomolgus monkeys, spider monkeys, and macaques, *e.g*., Rhesus. Rodents include mice, rats, woodchucks, ferrets, rabbits and hamsters. Domestic and game animals include cows, horses, pigs, deer, bison, buffalo, feline species, *e.g.,* domestic cat, canine species, *e.g.,* dog, fox, wolf, avian species, *e.g.,* chicken, emu, ostrich, and fish, *e.g.,* trout, catfish and salmon. Patient or subject includes any subset of the foregoing, *e.g.,* all of the above, but excluding one or more groups or species such as humans, primates or rodents. In certain embodiments, the subject is a mammal, *e.g.,* a primate, *e.g.,* a human. The terms, "patient" and "subject" are used interchangeably herein. A subject can be male or female. A subject can be one who has been previously diagnosed with or identified as suffering from or having a neurodegenerative disease or disorder, a disease or disorder associated with cancer, a disease or disorder associated with viral infection, or one or more complications related to such diseases or disorders but need not have already undergone treatment.

The term "nucleic acid" as used herein refers to a polymeric form of nucleotides, either ribonucleotides or deoxynucleotides or a modified form of either type of nucleotide, e.g., modification to the backbone or base. The terms should also be understood to include, as equivalents, analogs of either RNA or DNA made from nucleotide analogs, and, as applicable to the embodiment being described, single-stranded (such as sense or antisense) and doublestranded polynucleotides.

### Nucleic acid based TIA-1 inhibitors

As used herein, a "nucleic acid based inhibitor" is a nucleic acid having sufficient sequence complementarity with a target nucleic acid to hybridize thereto and inhibit the translation of a product of the gene, e.g., by promoting degradation of a target RNA or blocking translation of a target RNA. In some embodiment the nucleic acid based inhibitor is a nucleic acid that can hybridize with an mRNA. In an embodiment, the nucleic acid based inhibitor is a CRISPR RNA.

Nucleic acid-based inhibitors of TIA-1 can be, *e.g.,* double stranded RNA (dsRNA) that function, *e.g*., by an RNA interference (RNAi mechanism), an antisense RNA, or a microRNA (miRNA). In an embodiment the nucleic-acid based inhibitor binds to the target mRNA and inhibits the production of protein therefrom, *e.g.,* by cleavage of the target mRNA or by inhibiting translation, e.g., inhibiting the initiation of translation.

In some embodiments, the nucleic acid based inhibitor, *e.g.,* a dsRNA, preferentially or specifically inhibits the product of a mutant TIA-1 as compared to the product of a wild-type TIA-1. In some embodiments, the nucleic acid based inhibitor, *e.g.,* a dsRNA, preferentially or specifically inhibits the product of a first isoform, e.g., first splice isoform of TIA-1 as compared to the product of a second isoform of TIA-1. In yet another embodiment, the dsRNA targets a particular mutant or polymorphism (such as a single nucleotide polymorphism (SNP)), relative to a second allele, e.g., a wild-type allele. In this case, the nucleic acid based inhibitor, e.g., a dsRNA, can target the region of the TIA-1 containing the mutation or polymorphism. In some embodiments, a dsRNA targets two TIA-1 alleles with equal efficacy, e.g., within about 20%, 15%, 10%, 5%, 2%, or 1% knockdown efficacy. In this case, the nucleic acid based inhibitor, *e.g.,* a dsRNA, can target a region of the TIA-1 without a mutation or polymorphism.

In some embodiments, the nucleic acid based inhibitor is complementary to a region of TIA-1, e.g., a region of the TIA-1 sequence set out in SEQ ID NO: 1. In some embodiments, the TIA-1 sequence region is about 10, 11, 12, 13, 14, 15, 16, 17, 18, 19, 20, 21, 22, 23, 24, 25, 26, 27, 28, 29, 30, 35, 40, 45, or 50 nucleotides in length. In some embodiments, the TIA-1 sequence region begins at position 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 698, 699, 700, 701, 702, 703, 704, 705, 706, 707, 708, 709, 710, 711, 712, 713, 714, 715, 716, 717, 718, 719, 720, 721, 722, 723, 724, 725, 726, 727, 728, 729, 730, 731, 732, 733, 734, 735, 736, 737, 738, 739, 740, 741, 742, 743, 744, 745, 746, 747, 748, 749, 750, 751, 752, 753, 754, 755, 756, 757, 758, 759, 760, 761, 762, 763, 764, 765, 766, 767, 768, 769, 770, 771, 772, 773, 774, 775, 776, 777, 778, 779, 780, 781, 782, 783, 784, 785, 786, 787, 788, 789, 790, 791, 792, 793, 794, 795, 796, 797, 798, 799, 800, 801, 802, 803, 804, 805, 806, 807, 808, 809, 810, 811, 812, 813, 814, 815, 816, 817, 818, 819, 820, 821, 822, 823, 824, 825, 826, 827, 828, 829, 830, 831, 832, 833, 834, 835, 836, 837, 838, 839, 840, 841, 842, 843, 844, 845, 846, 847, 848, 849, 850, 851, 852, 853, 854, 855, 856, 857, 858, 859, 860, 861, 862, 863, 864, 865, 866, 867, 868, 869, 870, 871, 872, 873, 874, 875, 876, 877, 878, 879, 880, 881, 882, 883, 884, 885, 886, 887, 888, 889, 890, 891, 892, 893, 894, 895, 896, 897, 898, 899, 900, 901, 902, 903, 904, 905, 906, 907, 908, 909, 910, 911, 912, 913, 914, 915, 916, 917, 918, 919, 920, 921, 922, 923, 924, 925, 926, 927, 928, 929, 930, 931, 932, 933, 934, 935, 936, 937, 938, 939, 940, 941, 942, 943, 944, 945, 946, 947, 948, 949, 950, 951, 952, 953, 954, 955, 956, 957, 958, 959, 960, 961, 962, 963, 964, 965, 966, 967, 968, 969, 970, 971, 972, 973, 974, 975, 976, 977, 978, 979, 980, 981, 982, 983, 984, 985, 986, 987, 988, 989, 990, 991, 992, 993, 994, 995, 996, 997, 998, 999, 1000, 1001, 1002, 1003, 1004, 1005, 1006, 1007, 1008, 1009, 1010, 1011, 1012, 1013, 1014, 1015, 1016, 1017, 1018, 1019, 1020, 1021, 1022, 1023, 1024, 1025, 1026, 1027, 1028, 1029, 1030, 1031, 1032, 1033, 1034, 1035, 1036, 1037, 1038, 1039, 1040, 1041, 1042, 1043, 1044, 1045, 1046, 1047, 1048, 1049, 1050, 1051, 1052, 1053, 1054, 1055, 1056, 1057, 1058, 1059, 1060, 1061, 1062, 1063, 1064, 1065, 1066, 1067, 1068, 1069, 1070, 1071, 1072, 1073, 1074, 1075, 1076, 1077, 1078, 1079, 1080, 1081, 1082, 1083, 1084, 1085, 1086, 1087, 1088, 1089, 1090, 1091, 1092, 1093, 1094, 1095, 1096, 1097, 1098, 1099, 1100, 1101, 1102, 1103, 1104, 1105, 1106, 1107, 1108, 1109, 1110, 1111, 1112, 1113, 1114, 1115, 1116, 1117, 1118, 1119, 1120, 1121, 1122, 1123, 1124, 1125, 1126, 1127, 1128, 1129, 1130, 1131, 1132, 1133, 1134, 1135, 1136, 1137, 1138, 1139, 1140, 1141, 1142, 1143, 1144, 1145, 1146, 1147, 1148, 1149, 1150, 1151, 1152, 1153, 1154, 1155, 1156, 1157, 1158, 1159, 1160, 1161, 1162, 1163, 1164, 1165, 1166, 1167, 1168, 1169, 1170, 1171, 1172, 1173, 1174, 1175, 1176, 1177, 1178, 1179, 1180, 1181, 1182, 1183, 1184, 1185, 1186, 1187, 1188, 1189, 1190, 1191, 1192, 1193, 1194, 1195, 1196, 1197, 1198, 1199, 1200, 1201, 1202, 1203, 1204, 1205, 1206, 1207, 1208, 1209, 1210, 1211, 1212, 1213, 1214, 1215, 1216, 1217, 1218, 1219, 1220, 1221, 1222, 1223, 1224, 1225, 1226, 1227, 1228, 1229, 1230, 1231, 1232, 1233, 1234, 1235, 1236, 1237, 1238, 1239, 1240, 1241, 1242, 1243, 1244, 1245, 1246, 1247, 1248, 1249, 1250, 1251, 1252, 1253, 1254, 1255, 1256, 1257, 1258, 1259, 1260, 1261, 1262, 1263, 1264, 1265, 1266, 1267, 1268, 1269, 1270, 1271, 1272, 1273, 1274, 1275, 1276, 1277, 1278, 1279, 1280, 1281, 1282, 1283, 1284, 1285, 1286, 1287, 1288, 1289, 1290, 1291, 1292, 1293, 1294, 1295, 1296, 1297, 1298, 1299, 1300, 1301, 1302, 1303, 1304, 1305, 1306, 1307, 1308, 1309,1310,1311,1312,1313,1314,1315,1316,1317,1318,1319,1320,1321,1322,1323, 1324, 1325, 1326, 1327, 1328, 1329, 1330, 1331, 1332, 1333, 1334, 1335, 1336, 1337, 1338, 1339, 1340, 1341, 1342, 1343, 1344, 1345, 1346, 1347, 1348, 1349, 1350, 1351, 1352, 1353, 1354, 1355, 1356, 1357, 1358, 1359, 1360, 1361, 1362, 1363, 1364, 1365, 1366, 1367, 1368, 1369, 1370, 1371, 1372, 1373, 1374, 1375, 1376, 1377, 1378, 1379, 1380, 1381, 1382, 1383, 1384, 1385, 1386, 1387, 1388, 1389, 1390, 1391, 1392, 1393, 1394, 1395, 1396, 1397, 1398, 1399, 1400, 1401, 1402, 1403, 1404, 1405, 1406, 1407, 1408, 1409, 1410, 1411, 1412, 1413, 1414,1415,1416,1417,1418,1419,1420,1421,1422,1423,1424,1425,1426,1427,1428, 1429, 1430, 1431, 1432, 1433, 1434, 1435, 1436, 1437, 1438, 1439, 1440, 1441, 1442, 1443, 1444, 1445, 1446, 1447, 1448, 1449, 1450, 1451, 1452, 1453, 1454, 1455, 1456, 1457, 1458, 1459, 1460, 1461, 1462, 1463, 1464, 1465, 1466, 1467, 1468, 1469, 1470, 1471, 1472, 1473, 1474, 1475, 1476, 1477, 1478, 1479, 1480, 1481, 1482, 1483, 1484, 1485, 1486, 1487, 1488, 1489, 1490, 1491, 1492, 1493, 1494, 1495, 1496, 1497, 1498, 1499, 1500, 1501, 1502, 1503, 1504, 1505, 1506, 1507, 1508, 1509, 1510, 1511, 1512, 1513, 1514, 1515, 1516, 1517, 1518, 1519, 1520, 1521, 1522, 1523, 1524, 1525, 1526, 1527, 1528, 1529, 1530, 1531, 1532, 1533, 1534, 1535, 1536, 1537, 1538, 1539, 1540, 1541, 1542, 1543, 1544, 1545, 1546, 1547, 1548, 1549, 1550, 1551, 1552, 1553, 1554, 1555, 1556, 1557, 1558, 1559, 1560, 1561, 1562, 1563, 1564, 1565, 1566, 1567, 1568, 1569, 1570, 1571, 1572, 1573, 1574, 1575, 1576, 1577, 1578, 1579, 1580, 1581, 1582, 1583, 1584, 1585, 1586, 1587, 1588, 1589, 1590, 1591, 1592, 1593, 1594, 1595, 1596, 1597, 1598, 1599, 1600, 1601, 1602, 1603, 1604, 1605, 1606, 1607, 1608, 1609, 1610, 1611, 1612, 1613, 1614, 1615, 1616, 1617, 1618, 1619, 1620, 1621, 1622, 1623, 1624, 1625, 1626, 1627, 1628, 1629, 1630, 1631, 1632, 1633, 1634, 1635, 1636, 1637, 1638, 1639, 1640, 1641, 1642, 1643, 1644, 1645, 1646, 1647, 1648, 1649, 1650, 1651, 1652, 1653, 1654, 1655, 1656, 1657, 1658, 1659, 1660, 1661, 1662, 1663, 1664, 1665, 1666, 1667, 1668, 1669, 1670, 1671, 1672, 1673, 1674, 1675, 1676, 1677, 1678, 1679, 1680, 1681, 1682, 1683, 1684, 1685, 1686, 1687, 1688, 1689, 1690, 1691, 1692, 1693, 1694, 1695, 1696, 1697, 1698, 1699, 1700, 1701, 1702, 1703, 1704, 1705, 1706, 1707, 1708, 1709, 1710, 1711, 1712, 1713, 1714, 1715, 1716, 1717, 1718, 1719, 1720, 1721, 1722, 1723, 1724, 1725, 1726, 1727, 1728, 1729, 1730, 1731, 1732, 1733, 1734, 1735, 1736, 1737, 1738, 1739, 1740, 1741, 1742, 1743, 1744,1745,1746,1747,1748,1749,1750,1751,1752,1753,1754,1755,1756,1757,1758, 1759, 1760, 1761, 1762, 1763, 1764, 1765, 1766, 1767, 1768, 1769, 1770, 1771, 1772, 1773, 1774, 1775, 1776, 1777, 1778, 1779, 1780, 1781, 1782, 1783, 1784, 1785, 1786, 1787, 1788, 1789, 1790, 1791, 1792, 1793, 1794, 1795, 1796, 1797, 1798, 1799, 1800, 1801, 1802, 1803, 1804, 1805, 1806, 1807, 1808, 1809, 1810, 1811, 1812, 1813, 1814, 1815, 1816, 1817, 1818, 1819, 1820, 1821, 1822, 1823, 1824, 1825, 1826, 1827, 1828, 1829, 1830, 1831, 1832, 1833, 1834, 1835, 1836, 1837, 1838, 1839, 1840, 1841, 1842, 1843, 1844, 1845, 1846, 1847, 1848, 1849, 1850, 1851, 1852, 1853, 1854, 1855, 1856, 1857, 1858, 1859, 1860, 1861, 1862, 1863, 1864, 1865, 1866, 1867, 1868, 1869, 1870, 1871, 1872, 1873, 1874, 1875, 1876, 1877, 1878, 1879, 1880, 1881, 1882, 1883, 1884, 1885, 1886, 1887, 1888, 1889, 1890, 1891, 1892, 1893, 1894, 1895, 1896, 1897, 1898, 1899, 1900, 1901, 1902, 1903, 1904, 1905, 1906, 1907, 1908, 1909, 1910, 1911, 1912, 1913, 1914, 1915, 1916, 1917, 1918, 1919, 1920, 1921, 1922, 1923, 1924, 1925, 1926, 1927, 1928, 1929, 1930, 1931, 1932, 1933, 1934, 1935, 1936, 1937, 1938, 1939, 1940, 1941, 1942, 1943, 1944, 1945, 1946, 1947, 1948, 1949, 1950, 1951, 1952, 1953, 1954, 1955, 1956, 1957, 1958, 1959, 1960, 1961, 1962, 1963, 1964, 1965, 1966, 1967, 1968, 1969, 1970, 1971, 1972, 1973, 1974, 1975, 1976, 1977, 1978, 1979, 1980, 1981, 1982, 1983, 1984, 1985, 1986, 1987, 1988, 1989, 1990, 1991, 1992, 1993, 1994, 1995, 1996, 1997, 1998, 1999, 2000, 2001, 2002, 2003, 2004, 2005, 2006, 2007, 2008, 2009, 2010, 2011, 2012, 2013, 2014, 2015, 2016, 2017, 2018, 2019, 2020, 2021, 2022, 2023, 2024, 2025, 2026, 2027, 2028, 2029, 2030, 2031, 2032, 2033, 2034, 2035, 2036, 2037, 2038, 2039, 2040, 2041, 2042, 2043, 2044, 2045, 2046, 2047, 2048, 2049, 2050, 2051, 2052, 2053, 2054, 2055, 2056, 2057, 2058, 2059, 2060, 2061, 2062, 2063, 2064, 2065, 2066, 2067, 2068, 2069, 2070, 2071, 2072, 2073, 2074, 2075, 2076, 2077, 2078, 2079, 2080, 2081, 2082, 2083, 2084, 2085, 2086, 2087, 2088, 2089, 2090, 2091, 2092, 2093, 2094, 2095, 2096, 2097, 2098, 2099, 2100, 2101, 2102, 2103, 2104, 2105, 2106, 2107, 2108, 2109, 2110, 2111, 2112, 2113, 2114, 2115, 2116, 2117, 2118, 2119, 2120, 2121, 2122, 2123, 2124, 2125, 2126, 2127, 2128, 2129, 2130, 2131, 2132, 2133, 2134,2135,2136,2137,2138,2139,2140,2141,2142,2143,2144,2145,2146,2147,2148, 2149, 2150, 2151, 2152, 2153, 2154, 2155, 2156, 2157, 2158, 2159, 2160, 2161, 2162, 2163, 2164, 2165, 2166, 2167, 2168, 2169, 2170, 2171, 2172, 2173, 2174, 2175, 2176, 2177, 2178, 2179, 2180, 2181, 2182, 2183, 2184, 2185, 2186, 2187, 2188, 2189, 2190, 2191, 2192, 2193, 2194, 2195, 2196, 2197, 2198, 2199, 2200, 2201, 2202, 2203, 2204, 2205, 2206, 2207, 2208, 2209, 2210, 2211, 2212, 2213, 2214, 2215, 2216, 2217, 2218, 2219, 2220, 2221, 2222, 2223, 2224, 2225, 2226, 2227, 2228, 2229, 2230, 2231, 2232, 2233, 2234, 2235, 2236, 2237, 2238, 2239, 2240, 2241, 2242, 2243, 2244, 2245, 2246, 2247, 2248, 2249, 2250, 2251, 2252, 2253, 2254, 2255, 2256, 2257, 2258, 2259, 2260, 2261, 2262, 2263, 2264, 2265, 2266, 2267, 2268, 2269, 2270, 2271, 2272, 2273, 2274, 2275, 2276, 2277, 2278, 2279, 2280, 2281, 2282, 2283, 2284, 2285, 2286, 2287, 2288, 2289, 2290, 2291, 2292, 2293, 2294, 2295, 2296, 2297, 2298, 2299, 2300, 2301, 2302, 2303, 2304, 2305, 2306, 2307, 2308, 2309, 2310, 2311, 2312, 2313, 2314, 2315, 2316, 2317, 2318, 2319, 2320, 2321, 2322, 2323, 2324, 2325, 2326, 2327, 2328, 2329, 2330, 2331, 2332, 2333, 2334, 2335, 2336, 2337, 2338, 2339, 2340, 2341, 2342, 2343, 2344, 2345, 2346, 2347, 2348, 2349, 2350, 2351, 2352, 2353, 2354, 2355, 2356, 2357, 2358, 2359, 2360, 2361, 2362, 2363, 2364, 2365, 2366, 2367, 2368, 2369, 2370, 2371, 2372, 2373, 2374, 2375, 2376, 2377, 2378, 2379, 2380, 2381, 2382, 2383, 2384, 2385, 2386, 2387, 2388, 2389, 2390, 2391, 2392, 2393, 2394, 2395, 2396, 2397, 2398, 2399, 2400, 2401, 2402, 2403, 2404, 2405, 2406, 2407, 2408, 2409, 2410, 2411, 2412, 2413, 2414, 2415, 2416, 2417, 2418, 2419, 2420, 2421, 2422, 2423, 2424, 2425, 2426, 2427, 2428, 2429, 2430, 2431, 2432, 2433, 2434,2435,2436,2437,2438,2439,2440,2441,2442,2443,2444,2445,2446,2447,2448, 2449, 2450, 2451, 2452, 2453, 2454, 2455, 2456, 2457, 2458, 2459, 2460, 2461, 2462, 2463, 2464, 2465, 2466, 2467, 2468, 2469, 2470, 2471, 2472, 2473, 2474, 2475, 2476, 2477, 2478, 2479, 2480, 2481, 2482, 2483, 2484, 2485, 2486, 2487, 2488, 2489, 2490, 2491, 2492, 2493, 2494, 2495, 2496, 2497, 2498, 2499, 2500, 2501, 2502, 2503, 2504, 2505, 2506, 2507, 2508, 2509, 2510, 2511, 2512, 2513, 2514, 2515, 2516, 2517, 2518, 2519, 2520, 2521, 2522, 2523, 2524,2525,2526,2527,2528,2529,2530,2531,2532,2533,2534,2535,2536,2537,2538, 2539, 2540, 2541, 2542, 2543, 2544, 2545, 2546, 2547, 2548, 2549, 2550, 2551, 2552, 2553, 2554, 2555, 2556, 2557, 2558, 2559, 2560, 2561, 2562, 2563, 2564, 2565, 2566, 2567, 2568, 2569, 2570, 2571, 2572, 2573, 2574, 2575, 2576, 2577, 2578, 2579, 2580, 2581, 2582, 2583, 2584, 2585, 2586, 2587, 2588, 2589, 2590, 2591, 2592, 2593, 2594, 2595, 2596, 2597, 2598, 2599, 2600, 2601, 2602, 2603, 2604, 2605, 2606, 2607, 2608, 2609, 2610, 2611, 2612, 2613, 2614, 2615, 2616, 2617, 2618, 2619, 2620, 2621, 2622, 2623, 2624, 2625, 2626, 2627, 2628, 2629, 2630, 2631, 2632, 2633, 2634, 2635, 2636, 2637, 2638, 2639, 2640, 2641, 2642, 2643, 2644,2645,2646,2647,2648,2649,2650,2651,2652,2653,2654,2655,2656,2657,2658, 2659, 2660, 2661, 2662, 2663, 2664, 2665, 2666, 2667, 2668, 2669, 2670, 2671, 2672, 2673, 2674, 2675, 2676, 2677, 2678, 2679, 2680, 2681, 2682, 2683, 2684, 2685, 2686, 2687, 2688, 2689, 2690, 2691, 2692, 2693, 2694, 2695, 2696, 2697, 2698, 2699, 2700, 2701, 2702, 2703, 2704, 2705, 2706, 2707, 2708, 2709, 2710, 2711, 2712, 2713, 2714, 2715, 2716, 2717, 2718, 2719, 2720, 2721, 2722, 2723, 2724, 2725, 2726, 2727, 2728, 2729, 2730, 2731, 2732, 2733, 2734, 2735, 2736, 2737, 2738, 2739, 2740, 2741, 2742, 2743, 2744, 2745, 2746, 2747, 2748, 2749, 2750, 2751, 2752, 2753, 2754, 2755, 2756, 2757, 2758, 2759, 2760, 2761, 2762, 2763, 2764, 2765, 2766, 2767, 2768, 2769, 2770, 2771, 2772, 2773, 2774, 2775, 2776, 2777, 2778, 2779, 2780, 2781, 2782, 2783, 2784, 2785, 2786, 2787, 2788, 2789, 2790, 2791, 2792, 2793, 2794, 2795, 2796, 2797, 2798, 2799, 2800, 2801, 2802, 2803, 2804, 2805, 2806, 2807, 2808, 2809, 2810, 2811, 2812, 2813, 2814, 2815, 2816, 2817, 2818, 2819, 2820, 2821, 2822, 2823, 2824, 2825, 2826, 2827, 2828, 2829, 2830, 2831, 2832, 2833, 2834, 2835, 2836, 2837, 2838, 2839, 2840, 2841, 2842, 2843, 2844, 2845, 2846, 2847, 2848, 2849, 2850, 2851, 2852, 2853, 2854, 2855, 2856, 2857, 2858, 2859, 2860, 2861, 2862, 2863, 2864, 2865, 2866, 2867, 2868, 2869, 2870, 2871, 2872, 2873, 2874, 2875, 2876, 2877, 2878, 2879, 2880, 2881, 2882, 2883, 2884, 2885, 2886, 2887, 2888, 2889, 2890, 2891, 2892, 2893, 2894, 2895, 2896, 2897, 2898, 2899, 2900, 2901, 2902, 2903, 2904, 2905, 2906, 2907, 2908, 2909, 2910, 2911, 2912, 2913, 2914, 2915, 2916, 2917, 2918, 2919, 2920, 2921, 2922, 2923, 2924, 2925, 2926, 2927, 2928, 2929, 2930, 2931, 2932, 2933, 2934, 2935, 2936, 2937, 2938, 2939, 2940, 2941, 2942, 2943, 2944,2945,2946,2947,2948,2949,2950,2951,2952,2953,2954,2955,2956,2957,2958, 2959, 2960, 2961, 2962, 2963, 2964, 2965, 2966, 2967, 2968, 2969, 2970, 2971, 2972, 2973, 2974, 2975, 2976, 2977, 2978, 2979, 2980, 2981, 2982, 2983, 2984, 2985, 2986, 2987, 2988, 2989, 2990, 2991, 2992, 2993, 2994, 2995, 2996, 2997, 2998, 2999, 3000, 3001, 3002, 3003, 3004, 3005, 3006, 3007, 3008, 3009, 3010, 3011, 3012, 3013, 3014, 3015, 3016, 3017, 3018, 3019, 3020, 3021, 3022, 3023, 3024, 3025, 3026, 3027, 3028, 3029, 3030, 3031, 3032, 3033, 3034, 3035, 3036, 3037, 3038, 3039, 3040, 3041, 3042, 3043, 3044, 3045, 3046, 3047, 3048, 3049, 3050, 3051, 3052, 3053, 3054, 3055, 3056, 3057, 3058, 3059, 3060, 3061, 3062, 3063, 3064, 3065, 3066, 3067, 3068, 3069, 3070, 3071, 3072, 3073, 3074, 3075, 3076, 3077, 3078, 3079, 3080, 3081, 3082, 3083, 3084, 3085, 3086, 3087, 3088, 3089, 3090, 3091, 3092, 3093, 3094, 3095, 3096, 3097, 3098, 3099, 3100, 3101, 3102, 3103, 3104, 3105, 3106, 3107, 3108, 3109,3110,3111,3112,3113,3114,3115,3116,3117,3118,3119,3120,3121,3122,3123, 3124, 3125, 3126, 3127, 3128, 3129, 3130, 3131, 3132, 3133, 3134, 3135, 3136, 3137, 3138, 3139, 3140, 3141, 3142, 3143, 3144, 3145, 3146, 3147, 3148, 3149, 3150, 3151, 3152, 3153, 3154, 3155, 3156, 3157, 3158, 3159, 3160, 3161, 3162, 3163, 3164, 3165, 3166, 3167, 3168, 3169, 3170, 3171, 3172, 3173, 3174, 3175, 3176, 3177, 3178, 3179, 3180, 3181, 3182, 3183, 3184, 3185, 3186, 3187, 3188, 3189, 3190, 3191, 3192, 3193, 3194, 3195, 3196, 3197, 3198, 3199, 3200, 3201, 3202, 3203, 3204, 3205, 3206, 3207, 3208, 3209, 3210, 3211, 3212, 3213, 3214, 3215, 3216, 3217, 3218, 3219, 3220, 3221, 3222, 3223, 3224, 3225, 3226, 3227, 3228, 3229, 3230, 3231, 3232, 3233, 3234, 3235, 3236, 3237, 3238, 3239, 3240, 3241, 3242, 3243, 3244, 3245, 3246, 3247, 3248, 3249, 3250, 3251, 3252, 3253, 3254, 3255, 3256, 3257, 3258, 3259, 3260, 3261, 3262, 3263, 3264, 3265, 3266, 3267, 3268, 3269, 3270, 3271, 3272, 3273, 3274, 3275, 3276, 3277, 3278, 3279, 3280, 3281, 3282, 3283, 3284, 3285, 3286, 3287, 3288, 3289, 3290, 3291, 3292, 3293, 3294, 3295, 3296, 3297, 3298, 3299, 3300, 3301, 3302, 3303, 3304, 3305, 3306, 3307, 3308, 3309, 3310, 3311, 3312, 3313, 3314, 3315, 3316, 3317, 3318, 3319, 3320, 3321, 3322, 3323, 3324, 3325, 3326, 3327, 3328, 3329, 3330, 3331, 3332, 3333, 3334, 3335, 3336, 3337, 3338, 3339, 3340, 3341, 3342, 3343, 3344, 3345, 3346, 3347, 3348, 3349, 3350, 3351, 3352, 3353, 3354, 3355, 3356, 3357, 3358, 3359, 3360, 3361, 3362, 3363, 3364, 3365, 3366, 3367, 3368, 3369, 3370, 3371, 3372, 3373, 3374, 3375, 3376, 3377, 3378, 3379, 3380, 3381, 3382, 3383, 3384, 3385, 3386, 3387, 3388, 3389, 3390, 3391, 3392, 3393, 3394, 3395, 3396, 3397, 3398, 3399, 3400, 3401, 3402, 3403, 3404, 3405, 3406, 3407, 3408, 3409, 3410, 3411, 3412, 3413, 3414, 3415, 3416, 3417, 3418, 3419, 3420, 3421, 3422, 3423, 3424, 3425, 3426, 3427, 3428, 3429, 3430, 3431, 3432, 3433, 3434, 3435, 3436, 3437, 3438, 3439, 3440, 3441, 3442, 3443, 3444, 3445, 3446, 3447, 3448, 3449, 3450, 3451, 3452, 3453, 3454, 3455, 3456, 3457, 3458, 3459, 3460, 3461, 3462, 3463, 3464, 3465, 3466, 3467, 3468, 3469, 3470, 3471, 3472, 3473, 3474, 3475, 3476, 3477, 3478, 3479, 3480, 3481, 3482, 3483, 3484, 3485, 3486, 3487, 3488, 3489, 3490, 3491, 3492, 3493, 3494, 3495, 3496, 3497, 3498, 3499, 3500, 3501, 3502, 3503, 3504, 3505, 3506, 3507, 3508, 3509, 3510, 3511, 3512, 3513, 3514, 3515, 3516, 3517, 3518, 3519, 3520, 3521, 3522, 3523, 3524, 3525, 3526, 3527, 3528, 3529, 3530, 3531, 3532, 3533, 3534, 3535, 3536, 3537, 3538, 3539, 3540, 3541, 3542, 3543, 3544, 3545, 3546, 3547, 3548, 3549, 3550, 3551, 3552, 3553, 3554, 3555, 3556, 3557, 3558, 3559, 3560, 3561, 3562, 3563, 3564, 3565, 3566, 3567, 3568, 3569, 3570, 3571, 3572, 3573, 3574, 3575, 3576, 3577, 3578, 3579, 3580, 3581, 3582, 3583, 3584, 3585, 3586, 3587, 3588, 3589, 3590, 3591, 3592, 3593, 3594, 3595, 3596, 3597, 3598, 3599, 3600, 3601, 3602, 3603, 3604, 3605, 3606, 3607, 3608, 3609, 3610, 3611, 3612, 3613, 3614, 3615, 3616, 3617, 3618, 3619, 3620, 3621, 3622, 3623, 3624, 3625, 3626, 3627, 3628, 3629, 3630, 3631, 3632, 3633, 3634, 3635, 3636, 3637, 3638, 3639, 3640, 3641, 3642, 3643, 3644, 3645, 3646, 3647, 3648, 3649, 3650, 3651, 3652, 3653, 3654, 3655, 3656, 3657, 3658, 3659, 3660, 3661, 3662, 3663, 3664, 3665, 3666, 3667, 3668, 3669, 3670, 3671, 3672, 3673, 3674, 3675, 3676, 3677, 3678, 3679, 3680, 3681, 3682, 3683, 3684, 3685, 3686, 3687, 3688, 3689, 3690, 3691, 3692, 3693, 3694, 3695, 3696, 3697, 3698, 3699, 3700, 3701, 3702, 3703, 3704, 3705, 3706, 3707, 3708, 3709, 3710, 3711, 3712, 3713, 3714, 3715, 3716, 3717, 3718, 3719, 3720, 3721, 3722, 3723, 3724, 3725, 3726, 3727, 3728, 3729, 3730, 3731, 3732, 3733, 3734, 3735, 3736, 3737, 3738, 3739, 3740, 3741, 3742, 3743, 3744, 3745, 3746, 3747, 3748, 3749, 3750, 3751, 3752, 3753, 3754, 3755, 3756, 3757, 3758, 3759, 3760, 3761, 3762, 3763, 3764, 3765, 3766, 3767, 3768, 3769, 3770, 3771, 3772, 3773, 3774, 3775, 3776, 3777, 3778, 3779, 3780, 3781, 3782, 3783, 3784, 3785, 3786, 3787, 3788, 3789, 3790, 3791, 3792, 3793, 3794, 3795, 3796, 3797, 3798, 3799, 3800, 3801, 3802, 3803, 3804, 3805, 3806, 3807, 3808, 3809, 3810, 3811, 3812, 3813, 3814, 3815, 3816, 3817, 3818, 3819, 3820, 3821, 3822, 3823, 3824, 3825, 3826, 3827, 3828, 3829, 3830, 3831, 3832, 3833, 3834, 3835, 3836, 3837, 3838, 3839, 3840, 3841, 3842, 3843, 3844, 3845, 3846, 3847, 3848, 3849, 3850, 3851, 3852, 3853, 3854, 3855, 3856, 3857, 3858, 3859, 3860, 3861, 3862, 3863, 3864, 3865, 3866, 3867, 3868, 3869, 3870, 3871, 3872, 3873, 3874, 3875, 3876, 3877, 3878, 3879, 3880, 3881, 3882, 3883, 3884, 3885, 3886, 3887, 3888, 3889, 3890, 3891, 3892, 3893, 3894, 3895, 3896, 3897, 3898, 3899, 3900, 3901, 3902, 3903, 3904, 3905, 3906, 3907, 3908, 3909, 3910, 3911, 3912, 3913, 3914, 3915, 3916, 3917, 3918, 3919, 3920, 3921, 3922, 3923, 3924, 3925, 3926, 3927, 3928, 3929, 3930, 3931, 3932, 3933, 3934, 3935, 3936, 3937, 3938, 3939, 3940, 3941, 3942, 3943, 3944, 3945, 3946, 3947, 3948, 3949, 3950, 3951, 3952, 3953, 3954, 3955, 3956, 3957, 3958, 3959, 3960, 3961, 3962, 3963, 3964, 3965, 3966, 3967, 3968, 3969, 3970, 3971, 3972, 3973, 3974, 3975, 3976, 3977, 3978, 3979, 3980, 3981, 3982, 3983, 3984, 3985, 3986, 3987, 3988, 3989, 3990, 3991, 3992, 3993, 3994, 3995, 3996, 3997, 3998, 3999, 4000, 4001, 4002, 4003, 4004, 4005, 4006, 4007, 4008, 4009, 4010, 4011, 4012, 4013, 4014, 4015, 4016, 4017, 4018, 4019, 4020, 4021, 4022, 4023, 4024, 4025, 4026, 4027, 4028, 4029, 4030, 4031, 4032, 4033, 4034, 4035, 4036, 4037, 4038, 4039, 4040, 4041, 4042, 4043, 4044, 4045, 4046, 4047, 4048, 4049, 4050, 4051, 4052, 4053, 4054, 4055, 4056, 4057, 4058, 4059, 4060, 4061, 4062, 4063, 4064, 4065, 4066, 4067, 4068, 4069, 4070, 4071, 4072, 4073, 4074, 4075, 4076, 4077, 4078, 4079, 4080, 4081, 4082, 4083, 4084, 4085, 4086, 4087, 4088, 4089, 4090, 4091, 4092, 4093, 4094, 4095, 4096, 4097, 4098, 4099, 4100, 4101, 4102, 4103, 4104, 4105, 4106, 4107, 4108, 4109, 4110, 4111, 4112, 4113, 4114, 4115, 4116, 4117, 4118, 4119, 4120, 4121, 4122, 4123, 4124, 4125, 4126, 4127, 4128, 4129, 4130, 4131, 4132, 4133, 4134, 4135, 4136, 4137, 4138, 4139, 4140, 4141, 4142, 4143, 4144, 4145, 4146, 4147, 4148, 4149, 4150, 4151, 4152, 4153, 4154, 4155, 4156, 4157, 4158, 4159, 4160, 4161, 4162, 4163, 4164, 4165, 4166, 4167, 4168, 4169, 4170, 4171, 4172, 4173, 4174, 4175, 4176, 4177, 4178, 4179, 4180, 4181, 4182, 4183, 4184, 4185, 4186, 4187, 4188, 4189, 4190, 4191, 4192, 4193, 4194, 4195, 4196, 4197, 4198, 4199, 4200, 4201, 4202, 4203, 4204, 4205, 4206, 4207, 4208, 4209, 4210, 4211, 4212, 4213, 4214, 4215, 4216, 4217, 4218, 4219, 4220, 4221, 4222, 4223, 4224, 4225, 4226, 4227, 4228, 4229, 4230, 4231, 4232, 4233, 4234, 4235, 4236, 4237, 4238, 4239, 4240, 4241, 4242, 4243, 4244, 4245, 4246, 4247, 4248, 4249, 4250, 4251, 4252, 4253, 4254, 4255, 4256, 4257, 4258, 4259, 4260, 4261, 4262, 4263, 4264, 4265, 4266, 4267, 4268, 4269, 4270, 4271, 4272, 4273, 4274, 4275, 4276, 4277, 4278, 4279, 4280, 4281, 4282, 4283, 4284, 4285, 4286, 4287, 4288, 4289, 4290, 4291, 4292, 4293, 4294, 4295, 4296, 4297, 4298, 4299, 4300, 4301, 4302, 4303, 4304, 4305, 4306, 4307, 4308, 4309, 4310, 4311, 4312, 4313, 4314, 4315, 4316, 4317, 4318, 4319, 4320, 4321, 4322, 4323, 4324, 4325, 4326, 4327, 4328, 4329, 4330, 4331, 4332, 4333, 4334, 4335, 4336, 4337, 4338, 4339, 4340, 4341, 4342, 4343, 4344, 4345, 4346, 4347, 4348, 4349, 4350, 4351, 4352, 4353, 4354, 4355, 4356, 4357, 4358, 4359, 4360, 4361, 4362, 4363, 4364, 4365, 4366, 4367, 4368, 4369, 4370, 4371, 4372, 4373, 4374, 4375, 4376, 4377, 4378, 4379, 4380, 4381, 4382, 4383, 4384, 4385, 4386, 4387, 4388, 4389, 4390, 4391, 4392, 4393, 4394, 4395, 4396, 4397, 4398, 4399, 4400, 4401, 4402, 4403, 4404, 4405, 4406, 4407, 4408, 4409, 4410, 4411, 4412, 4413, 4414, 4415, 4416, 4417, 4418, 4419, 4420, 4421, 4422, 4423, 4424, 4425, 4426, 4427, 4428, 4429, 4430, 4431, 4432, 4433, 4434, 4435, 4436, 4437, 4438, 4439, 4440, 4441, 4442, 4443, 4444, 4445, 4446, 4447, 4448, 4449, 4450, 4451, 4452, 4453, 4454, 4455, 4456, 4457, 4458, 4459, 4460, 4461, 4462, 4463, 4464, 4465, 4466, 4467, 4468, 4469, 4470, 4471, 4472, 4473, 4474, 4475, 4476, 4477, 4478, 4479, 4480, 4481, 4482, 4483, 4484, 4485, 4486, 4487, 4488, 4489, 4490, 4491, 4492, 4493, 4494, 4495, 4496, 4497, 4498, 4499, 4500, 4501, 4502, 4503, 4504, 4505, 4506, 4507, 4508, 4509, 4510, 4511, 4512, 4513, 4514, 4515, 4516, 4517, 4518, 4519, 4520, 4521, 4522, 4523, 4524, 4525, 4526, 4527, 4528, 4529, 4530, 4531, 4532, 4533, 4534, 4535, 4536, 4537, 4538, 4539, 4540, 4541, 4542, 4543, 4544, 4545, 4546, 4547, 4548, 4549, 4550, 4551, 4552, 4553, 4554, 4555, 4556, 4557, 4558, 4559, 4560, 4561, 4562, 4563, 4564, 4565, 4566, 4567, 4568, 4569, 4570, 4571, 4572, 4573, 4574, 4575, 4576, 4577, 4578, 4579, 4580, 4581, 4582, 4583, 4584, 4585, 4586, 4587, 4588, 4589, 4590, 4591, 4592, 4593, 4594, 4595, 4596, 4597, 4598, 4599, or 4600, of TIA-1, e.g., of the TIA-1 sequence set out in SEQ ID NO: 1. In some embodiments, the TIA-1 sequence region is disposed in a 5'UTR, 3' UTR, intron, or exon of TIA-1. In some embodiments, the TIA-1 sequence region overlaps a boundary between a 5'UTR and coding region, 3' UTR and coding region, intron and exon (e.g., in a pre-mRNA), or exon and exon (e.g., in spliced mRNA).

In some embodiments, the nucleic acid based inhibitor is more than 50% AU or AT, e.g., is at least 51%, 52%, 53%, 54%, 55%, 56%, 57%, 58%, 59%, 60%, 61%, 62%, 63%, 64%, 65%, 66%, 67%, 68%, 69%, or 70% A or T or U nucleotides. In some embodiments, the nucleic acid based inhibitor is more than 50% GC, e.g., is at least 51%, 52%, 53%, 54%, 55%, 56%, 57%, 58%, 59%, 60%, 61%, 62%, 63%, 64%, 65%, 66%, 67%, 68%, 69%, or 70% G or C nucleotides.

The TIA-1 gene is sometimes referred to as TIA1, T-Cell-Restricted Intracellular Antigen-1, TIA1 Cytotoxic Granule-Associated RNA Binding Protein, or WDM. The TIA-1 protein has RNA-binding activity and possesses nucleolytic activity against cytotoxic lymphocyte target cells. The TIA-1 gene encodes an approximately 40 kDa protein that undergoes proteolytic processing to yield a 15 kDa mature form.

In some embodiments, the sequence of the human TIA-1 gene is the sequence set out in GenBank Accession number NG_029967 (46204 bp) entry dated 11-MAY-2015, which is herein incorporated by reference in its entirety. In some embodiments, the sequence of the human TIA-1 gene is the isoform 1 sequence set out in GenBank Accession number NM_022037.2 and provided herein as SEQ ID NO: 1:

In SEQ ID NO: 1, nucleotides 1526-1545, which form the binding site for the shRNA of SEQ ID NO: 4, are underlined.

In some embodiments, the sequence of the human TIA-1 gene is the isoform 2 sequence set out in GenBank Accession number NM_022173.2, dated 15-MAR-2015, which is incorporated by reference in its entirety.

In some embodiments, the sequence of the human TIA-1 mRNA is an mRNA sequence encoding a splice isoform of the TIA-1 protein as set out below, wherein the sequence identified as SPIP31483 is SEQ ID NO: 5, the sequence identified as SPIP31483-2 is SEQ ID NO: 6, and the sequence identified as SPIP31483-3 is SEQ ID NO: 7.

In some embodiments, the TIA-1 nucleic acid based inhibitor comprises the short hairpin sequence of SEQ ID NO: 4:
CCGGCGATGCTGGATGTCTGCCAATCTCGAGATTGGCAGACATCCAGCATCGTTTTTG

The underlined sequences of SEQ ID NO: 4 indicate the guide and passenger strands, which are provided below as SEQ ID NOs. 2 and 3.

The guide strand sequence of the hairpin of SEQ ID NO: 4 is provided herein as SEQ ID NO: 2: ATTGGCAGACATCCAGCATC.

The passenger strand sequence of the hairpin of SEQ ID NO: 4 is provided herein as SEQ ID NO: 3: GATGCTGGATGTCTGCCAAT.

In some embodiments, the TIA-1 nucleic acid based inhibitor comprises a short hairpin sequence of SEQ ID NO: 8:

### CCGGCGATGGTGGATGTTTGCCAATCTCGAGATTGGCAAACATCCACCATCGTTTTTG

The underlined sequences of SEQ ID NO: 8 indicate the guide and passenger strands, which are provided below as SEQ ID NOs. 9 and 10. The shRNA of SEQ ID NO: 8 is capable of hybridizing with perfect complementarity to a mouse TIA-1 nucleic acid, e.g., the TIA-1 transcript variant 1 mRNA deposited in Genbank under Accession Number NM_011585, 15-FEB-2015, which is herein incorporated by reference. The shRNA of SEQ ID NO: 8 is capable of hybridizing, e.g., with two mismatches, to a human TIA-1 nucleic acid.

The guide strand sequence of the hairpin of SEQ ID NO: 8 is provided herein as SEQ ID NO: 9: ATTGGCAAACATCCACCATC.

The passenger strand sequence of the hairpin of SEQ ID NO: 8 is provided herein as SEQ ID NO: 10: GATGGTGGATGTTTGCCAAT.

### Double stranded RNA (dsRNA)

A nucleic acid based inhibitor useful for decreasing TIA-1 function can be, *e.g.,* a dsRNA, such as a dsRNA that acts by an RNAi mechanism. RNAi refers to the process of sequence-specific post-transcriptional gene silencing in subjects, e.g., animals, mediated by short interfering RNAs (siRNAs). dsRNAs as used herein are understood to include siRNAs. Typically, inhibition of TIA-1 by dsRNAs does not trigger the interferon response that results from dsRNA-mediated activation of protein kinase PKR and 2',5'-oligoadenylate synthetase resulting in non-specific cleavage of mRNA by ribonuclease L.

dsRNAs targeting TIA-1, *e.g.,* a wild-type or mutant TIA-1, can be unmodified or chemically modified, e.g., as described herein. The dsRNA can be chemically synthesized, expressed from a vector, or enzymatically synthesized. The disclosure also features various chemically modified synthetic dsRNA molecules capable of modulating TIA-1 gene expression or activity in cells by RNA interference (RNAi). The use of chemically modified dsRNA improves various properties of native dsRNA molecules, such as through increased resistance to nuclease degradation *in vivo* and/or through improved cellular uptake.

The dsRNAs targeting nucleic acid can be composed of two separate RNAs, or of one RNA strand, which is folded to form a hairpin structure. Hairpin dsRNAs are typically referred to as shRNAs.

An shRNA that targets TIA-1, *e.g.,* a mutant or wild-type TIA-1 gene can be expressed from a vector, e.g., viral vector, such as a lentiviral or adenoviral vector. In certain embodiments, a suitable dsRNA for inhibiting expression of a TIA-1 gene will be identified by screening an siRNA library, such as an adenoviral or lentiviral siRNA library.

In an embodiment, a dsRNA that targets TIA-1 is about 15 to about 30 base pairs in length (*e.g.,* about 16, 17, 18, 19, 20, 21, 22, 23, 24, 25, 26, 27, 28, or 29) base pairs in length. In an embodiment, each strand of a dsRNA that targets TIA-1 is independently about 15 to about 30 nucleotides in length (*e.g.,* about 16, 17, 18, 19, 20, 21, 22, 23, 24, 25, 26, 27, 28, or 29) nucleotides in length. In an embodiment, the dsRNA includes overhanging ends of about 1 to about 3 (*e.g.,* about 1, 2, or 3) nucleotides. By "overhang" is meant that 3'-end of one strand of the dsRNA extends beyond the 5'-end of the other strand, or vice versa. The dsRNA can have an overhang on one or both ends of the dsRNA molecule. In some embodiments, the single-stranded overhang is located at the 3'-terminal end of the antisense strand, or, alternatively, at the 3'-terminal end of the sense strand. In some embodiments, the overhang is a TT or UU dinucleotide overhang, *e.g.,* a TT or UU dinucleotide overhang. For example, in an embodiment, the dsRNA includes a 21-nucleotide antisense strand, a 19 base pair duplex region, and a 3'-terminal dinucleotide. In yet another embodiment, a dsRNA includes a duplex nucleic acid where both ends are blunt, or alternatively, where one of the ends is blunt.

In an embodiment, the dsRNA includes a first and a second strand, each strand is about 18 to about 28 nucleotides in length, *e.g.,* about 19 to about 23 nucleotides in length, the first strand of the dsRNA includes a nucleotide sequence having sufficient complementarity to the TIA-1 RNA for the dsRNA to direct cleavage of the TIA-1 mRNA via RNA interference, and the second strand of the dsRNA includes a nucleotide sequence that is complementary to the first strand.

In an embodiment, a dsRNA targeting a TIA-1 gene can target wild-type and mutant forms of the gene, or can target different allelic isoforms of the same gene, or can target different splice isoforms of the same gene. For example, the dsRNA will target a sequence that is identical in two or more of the different isoforms. In an embodiment, a dsRNA will preferentially or specifically target a mutant TIA-1 RNA, or a particular TIA-1 polymorphism. In an embodiment, a dsRNA will preferentially or specifically target wild-type TIA-1 RNA.

In an embodiment, a dsRNA targeting a TIA-1 RNA includes one or more chemical modifications. Non-limiting examples of such chemical modifications include without limitation phosphorothioate internucleotide linkages, phosphodiester linkage, 2'-deoxyribonucleotides, 2'-O-methyl ribonucleotides, 2'-O-methoxyethyl ribonucleotides, 2'-deoxy-2'-fluoro ribonucleotides, "universal base" nucleotides, "acyclic" nucleotides, 5-C-methyl nucleotides, and terminal glyceryl and/or inverted deoxy abasic residue incorporation. In some embodiments, the nucleic acid based inhibitor comprises at least 1, 2, 3, 4, or 5 of said modifications. In some embodiment, the nucleic acid inhibitor comprises a backbone modification. In some embodiments, the nucleic acid inhibitor comprises a sugar modification, e.g., a 2'O modification. In some embodiments, the nucleic acid inhibitor comprises at least one backbone modification and at least one sugar modification, e.g., a 2'O modification. Such chemical modifications have been shown to preserve RNAi activity in cells while at the same time, dramatically increasing the serum stability of these compounds. Furthermore, one or more phosphorothioate substitutions are well-tolerated and have been shown to confer substantial increases in serum stability for modified dsRNA constructs. Other suitable chemical modifications are described herein, e.g., in the section entitled *"Chemically modified nucleic acids."*

In an embodiment, a dsRNA targeting a TIA-1 RNA includes modified nucleotides while maintaining the ability to mediate RNAi. The modified nucleotides can be used to improve *in vitro* or *in vivo* characteristics such as stability, activity, and/or bioavailability. For example, the dsRNA can include modified nucleotides as a percentage of the total number of nucleotides present in the molecule. As such, the dsRNA can generally include about 5% to about 100% modified nucleotides (*e.g*., about 5%, 10%, 15%, 20%, 25%, 30%, 35%, 40%, 45%, 50%, 55%, 60%, 65%, 70%, 75%, 80%, 85%, 90%, 95% or 100% modified nucleotides).

In some embodiments, the dsRNA targeting TIA-1 is about 21 nucleotides long. In another embodiment, the dsRNA does not contain any ribonucleotides, and in another embodiment, the dsRNA includes one or more ribonucleotides. In an embodiment, each strand of the dsRNA molecule independently includes about 15 to about 30 (*e.g.,* about 15, 16, 17, 18, 19, 20, 21, 22, 23, 24, 25, 26, 27, 28, 29, or 30) nucleotides, wherein each strand includes about 15 to about 30 *(e.g.,* about 15, 16, 17, 18, 19, 20, 21, 22, 23, 24, 25, 26, 27, 28, 29, or 30) nucleotides that are complementary to the nucleotides of the other strand. In an embodiment, one of the strands of the dsRNA includes a nucleotide sequence that is complementary to a nucleotide sequence or a portion thereof of the TIA-1 gene, and the second strand of the dsRNA includes a nucleotide sequence substantially similar to the nucleotide sequence of the TIA-1 gene or a portion thereof.

In an embodiment, the dsRNA targeting TIA-1 includes an antisense region having a nucleotide sequence that is complementary to a nucleotide sequence of the TIA-1 gene or a portion thereof, and a sense region having a nucleotide sequence substantially similar to the nucleotide sequence of the TIA-1 gene or a portion thereof. In an embodiment, the antisense region and the sense region independently include about 15 to about 30 (*e.g.,* about 15, 16, 17, 18, 19, 20, 21, 22, 23, 24, 25, 26, 27, 28, 29, or 30) nucleotides, where the antisense region includes about 15 to about 30 (*e.g.,* about 15, 16, 17, 18, 19, 20, 21, 22, 23, 24, 25, 26, 27, 28, 29, or 30) nucleotides that are complementary to nucleotides of the sense region. In some embodiments, the antisense region that is complementary to a nucleotide sequence of the TIA-1 gene or portion thereof comprises up to 1, 2, 3, 4, or 5 mismatches, insertions, or deletions compared to the corresponding region of the TIA-1 gene. In some embodiments, the antisense region that is complementary to a nucleotide sequence of the TIA-1 gene or portion thereof has perfect complementarity with the corresponding region of the TIA-1 gene.

As used herein, the term "dsRNA" is meant to include nucleic acid molecules that are capable of mediating sequence specific RNAi, such as short interfering RNA (siRNA), short hairpin RNA (shRNA), short interfering oligonucleotide, short interfering nucleic acid, short interfering modified oligonucleotide, chemically modified siRNA, post-transcriptional gene silencing RNA (ptgsRNA), and others. In addition, as used herein, the term "RNAi" is meant to include sequence specific RNA interference, such as post transcriptional gene silencing, translational inhibition, or epigenetics.

### Antisense nucleic acids

Suitable nucleic acid based inhibitors include antisense nucleic acids. While not being bound by theory it is believed that antisense inhibition is typically based upon hydrogen bonding-based hybridization of oligonucleotide strands or segments such that at least one strand or segment is cleaved, degraded, or otherwise rendered inoperable.

An antisense agent can bind TIA-1 DNA. In embodiments it inhibits replication or transcription or both. While not being bound by theory it is believed that an antisense agent can also function to inhibit target RNA translocation, e.g., to a site of protein translation, translation of protein from the RNA, splicing of the RNA to yield one or more RNA species, and catalytic activity or complex formation involving the RNA.

Antisense agents can include, for example, from about 8 to about 80 nucleobases (*i.e.,* from about 8 to about 80 nucleotides), *e.g*., about 8 to about 50 nucleobases, or about 12 to about 30 nucleobases. In some embodiments, the antisense agent is 12, 13, 14, 15, 16, 17, 18, 19, 20, 21, 22, 23, 24, 25, 26, 27, 28, 29, or 30 nucleobases in length. Antisense compounds include ribozymes, external guide sequence (EGS) oligonucleotides (oligozymes), and other short catalytic RNAs or catalytic oligonucleotides which hybridize to the target nucleic acid and modulate its expression. Antisense compounds can include a stretch of at least eight consecutive nucleobases that are complementary to a sequence in the target gene. An oligonucleotide need not be 100% complementary to its target nucleic acid sequence to be specifically hybridizable.

Hybridization of antisense oligonucleotides with mRNA (*e.g.,* an mRNA encoding TIA-1) can interfere with one or more of the normal functions of mRNA. While not being bound by theory it is believed that the functions of mRNA to be interfered with include all key functions such as, for example, translocation of the RNA to the site of protein translation, translation of protein from the RNA, splicing of the RNA to yield one or more mRNA species, and catalytic activity which may be engaged in by the RNA. Binding of specific protein(s) to the RNA may also be interfered with by antisense oligonucleotide hybridization to the RNA.

Exemplary antisense compounds include DNA or RNA sequences that specifically hybridize to the target nucleic acid, *e.g.,* the mRNA encoding TIA-1. The complementary region can extend for between about 8 to about 80 nucleobases. The compounds can include one or more modified nucleobases. Modified nucleobases may include, *e.g*., 5-substituted pyrimidines such as 5-iodouracil, 5-iodocytosine, and C5-propynyl pyrimidines such as C5-propynylcytosine and C5-propynyluracil. Other suitable modified nucleobases include N⁴-(C₁-C₁₂) alkylaminocytosines and N⁴,N⁴-(C₁-C₁₂) dialkylaminocytosines. Modified nucleobases may also include 7-substituted-5-aza-7-deazapurines and 7-substituted-7-deazapurines such as, for example, 7-iodo-7-deazapurines, 7-cyano-7-deazapurines, 7-aminocarbonyl-7-deazapurines. Examples of these include 6-amino-7-iodo-7-deazapurines, 6-amino-7-cyano-7-deazapurines, 6-amino-7-aminocarbonyl-7-deazapurines, 2-amino-6-hydroxy-7-iodo-7-deazapurines, 2-amino-6-hydroxy-7-cyano-7-deazapurines, and 2-amino-6-hydroxy-7-aminocarbonyl-7-deazapurines. Furthermore, N⁶-(C₁-C₁₂) alkylaminopurines and N⁶,N⁶-(C₁-C₁₂) dialkylaminopurines, including N⁶-methylaminoadenine and N⁶,N⁶-dimethylaminoadenine, are also suitable modified nucleobases. Similarly, other 6-substituted purines including, for example, 6-thioguanine may constitute appropriate modified nucleobases. Other suitable nucleobases include 2-thiouracil, 8-bromoadenine, 8-bromoguanine, 2-fluoroadenine, and 2-fluoroguanine. Derivatives of any of the aforementioned modified nucleobases are also appropriate. Substituents of any of the preceding compounds may include C₁-C₃₀ alkyl, C₂-C₃₀ alkenyl, C₂-C₃₀ alkynyl, aryl, aralkyl, heteroaryl, halo, amino, amido, nitro, thio, sulfonyl, carboxyl, alkoxy, alkylcarbonyl, alkoxycarbonyl, and the like. An antisense agent can also have a chemical modification described herein in the section entitled *"Chemically modified nucleic acids."*

### MicroRNA

In some embodiments, the nucleic acid-based inhibitor suitable for targeting TIA-1 is a microRNA (miRNA). A miRNA is a single stranded RNA that regulates the expression of target mRNAs either by mRNA cleavage, translational repression/inhibition or heterochromatic silencing. The miRNA is 18 to 25 nucleotides, typically 21 to 23 nucleotides in length. In some embodiments, the miRNA includes chemical modifications, such as one or more modifications described herein. In embodiments, a pre-miRNA and/or pri-miRNA is processed into a miRNA inhibitor targeting TIA-1.

In some embodiments, a nucleic acid based inhibitor, e.g., a miRNA, targeting TIA-1 has partial complementarity (*i.e.,* less than 100% complementarity) with the target TIA-1 mRNA. miRNAs often have partial complementarity to their targets. As examples, partial complementarity can include various mismatches or non-base paired nucleotides (e.g., 1, 2, 3, 4, 5 or more mismatches or non-based paired nucleotides, such as nucleotide bulges), which can result in bulges, loops, or overhangs that result between the antisense strand or antisense region of the nucleic acid-based inhibitor and the corresponding target nucleic acid molecule.

A miRNA can also have a chemical modification described herein, e.g., in the section entitled *"Chemically modified nucleic acids."*

### Long interfering nucleic acids

In some embodiments, the nucleic acid based inhibitor comprises a long interfering nucleic acid. A long interfering nucleic acid is typically a double stranded nucleic acid, e.g., DNA or RNA. Long interfering nucleic acids are sometimes immunostimulatory in mammalian cells, e.g., can activate the interferon response. The interferon response can lead to global suppression of translation and global RNA degradation.

In some embodiments, the long interfering nucleic acid comprises one or two strands that are each independently about 50-100, 100-150, 150-200, 200-250, 250-300, 300-350, 350-400, 450-500, or greater than 500 nucleotides in length. In some embodiments, the long interfering nucleic acid is at least about 50, 100, 150, 200, 250, 300, 350, 450, or 500 nucleotides in length.

A long interfering RNA can also have a chemical modification described herein, e.g., in the section entitled *"Chemically modified nucleic acids."*

### Aptamers

Nucleic acid aptamers are typically short, structured, single-stranded RNA or DNA oligonucleotides engineered to bind strongly and specifically to a target. Aptamers can be used as therapeutics, or as targeting agents to deliver another therapeutic (e.g., a dsRNA or antisense molecule) to a desired location. Thus, in some embodiments, a nucleic acid-based inhibitor comprises an aptamer and a second portion, wherein the second portion comprises a nucleic acid capable of mediating mRNA degradation or inhibition of translation.

In some embodiments, the aptamer binds to a target, e.g., target protein, present in neurons, e.g., neurons of the CNS. The target may be, e.g., present on the cell surface of neurons, e.g., a transmembrane protein, a surface-associated protein, a lipid-anchored protein, or a carbohydrate moiety.

Aptamers can be produced using high throughput screening technologies, e.g., providing a pool of different candidate sequences, selecting sequences with affinity for a target, creating a new pool based on the sequences that had affinity for the target (e.g., by performing amplification with mutagenesis), and repeating these steps until an aptamer with the desired affinity is generated.

An aptamer can also have a chemical modification described herein, e.g., in the section entitled *"Chemically modified nucleic acids."*

### CRISPR Systems

In some embodiments, the nucleic acid based inhibitor comprises a CRISPR RNA. "CRISPR" refers to a set of clustered regularly interspaced short palindromic repeats, or a system comprising such a set of repeats. "Cas" refers to a CRISPR-associated protein. A "CRISPR/Cas" system refers to a system derived from CRISPR and Cas which can be used to alter expression of, e.g., eliminate expression of, a target gene such as TIA-1.

Naturally-occurring CRISPR/Cas systems are found in approximately 40% of sequenced eubacteria genomes and 90% of sequenced archaea. Grissa et al. (2007) BMC Bioinformatics 8: 172. This system is a type of prokaryotic immune system that confers resistance to foreign genetic elements such as plasmids and phages and provides a form of acquired immunity. Barrangou et al. (2007) Science 315: 1709-1712; Marragini et al. (2008) Science 322: 1843-1845.

The CRISPR/Cas system has been modified for use in gene editing (silencing, enhancing or changing specific genes) in eukaryotes such as mice or primates. Wiedenheft et al. (2012) Nature 482: 331-8. This can be accomplished by introducing into the eukaryotic cell a plasmid containing a specifically designed CRISPR and one or more appropriate Cas.

The CRISPR sequence, sometimes called a CRISPR locus, comprises alternating repeats and spacers. In a naturally-occurring CRISPR, the spacers usually comprise sequences foreign to the bacterium such as a plasmid or phage sequence; in a TIA-1 targeted CRISPR/Cas system, the spacers can be derived from the TIA-1 gene sequence.

RNA from the CRISPR locus is constitutively expressed and processed by Cas proteins into small RNAs. These comprise a spacer flanked by a repeat sequence. The RNAs guide other Cas proteins to silence exogenous genetic elements at the RNA or DNA level. Horvath et al. (2010) Science 327: 167-170; Makarova et al. (2006) Biology Direct 1: 7. The spacers thus serve as templates for RNA molecules, analogously to siRNAs. Pennisi (2013) Science 341: 833-836.

As these naturally occur in many different types of bacteria, the exact arrangements of the CRISPR and structure, function and number of Cas genes and their product differ somewhat from species to species. Haft et al. (2005) PLoS Comput. Biol. 1: e60; Kunin et al. (2007) Genome Biol. 8: R61 ; Mojica et al. (2005) /. Mol. Evol. 60: 174-182; Bolotin et al. (2005) Microbiol. 151 : 2551-2561; Pourcel et al. (2005) Microbiol. 151: 653-663; and Stern et al. (2010) Trends. Genet. 28: 335-340. For example, the Cse (Cas subtype, E. coli) proteins (e.g., CasA) form a functional complex, Cascade, that processes CRISPR RNA transcripts into spacer-repeat units that Cascade retains. Brouns et al. (2008) Science 321 : 960-964. In other prokaryotes, Cas6 processes the CRISPR transcript. The CRISPR-based phage inactivation in E. coli requires Cascade and Cas3, but not Casl or Cas2. The Cmr (Cas RAMP module) proteins in *Pyrococcus furiosus* and other prokaryotes form a functional complex with small CRISPR RNAs that recognizes and cleaves complementary target RNAs. A simpler CRISPR system relies on the protein Cas9, which is a nuclease with two active cutting sites, one for each strand of the double helix. Combining Cas9 and modified CRISPR locus RNA can be used in a system for gene editing. Pennisi (2013) Science 341 : 833-836. Other suitable Cas proteins include Cpf1 proteins, homologs of which are found in *Acidominococcus* and *Lachnospiraceae.*

The CRISPR/Cas system can thus be used to edit a TIA-1 gene (e.g., adding or deleting one or more base pairs), introducing a premature stop which thus decreases expression of a TIA-1 gene, inactivate or remove an aggregation domain of TIA-1 (e.g., a prion-like, poly-glycine rich domain, e.g., as described in Gilks et al. Mol Biol Cell. 2004 Dec; 15(12):5383-98), or introduce a mutation that reduces levels or activity of TIA-1, or any combination thereof. The CRISPR/Cas system can alternatively be used like RNA interference, turning off a TIA-1 gene in a reversible fashion. In a mammalian cell, for example, the RNA can guide the Cas protein to a TIA-1 promoter, sterically blocking RNA polymerases.

Artificial CRISPR/Cas systems can be generated which inhibit TIA-1, using technology known in the art, e.g., that described in U.S. Publication No. 20140068797, and Cong (2013) Science 339: 819-823. Other artificial CRISPR/Cas systems that are known in the art may also be generated which inhibit TIA-1, e.g., that described in Tsai (2014) Nature Biotechnol., 32:6 569-576, U.S. Patent No.: 8,871,445; 8,865,406; 8,795,965; 8,771,945; and 8,697,359.

### Chemically modified nucleic acids

The nucleic acid based inhibitor can comprise one or more chemical modification. In some embodiments, the nucleic acid based inhibitor comprises at least 1, 2, 3, 4, or 5 of said modifications. In some embodiment, the nucleic acid inhibitor comprises one or more backbone modifications. In some embodiments, the nucleic acid inhibitor comprises one or more sugar modification, e.g., a 2'O modification. In some embodiments, the nucleic acid inhibitor comprises one or more backbone modifications and one or more sugar modification, e.g., a 2'O modification. In some embodiments, the nucleic acid inhibitor comprises one or more nucleobase modifications. In some embodiments, the nucleic acid inhibitor comprises one or more nucleobase modifications and one or more sugar modifications, e.g. a 2'O modification. In some embodiments, the nucleic acid inhibitor comprises one or more nucleobase modifications and one or more backbone modifications. In some embodiments, the nucleic acid inhibitor comprises one or more nucleobase modifications, one or more sugar modifications, e.g. a 2'O modification, and one or more backbone modifications. Various nucleobase modifications, sugar modifications, and backbone modifications are set out herein.

Modified nucleobases may include, *e.g.*, 5-substituted pyrimidines such as 5-iodouracil, 5-iodocytosine, 5-C-methyl nucleotides, and C5-propynyl pyrimidines such as C5-propynylcytosine and C5-propynyluracil. Other suitable modified nucleobases include N⁴-(C₁-C₁₂) alkylaminocytosines and N⁴,N⁴-(C₁-C₁₂) dialkylaminocytosines. Modified nucleobases may also include 7-substituted-5-aza-7-deazapurines and 7-substituted-7-deazapurines such as, for example, 7-iodo-7-deazapurines, 7-cyano-7-deazapurines, 7-aminocarbonyl-7-deazapurines. Examples of these include 6-amino-7-iodo-7-deazapurines, 6-amino-7-cyano-7-deazapurines, 6-amino-7-aminocarbonyl-7-deazapurines, 2-amino-6-hydroxy-7-iodo-7-deazapurines, 2-amino-6-hydroxy-7-cyano-7-deazapurines, and 2-amino-6-hydroxy-7-aminocarbonyl-7-deazapurines. Furthermore, N⁶-(C₁-C₁₂) alkylaminopurines and N⁶,N⁶-(C₁-C₁₂) dialkylaminopurines, including N⁶-methylaminoadenine and N⁶,N⁶-dimethylaminoadenine, are also suitable modified nucleobases. Similarly, other 6-substituted purines including, for example, 6-thioguanine may constitute appropriate modified nucleobases. Other suitable nucleobases include 2-thiouracil, 8-bromoadenine, 8-bromoguanine, 2-fluoroadenine, and 2-fluoroguanine. Derivatives of any of the aforementioned modified nucleobases are also appropriate. Substituents of any of the preceding compounds may include C₁-C₃₀ alkyl, C₂-C₃₀ alkenyl, C₂-C₃₀ alkynyl, aryl, aralkyl, heteroaryl, halo, amino, amido, nitro, thio, sulfonyl, carboxyl, alkoxy, alkylcarbonyl, alkoxycarbonyl, and the like. Modified nucleobases also include 5-bromo-uridine, 5-iodo-uridine, 4-thio-uridine, N3-methyl uridine, and 2,6-diamino-purine. The modified nucleobases may also be a "universal base" nucleotide, or "acyclic" nucleotide. In some embodiments, the universal base nucleotide can pair with high affinity to one of at least 2, 3, or 4 different bases. Universal bases include hypoxanthine, nitroazoles, isocarbostyril analogues, azole carboxamides, aromatic triazole analogues, inosine, deoxyinosine, nitroazole analogues, and hydrophobic aromatic non-hydrogen-bonding bases.

Backbone modifications include modifications to phosphates and/or sugars. A modified backbone may comprise one or more phosphorothioate internucleotide linkages, phosphodiester linkages, boranophosphate linkages, or a combination thereof. Nucleic acids with modified backbones also include peptide nucleic acid (PNA), morpholino, locked nucleic acid (LNA), glycol nucleic acid (GNA), and threose nucleic acid (TNA).

Sugar modifications, e.g., to an RNA molecule, include 2'-deoxyribonucleotides, 2'O-alkyl ribonucleotides (e.g., 2'-O-methyl ribonucleotides), 2'-aminoethyl ribonucleotides, 2'-O-methoxy ribonucleotides, 2'-O-methoxyethyl ribonucleotides, 2'-deoxy-2'-fluoro ribonucleotides, and sugar-modified ribonucleotides in which the 2'-OH is replaced by a group such as an H, OR, R, halo, SH, SR, NH₂, NHR, NR₂, or CN, wherein R is an alkyl moiety. The alkyl moiety may be, e.g., a straight or branched chain, and can have, e.g., 1, 2, 3, 4, 5, 6, or more carbons. Examples of saturated hydrocarbon groups include, but are not limited to, groups such as methyl, ethyl, n-propyl, isopropyl, n-butyl, t-butyl, isobutyl, sec-butyl, n-pentyl, isopentyl, homologs and isomers of, for example, n-pentyl, n-hexyl, and the like. Sugar modifications also include locked nucleic acid (LNA), unlocked nucleic acids (UNA). In embodiments, the modified sugar is a bicyclic sugar, e.g., which comprises 4'-(CH₂)--O-2' (LNA); 4'-(CH₂)₂--O-2' (ENA); or 4'-CH(CH₃)--O-2' (cEt). In embodiments, the modified sugar comprises 3'-fluoro-HNA (hexitol nucleic acid) or a 4'-(CH₂)ₙ--O-2' bridge, wherein n is 1 or 2. In embodiments, the nucleotide having a modified sugar is a modified tetrahydropyran nucleoside such as hexitol nucleic acid (HNA), anitol nucleic acid (ANA), manitol nucleic acid (MNA) or fluoro HNA (F-HNA).

In some embodiments, the nucleic acid based inhibitor, e.g., an antisense molecule, comprises one or more of a 2'O-Me (2'-O-methyl) nucleotide, PMO (phosphorodiamidate morpholino) nucleotide, 2'-MOE (2'-O-methoxyethyl) nucleotide, and S-cEt nucleotide (cEt, 2',4'-constrained 2'-O-ethyl), e.g., as described in Rigo et al., "Pharmacology of a Central Nervous System Delivered 2'-OMethoxyethyl-Modified Survival of Motor Neuron Splicing Oligonucleotide in Mice and Nonhuman Primates" J Pharmacol Exp Ther 350:46-55, July 2014. In embodiments, the nucleic acid based inhibitor, e.g., antisense RNA, comprises one or more 2'MOE nucleotides, e.g., comprises 1, 2, 3, 4, 5, 6, 7, 8, 9, 10, 11, 12, 13, 14, 15, 16, 17, 18, 19, or 20 2'-MOE nucleotides. In embodiments, every nucleotide of the nucleic acid based inhibitor (e.g., antisense RNA) is a 2'MOE nucleotide. In embodiments, the 2'-MOE nucleotide has a structure according to the following formula:

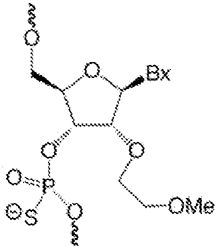

In embodiments, the nucleic acid based inhibitor, e.g., antisense RNA, comprises one or more cEt (e.g., S-cEt) nucleotides, e.g., comprises 1, 2, 3, 4, 5, 6, 7, 8, 9, 10, 11, 12, 13, 14, 15, 16, 17, 18, 19, or 20 cEt (e.g., S-cEt) nucleotides.

In embodiments, the nucleic acid based inhibitor, e.g., antisense RNA, comprises one or more cEt (e.g., S-cEt) nucleotides (e.g., 1, 2, 3, 4, 5, 6, 7, 9, 10, 11, 12, 13, 14, 15, 16, 17, 18, 19, or 20 or more), one or more 2'MOE nucleotides (e.g., 1, 2, 3, 4, 5, 6, 7, 9, 10, 11, 12, 13, 14, 15, 16, 17, 18, 19, or 20 or more), and one or more phosphorothioate internucleotide linkages (e.g., 1, 2, 3, 4, 5, 6, 7, 9, 10, 11, 12, 13, 14, 15, 16, 17, 18, 19, or 20 or more). In embodiments, the nucleic acid based inhibitor, e.g., antisense RNA, comprises one or more 2'MOE nucleotides (e.g., 1, 2, 3, 4, 5, 6, 7, 9, 10, 11, 12, 13, 14, 15, 16, 17, 18, 19, or 20 or more) and one or more phosphorothioate internucleotide linkages (e.g., 1, 2, 3, 4, 5, 6, 7, 9, 10, 11, 12, 13, 14, 15, 16, 17, 18, 19, or 20 or more). In embodiments, the nucleic acid based inhibitor, e.g., antisense RNA, comprises one or more 2'-deoxynucleosides (e.g., 1, 2, 3, 4, 5, 6, 7, 9, 10, 11, 12, 13, 14, 15, 16, 17, 18, 19, or 20 or more).

In embodiments, the nucleic acid based inhibitor comprises one or more of: an inverted abasic moiety, hexitol nucleic acid, 4-C-hydroxymethyl deoxyribonucleic acid, 2' deoxythymidine, 2'-aminoethyl, or any combination thereof.

In some embodiments, the nucleic acid based inhibitor comprises, e.g., at the 5'-position, a moiety having one of the formulas: 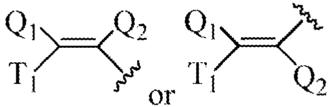 wherein:
T₁ is a phosphorus moiety having the formula: 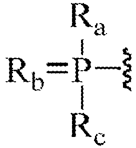 wherein:
Rₐ and R_{c} are each, independently, protected hydroxyl, protected thiol, C₁-C₆ alkyl, substituted C₁-C₆ alkyl, C₁-C₆ alkoxy, substituted C₁-C₆ alkoxy, protected amino or substituted amino;
R_{b} is O or S;
Q₁ and Q₂ are each, independently, H, halogen, C₁-C₆ alkyl, substituted C₁-C₆ alkyl, C₁-C₆ alkoxy, or substituted C₁-C₆ alkoxy;
each substituted group comprises one or more optionally protected substituent groups independently selected from halogen, OJ₁, N(J₁)(J₂), =NJ₁, SJ₁, N₃, CN, OC(=X₂)J₁, OC(=X₂)N(J₁)(J₂) and C(=X₂)N(J₁)(J₂);
X₂ is O, S or NJ₃; and
J₁, J₂ and J₃ are each, independently, H or C₁-C₆ alkyl.

In embodiments, the nucleic acid based inhibitor comprises, e.g., at the 5'-position, a moiety having one of the formulas:

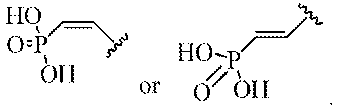

In some embodiments, the nucleic acid based inhibitor comprises, e.g., at the 5'-position, a moiety having one of the formulas: 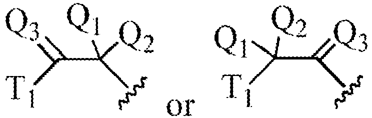 wherein:
T₁ is a phosphorus moiety having the formula: 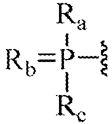 wherein:
Rₐ and R_{c} are each, independently, protected hydroxyl, protected thiol, C₁-C₆ alkyl, substituted C₁-C₆ alkyl, C₁-C₆ alkoxy, substituted C₁-C₆ alkoxy, protected amino or substituted amino;
R_{b} is O or S;
Q₁ and Q₂ are each, independently, H, halogen, C₁-C₆ alkyl, substituted C₁-C₆ alkyl, C₁-C₆ alkoxy, or substituted C₁-C₆ alkoxy;
Q₃ is O, S, N(R₅) or C(R₆)(R₇);
R₅, R₆ and R₇ are each, independently, H, C₁-C₆ alkyl, substituted C₁-C₆ alkyl or C₁-C₆ alkoxy;
each substituted group comprises one or more optionally protected substituent groups independently selected from halogen, OJ₁, N(J₁)(J₂), =NJ₁, SJ₁, N₃, CN, OC(=X₂)J₁, OC(=X₂)N(J₁)(J₂) and C(=X₂)N(J₁)(J₂);
X₂ is O, S or NJ₃; and
J₁, J₂ and J₃ are each, independently, H or C₁-C₆ alkyl.

In embodiments, the nucleic acid based inhibitor comprises one or more of: 1-Aminohexane, 12-Hydroxydodecyl 2,3,4,6-Tetra-O-Methyl-b-D-Glucopyranoside, 12-[(2,3,4,6-Tetra-O-methyl-b-D-glucopyranosyloxy)]dodecyl (2-cyanoethyl) (N,N-diisopropyl) phosphoramidite derivative, 2,3-Di-Chlorobenzene, 2,4-bridged nucleic acid, 2,4-Carbocyclic-Ethylene-bridged nucleic acid-Locked nucleic acid, 2,4-Carbocyclic-Locked nucleic acid-Locked nucleic acid, 2,4-Difluorobenzene, 2,4-Difluorotoluene, 2,4-Difluorotoluene-2-Deoxyribonucleotide, 2-Aminoethoxymethyl, 2-Aminoethyl, 2-Aminopropoxymethyl, 2-Aminopropyl, 2-Aminopurineribonucleotide, 2-Cyanoethyl, 2-Deoxy, 2-Deoxy-2-Aminopurine-2-Deoxyribonucleotide, 2-Deoxy-2-Fluoro, 2-Deoxy-2-Fluoro-4-Thioarabinonucleic acid, 2-Deoxy-2-Fluoroarabinonucleic acid, 2-Deoxy-2-fluorouridine, 23.2-Deoxy-2-N,4-C-Ethylene-Locked nucleic acid, 2-Deoxyinosine, 2-Deoxynebularine, 2-Deoxythymidine, 2-Guanidinoethyl, 2-Hydroxy, 2-Hydroxyethyl 2,3,4,6-tetra-O-Methyl-b-D-Glucopyranoside, 2-Methoxy, 2-Methyl, 2-N-Adamant-1-yl-Methylcarbonyl-2-Amino-Locked nucleic acid, 2-N-Pyren-1-yl Methyl-2-Amino-Locked nucleic acid, 2-O-(2-Methoxyethyl), 2-O-Allyl-ribose, 2-O-Benzyl, 2-O-Fluoro, 2-O-Guanidinoethyl-ribose, 2-O-Guanidinopropyl, 2-O-Lysylaminohexyl, 2-O-methoxyethylribose, 2-O-Methyl-4-Thioribose, 2-Thiouridine, 3-Amino, 3-O-methylribose, 4-C- Aminomethyl-2-O-Methylribose, 4-C-Hydroxymethyl Deoxyribonucleic acid, 4-Methylbenzimidazole, 4-Thioribose, 5-Amino, 5-Bromo-Uridine, 5-Cholesterol, 5-Fluoro-2-Deoxyuridine, 5-Iodo-Uridine, 5-Methylcytosine, 5-Nitroindole-2-Deoxyribonucleotide, 5-Nitroindoleribonucleotide, 5-O-Methyl, 5-O-methylthymidine, 5-Phosphate, 5-Thio, Abasic(2-hydroxymethyl-tetrahydrofuran-3-ol), Alfa-L-Locked nucleic acid, Aminoisonucleotide-Adenine, Aminoisonucleotide-thymidine, Anthracene, Biotin, Boranophosphate, Boron cluster, Cyclohexenyl nucleic acid, D-Isonucleoside-adenine, D-Isonucleoside-uracil, Deoxyadenine, Deoxyuridine, Diaminopurine, Difluorotoluyl nucleotide, Dihydrouridine, Dimethoxy-Nitrophenyl Ethyl group, Dodecyl derivative, Fluorescein, Fluorobenzene, Fluorouridine, Galactose, Glucosamine analogue, Hexitol nucleic acid, Hydroxyphenyl-Conjugated, Hypoxanthine, Inosine, Inverted deoxy abasic, Inverted DeoxyThymidine, Inverted thymidine, L-isonucleoside thymidine, Lauric acid, Methyl, Methylcytosine, Methyleneamide, Methyluridine, Morpholinouridine, N-3 Methyluridine, N-hexylhexadecanamide, N4-Ethyl-N4 2-Deoxy-5-Methylcytidine, N6-Ethyl-N6 2-Deoxyadenosine, Naphthalene modification, Nebularine, Oxetane-Locked nucleic acid, Palmitic acid, Peptide nucleic acids, Phenyl, Phosphate, Phosphodiester, Phosphorothioate, Propynyluridine, Pseudouridine, Puromycin, Pyrene modification, Serinol nucleic acid, Thymidine analogue, Triazole-linked nucleic acid, Tricyclodeoxyribonucleic acid, Trifluoromethylbenzene, Unlocked nucleic acid, Xylo-3-fluororibose, Xylo-O-methylribose, [1,1-Biphenyl]-3,5-dimethanol, [3-(hydroxymethyl)-5-naphthalen-1-ylphenyl]methanol, [3-(hydroxymethyl)-5-phenanthren-9-ylphenyl]methanol, [3-(hydroxymethyl)-5-pyren-1-ylphenyl]methanol, [3-(hydroxymethyl)phenyl]methanol, or any combination thereof.

### Self-delivering RNAs

In some embodiments, the nucleic acid based inhibitor comprises a self-delivering RNA (sdRNA). Exemplary self-delivering RNAs are manufactured by Advirna. Some exemplary RNAs are disclosed, e.g., in US Pat. 8,796,443 and 9,080,171, each of which is incorporated herein by reference.

In some embodiments, a sdRNA comprises one or more of, e.g., all of, a single-stranded phosphorothioate region, a short duplex region, a nuclease-stabilizing chemical modification, and a lipophilic chemical modification. In some embodiments, a sdRNA comprises an isolated double stranded nucleic acid molecule comprising a guide strand and a passenger strand, wherein the isolated double stranded nucleic acid molecule includes a double stranded region and a single stranded region. The double stranded region can be, e.g., from 8-15 nucleotides long. The single stranded region can be at the 3' end of the guide strand and/or can be 4-12 nucleotides long. The single stranded region can contain one or more, e.g., 3, 4, 5, 6, 7, 8, 9, 10, 11 or 12 phosphorothioate modifications. In some embodiments, at least 40% of the nucleotides of the isolated double stranded nucleic acid molecule are modified. A hydrophobic conjugate can be attached to the isolated double stranded nucleic acid molecule. The hydrophobic conjugate can comprise a small molecule, optionally a sterol-type molecule, optionally cholesterol. In some embodiments, the isolated double stranded nucleic acid molecule does not form a hairpin.

In some embodiments, a sdRNA comprises an isolated asymmetric nucleic acid molecule comprising: a first polynucleotide wherein the first polynucleotide is complementary to a second polynucleotide and a target gene; and a second polynucleotide, wherein optionally the second polynucleotide is at least 6 nucleotides shorter than the first polynucleotide. The first polynucleotide can include a single stranded region of, e.g., 6, 7, 8, 9, 10, 11 or 12 nucleotides. The single stranded region of the first polynucleotide can contain, e.g., 3, 4, 5, 6, 7, 8, 9, 10, 11 or 12 phosphorothioate modifications. The asymmetric nucleic acid molecule can also include a double stranded region of, e.g., 8-15 nucleotides long. In some embodiments, at least 50% of C and U nucleotides in the double stranded region are 2' O-methyl modified or 2'-fluoro modified. A hydrophobic conjugate can be attached to the asymmetric nucleic acid molecule.

In some embodiments, a sdRNA comprises an isolated double stranded nucleic acid molecule comprising: a guide strand, e.g., of 17-21 nucleotides in length that has complementarity to a target gene, and a passenger strand, e.g., of 8-16 nucleotides in length. The isolated double stranded nucleic acid molecule can include a double stranded region of, e.g., 8-15 nucleotides and a single stranded region. The guide strand and the passenger strand can form the double stranded nucleic acid molecule having the double stranded region and the single stranded region, wherein the single stranded region is optionally at the 3' end of the guide strand and is, e.g., 4-12 nucleotides in length. The single stranded region can comprise one or more, e.g., about 2-12 phosphorothioate modifications. In some embodiments, at least 40% of the nucleotides of the isolated double stranded nucleic acid molecule are modified. The double stranded nucleic acid molecule can be linked to a hydrophobic conjugate. In some embodiments, the isolated double stranded nucleic acid molecule does not form a hairpin.

In some embodiments, a sdRNA comprises a guide strand and a passenger strand, wherein the sdRNA includes a double stranded region and a single stranded region. The double stranded region can be, e.g., from 8-15 nucleotides long. The single stranded region can be, e.g., at the 3' end of the guide strand and can be, e.g., 4-12 nucleotides long. In some embodiments, the single stranded region contains one or more, e.g., 3, 4, 5, 6, 7, 8, 9, 10, 11 or 12 phosphorothioate modifications. In some embodiments, at least 40% of the nucleotides of the isolated double stranded nucleic acid molecule are modified. In some embodiments, at least two Us and/or Cs include a hydrophobic modification, e.g., a hydrophobic modification selected from the group consisting of a thiophene, ethynyl, isobutyl benzyl and imidazole modification. In some embodiments, the hydrophobic modifications are located on positions 4 or 5 of the Us and/or Cs.

### Conjugates

Conjugate moieties of the disclosure (also referred to simply as "conjugates") are moieties that are connected either directly or indirectly to a nucleotide and can target entry into a cell by a variety of means. For instance, conjugate moieties can be lipid in nature. As such, lipid based conjugate moieties can include cationic lipids, neutral lipids, sphingolipids, and fatty acids including stearic, oleic, elaidic, linoleic, linoleaidic, linolenic, and myristic acids. Alternatively, the conjugate moieties can be proteinaceous in nature including peptides that are membrane translocating (e.g., TAT, penetratin, MAP) or cationic (e.g., poly(lys), poly(arg), poly(his), poly (lys/arg/his), or protamine).

Alternatively, the conjugate moiety can be a small molecule that, for instance, targets a particular receptor or is capable of inserting itself into the membrane and being absorbed by endocytic pathways. Thus, small molecules based on adamantanes, polyaromatic hydrocarbons (e.g., napthalenes, phenanthrenes, or pyrenes), macrocyles, steroids, or other chemical scaffolds, are all suitable conjugates. In some embodiments, the conjugate moiety comprises an antibody or an antigen-binding portion thereof (e.g., an scFv).

In some embodiments, conjugate moieties can be based on cationic polymers, such as polyethyleneimine, dendrimers, poly(alkylpyridinium) salts, or cationic albumin.

In some cases, the conjugate moieties are ligands for receptors or can associate with molecules that in turn associate with receptors. Included in this class are bile acids, small molecule drug ligands, vitamins, aptamers, carbohydrates, peptides (including but not limited to hormones, proteins, protein fragments, antibodies or antibody fragments), viral proteins (e.g., capsids), toxins (e.g., bacterial toxins), and more. In some embodiments, the conjugate moeity comprises a ligand for a receptor, e.g., a ligand for asialoglycoprotein receptor (ASGP-R), e.g., a ligand comprising 1, 2, or 3 N-acetylgalactosamine (GalNAc) moieties. In some embodiments, three GalNAc moieties are attached to the 3' terminus of the sense strand using a triantennary spacer. Also included are conjugates that are steroidal in nature e.g., cholesterol, cholestanol, cholanic acid, stigmasterol, pregnelone, progesterones, corticosterones, aldosterones, testosterones, estradiols, ergosterols, and more. Suitable conjugate moieties include cholesterol (CHOL), cholestanol (CHLN), cholanic acid (CHLA), stigmasterol (STIG), and ergosterol (ERGO).

In some embodiments, the molecules that target a particular receptor are modified to eliminate the possible loss of conjugated siRNAs to other sources. For instance, when cholesterol-conjugated siRNAs are placed in the presence of normal serum, a significant fraction of this material will associate with the albumin and/or other proteins in the serum, thus making the siRNA unavailable for e.g., interactions with LDLs. For this reason, the conjugate moieties can be modified in such a way that they continue to bind or associate with their intended target (e.g., LDLs) but have lesser affinities with unintended binding partners (e.g., serum albumin).

In some embodiments, the nucleic acid based inhibitor comprises a Dynamic PolyConjugate (DPC), which can reduce renal clearance. In a DPC, a nucleic acid is attached to a membrane-disrupting polymer (such as poly(butyl amino vinyl ether)) by a hydrolyzable linker. The linker may comprise a disulfide group. The DPC also comprises PEG moieties which are shed in the acidic environment in an endosome, once the DPC is taken up by a target cell. The DPC can also comprise a GalNAc targeting ligand. The PEG moieties may be linked to the polymer using carboxylated dimethyl maleic acid chemistry. DPC polymers may be made, e.g., using uncontrolled polymerization or controlled radical polymerizations, including atom-transfer radical polymerization and reversible addition-fragmentation chain transfer.

In some embodiments, the nucleic acid based inhibitor comprises a cell-penetrating peptide, e.g., one that can induce cell uptake through endocytosis or a non-endocytic mechanism.

In some embodiments, the conjugate moiety comprises a fluorescent molecule, e.g., Cy3, Cy5, FITC, TRITC, rhodamine, or derivative thereof.

In some embodiments, the nucleic acid based inhibitor comprises a PEG moiety.

In some embodiments, the nucleic acid based inhibitor is conjugated to a moiety described in Kanasty et al., "Delivery materials for siRNA therapeutics," Vol. 12, Nov. 2013, p. 967, or Gavrilova et al., "Therapeutic siRna: Principles, challenges, and strategies" Yale Journal of Biology and Medicine 85 (2012), pp. 187-200, each of which is incorporated by reference in its entirety.

### Expression constructs

The nucleic acid-based inhibitors described herein, *e.g*., antisense nucleic acids or dsRNAs described herein, can be incorporated into a gene construct, e.g., a construct to be used as a part of a gene therapy protocol to deliver nucleic acids that can be used to express and produce agents within cells. Expression constructs can also be used to produce a nucleic acid-based inhibitor that can then be administered to a subject. Expression constructs may be administered in any biologically-effective carrier, *e.g.,* any formulation or composition capable of effectively delivering the component gene to cells *in vivo.* Approaches include insertion of the subject gene in viral vectors including recombinant retroviruses, parvovirus, adenovirus, adeno-associated virus, lentivirus, and herpes simplex virus-1, or recombinant bacterial or eukaryotic plasmids. Viral vectors transfect cells directly; plasmid DNA can be delivered with the help of, for example, cationic liposomes (lipofectin) or derivatized (*e.g*., antibody conjugated) polylysine conjugates, gramacidin S, artificial viral envelopes or other such intracellular earners, as well as direct injection of the gene construct or CaPO₄ precipitation carried out *in vivo.* In an embodiment, the vector or delivery vehicle is a viral vector. In an embodiment, the virus is a DNA virus (e.g., dsDNA or ssDNA virus). In an embodiment, the virus is an RNA virus (e.g., an ssRNA virus).

In an embodiment, *in vivo* introduction of nucleic acid into a cell includes use of a viral vector containing nucleic acid, *e.g.,* a cDNA. Infection of cells with a viral vector has the advantage that a large proportion of the targeted cells can receive the nucleic acid. Additionally, molecules encoded within the viral vector, *e.g.,* by a cDNA contained in the viral vector, are expressed efficiently in cells which have taken up viral vector nucleic acid.

Retroviral vectors and adeno-associated virus vectors can be used as a recombinant gene delivery system for the transfer of exogenous genes *in vivo* particularly into humans. These vectors provide efficient delivery of genes into cells, and the transferred nucleic acids are stably integrated into the chromosomal DNA of the host. Protocols for producing recombinant retroviruses and for infecting cells *in vitro* or *in vivo* with such viruses can be found in Current Protocols in Molecular Biology, Ausubel, F. M. et al. (eds.) Greene Publishing Associates (1989), Sections 9.10-9.14 and other standard laboratory manuals. Examples of suitable retroviruses include pLJ, pZIP, pWE, and pEM which are known to those skilled in the art. Examples of suitable packaging virus lines for preparing both ecotropic and amphotropic retroviral systems include Crip, Cre, 2, and Am. Retroviruses have been used to introduce a variety of genes into many different cell types, including epithelial cells, *in vitro* and/or *in vivo* (see, for example, Eglitis et al. (1985) Science 230:1395-1398; Danos and Mulligan (1988) Proc. Natl. Acad. Sci. USA 85:6460-6464; Wilson et al. (1988) Proc. Natl. Acad. Sci. USA 85:3014-3018; Armentano et al. (1990) Proc. Natl. Acad. Sci. USA 87:6141-6145; Huber et al. (1991) Proc. Natl. Acad. Sci. USA 88:8039-8043; Ferry et al. (1991) Proc. Natl. Acad. Sci. USA 88:8377-8381; Chowdhury et al. (1991) Science 254:1802-1805; van Beusechem et al. (1992) Proc. Natl. Acad. Sci. USA 89:7640-7644; Kay et al. (1992) Human Gene Therapy 3:641-647; Dai et al. (1992) Proc. Natl. Acad. Sci. USA 89:10892-10895; Hwu et al. (1993) J. Immunol. 150:4104-4115; U.S. Pat. Nos. 4,868,116 and 4,980,286; PCT Pub. Nos. WO 89/07136, WO 89/02468, WO 89/05345, and WO 92/07573).

Another viral gene delivery system utilizes adenovirus-derived vectors. See, for example, Berkner et al. (1988) BioTechniques 6:616; Rosenfeld et al. (1991) Science 252:431-434; and Rosenfeld et al. (1992) Cell 68:143-155. Suitable adenoviral vectors derived from the adenovirus strain Ad type 5 d1324 or other strains of adenovirus (*e.g*., Ad2, Ad3, Ad7 etc.) are known to those skilled in the art.

Yet another viral vector system useful for delivery of the subject gene is the adeno-associated virus (AAV). See, for example, Flotte et al. (1992) Am. J. Respir. Cell. Mol. Biol. 7:349-356; Samulski et al. (1989) J. Virol. 63:3822-3828; and McLaughlin et al. (1989) J. Virol. 62:1963-1973.

In an embodiment, the nucleic acid based TIA-1 inhibitor is delivered by a recombinant AAV. In an embodiment, the AAV can incorporate its genome into that of a host cell. In embodiments, the virus, e.g., AAV, does not integrate into a subject's genome. In embodiments, the virus, e.g., AAV, lacks *rep* and/or *cap* genes and/or other genes that would otherwise promote integration into the genome. In an embodiment, the AAV is a self-complementary adeno-associated virus (scAAV), e.g., a scAAV that packages both strands, which anneal together to form double stranded DNA. AAV serotypes that may be used in the disclosed methods include, e.g., AAV1, AAV2, modified AAV2 (e.g., modifications at Y444F, Y500F, Y730F and/or S662V), AAV3, modified AAV3 (e.g., modifications at Y705F, Y731F and/or T492V), AAV4, AAV5, AAV6, modified AAV6 (e.g., modifications at S663V and/or T492V), AAV8, AAV 8.2, AAV9, AAV rh 10, and pseudotyped AAV, such as AAV2/8, AAV2/5, AAV2/6, and AAV2/9, can also be used in the disclosed methods. In some embodiments, the viral vector, e.g., AAV vector, comprises up to about 5 kb of nucleic acid, e.g., about 1-5, 2-5, 3-5, 4-5, or about 5 kb of nucleic acid.

In embodiments, the nucleic acid based TIA-1 inhibitor comprises a CRISPR RNA that is delivered by a virus, e.g., an AAV virus. Various CRISPR approaches are described herein, e.g., in the section entitled *"CRISPR Systems."* In embodiments, CRISPR is delivered via AAV, e.g., to neurons, e.g., as described in Swiech et al., "In vivo interrogation of gene function in the mammalian brain using CRISPR-Cas9" Nature Biotechnology 33, 102-106 (2015), which is herein incorporated by reference in its entirety.

In embodiments, the delivery method is one described in Kotterman and Schaffer "Engineering adeno-associated viruses for clinical gene therapy" Nat Rev Genet. 2014 July; 15(7): 445-451, Borel et al. "Recombinant AAV as a Platform for Translating the Therapeutic Potential of RNA Interference" Molecular Therapy vol. 22 no. 4, 692-701 Apr. 2014, Mcintyre et al., "A comparison of multiple shRNA expression methods for combinatorial RNAi" Genetic Vaccines and Therapy 2011, 9:9, each of which is herein incorporated by reference in its entirety.

In embodiments, the virus, e.g., AAV, comprises DNA which encodes one or more nucleic acid based TIA-1 inhibitor, e.g., a shRNA, pre-miRNA, or primary miRNA (pri-miRNA). In embodiments, the DNA further comprises one or more promoter, e.g., a Pol III promoter such as a U6 snRNA or H1 promoter, or a Pol II promoter, which can drive expression of the nucleic acid based TIA-1 inhibitor(s). In embodiments, the DNA comprises one or more transcriptional terminator sequence. In embodiments, the DNA comprises a plurality of cassettes, wherein one or more cassette (e.g., all cassettes) comprise(s) a promoter, a sequence encoding a nucleic acid based TIA-1 inhibitor (e.g., shRNA or miRNA), and a transcriptional terminator. In embodiments, the DNA comprises a spacer between cassettes, e.g., a spacer of about 10-1,000, 100-200, 100-160, 110-150, 120-140, or about 130 nucleotides. In some embodiments, adjacent cassettes are arranged in the same orientation, and in some embodiments, adjacent cassettes are arranged in opposite orientations.

In embodiments, the virus or viral vector comprises a plurality of nucleic acid based TIA-1 inhibitors, or DNA encoding the plurality of nucleic acid based TIA-1 inhibitors. In some embodiments, the nucleic acid comprises or encodes at least two or more (e.g., 3, 4, 5, 6, 7, 8, 9, 10, 20, 50, 100, or 200 or more) nucleic acid based TIA-1 inhibitor sequences, e.g., copies of the same sequence, or different sequences. In some embodiments, the plurality of nucleic acid based TIA-1 inhibitors are copies of the same sequence, and in other embodiments, the plurality of nucleic acid based TIA-1 inhibitors comprise two or more (e.g., 3, 4, 5, 6, 7, 8, 9, or 10 or more) different sequences. In some embodiments, the plurality of nucleic acid based TIA-1 inhibitors are configured as part of one nucleic acid molecule, e.g., form a concatemer or are configured as a plurality of cassettes.

In embodiments, the expression vector (e.g., a viral vector, e.g., AAV) comprises a promoter operably linked to the nucleic acid encoding the nucleic acid based TIA-1 inhibitor. The promoter may be, e.g., a Pol III promoter such as a U6 snRNA or H1 promoter, or a Pol II promoter. The promoter may be active in neurons, e.g., a WPRE, NSE, EF, synapsin, PDGF, enolase, beta-actin, periostin, or GFAP promoter or transcriptionally active portion or variant thereof. The promoter may be active in neurons of the PNS and/or CNS (e.g., in the striatum, hippocampus, cortex, or nigra). The promoter may be specific for neurons. Promoters active in neurons are described in Malmevik et al. "Identification of the miRNA targetome in hippocampal neurons using RIP-seq" Scientific Reports 5:12609, Xu et al. "Quantitative comparison of expression with adenoassociated virus (AAV-2) brain-specific gene cassettes" Gene Therapy (2001) 8, 1323-1332, Peel et al., "Efficient transduction of green fluorescent protein in spinal cord neurons using adeno-associated virus vectors containing cell type-specific promoters" Gene Therapy (1997) 4, 16-24, and Piras et al. "Systemic injection of AAV9 carrying a periostin promoter targets gene expression to a myofibroblast-like lineage in mouse hearts after reperfused myocardial infarction" Gene Therapy (2016), 1-10, each of which is incorporated herein by reference in its entirety.

### Kits

A nucleic acid based TIA-1 inhibitor described herein can be provided in a kit.

In an embodiment the kit includes (a) the nucleic acid based inhibitor described herein, *e.g.,* a composition that includes the nucleic acid based inhibitor described herein (wherein, *e.g.,* the compound can be an inhibitor described herein), and, optionally (b) informational material. The informational material can be descriptive, instructional, marketing or other material that relates to the methods described herein and/or the use of the inhibitor described herein for the methods described herein.

In one embodiment, the informational material can include information about production of the inhibitor, molecular weight of the compound, concentration, date of expiration, batch or production site information, and so forth. In one embodiment, the informational material relates to methods for administering the inhibitor.

In one embodiment, the informational material can include instructions to administer an inhibitor described herein in a suitable manner to perform the methods described herein, *e.g.,* in a suitable dose, dosage form, or mode of administration (*e.g*., a dose, dosage form, or mode of administration described herein). In another embodiment, the informational material can include instructions to administer an inhibitor described herein to a suitable subject, *e.g.,* a human, *e.g.,* a human having or at risk for a disorder described herein.

The kit can include one or more containers for the composition containing an inhibitor described herein. In some embodiments, the kit contains separate containers, dividers or compartments for the inhibitor and informational material. For example, the composition can be contained in a bottle, vial, or syringe, and the informational material can be contained in a plastic sleeve or packet. In other embodiments, the separate elements of the kit are contained within a single, undivided container. For example, the inhibitor is contained in a bottle, vial or syringe that has attached thereto the informational material in the form of a label. In some embodiments, the kit includes a plurality (*e.g.,* a pack) of individual containers, each containing one or more unit dosage forms (*e.g.,* a dosage form described herein) of an inhibitor described herein. For example, the kit includes a plurality of syringes, ampules, foil packets, or blister packs, each containing a single unit dose of an inhibitor described herein. The containers of the kits can be air tight, waterproof (*e.g*., impermeable to changes in moisture or evaporation), and/or light-tight.

The kit optionally includes a device suitable for administration of the composition, *e.g*., a syringe, inhalant, pipette, forceps, measured spoon, dropper (*e.g.,* eye dropper), swab (*e.g.,* a cotton swab or wooden swab), or any such delivery device. In an embodiment, the device is a medical implant device, *e.g.,* packaged for surgical insertion. In an embodiments, the device is an implant suitable for delivery to the CNS, e.g., to the brain.

In some embodiments, the kit comprises two or more, e.g., three, four, five, or more nucleic acid based inhibitors, e.g., nucleic acid based TIA-1 inhibitors. The TIA-1 inhibitors may, e.g., be directed against different regions of TIA-1, may comprise different chemical modifications, or may comprise different conjugate moieties.

### Combination therapies

In some embodiments, a nucleic acid based TIA-1 inhibitor described herein is administered together with an additional treatment for a neurodegenerative disease. The neurodegenerative disease can be, for instance, a tauopathy, e.g., Alzheimer's disease; a motor neuron disease, e.g., amyotrophic lateral sclerosis (ALS); frontotemporal dementia (FTD); frontotemporal dementia with parkinsonism (FTDP-17); frontotemporal lobar dementia (FTLD-TDP); Huntington's disease; Creutzfeld-Jacob disease; and spinomuscular atrophy.

In some embodiments, the additional treatment for a neurodegenerative disease (e.g., Alzheimer's disease) is a cholinesterase inhibitor such as Donepezil (Aricept), Rivastigmine (Exelon), and Galantamine (Razadyne). The additional treatment may also be a NMDA receptor antagonist such as memantine (Namenda).

In some embodiments, the additional treatment for a neurodegenerative disease (e.g., ALS) is a muscle relaxant such as Baclofen or Tizanidine (Zanaflex), or a NMDA receptor antagonist such as Riluzole (Rilutek).

In some embodiments, the additional treatment for a neurodegenerative disease (e.g., FTD) is an antidepressant or antipsychotic such as a selective serotonin reuptake inhibitors (SSRI). SSRIs include fluoxetine (Prozac), sertraline (Zoloft), paroxetine (Paxil), fluvoxamine (Luvox), citalopram (Celexa), and escitalopram (Lexapro). Non-SSRI antidepressants include trazodone (Desyrel), venlafaxine (Effexor), duloxetine (Cymbalta), bupropion (Wellbutrin), and mirtazepine (Remeron). In some embodiments, the additional treatment is a treatment for aphasia, e.g., Bromocriptine (Parlodel).

### Pharmaceutical compositions and methods of delivery

The amount and concentration of nucleic acid based TIA-1 inhibitors in the pharmaceutical compositions, as well as the quantity of the pharmaceutical composition administered to a subject, can be selected based on clinically relevant factors, such as medically relevant characteristics of the subject (*e.g.,* age, weight, gender, other medical conditions, and the like), the solubility of compounds in the pharmaceutical compositions, the potency and activity of the compounds, and the manner of administration of the pharmaceutical compositions. For further information on Routes of Administration and Dosage Regimes the reader is referred to Chapter 25.3 in Volume 5 of Comprehensive Medicinal Chemistry (Corwin Hansch; Chairman of Editorial Board), Pergamon Press 1990.

Nucleic acid based TIA-1 inhibitors can be administered orally, parenterally (including subcutaneously, intramuscularly, or intravenously), rectally, transdermally, buccally, or nasally. The inhibitors can be administered systemically or locally. Compositions, e.g., pharmaceutical compositions comprising nucleic acid based TIA-1 inhibitors may comprise any one or more of the compounds described herein.

The phrases "systemic administration," "administered systemically," "peripheral administration" and "administered peripherally" as used herein mean the administration of a compound, drug or other material other than directly into the site of action, e.g., central nervous system, such that it enters the patient's system and, thus, is subject to metabolism and other like processes, for example, subcutaneous administration.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

The nucleic acid based inhibitor may be formulated with a pharmaceutically acceptable carrier, e.g., that comprises one or more of excipients, such as vehicles adjuvants, pH adjusting and buffering agents, tonicity adjusting agents, stabilizers and wetting agents. Furthermore, in some embodiments, the nucleic acid based inhibitor is delivered in microcapsules, for example by coacervation techniques or by interfacial polymerization (e.g., hydroxymethylcellulose or gelatin-microcapsules and poly-(methylmethasylate) microcapsules, respectively) in colloidal drug delivery systems (for example, liposomes, microspheres, microemulsions, nano-particles, and nanocapsules or microemulsions).

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable carrier" as used herein means a pharmaceutically acceptable material, composition or vehicle, such as a liquid or solid filler, diluent, excipient, solvent or encapsulating material, involved in carrying or transporting the subject antagonists from one organ, or portion of the body, to another organ, or portion of the body. Each carrier must be "acceptable" in the sense of being compatible with the other ingredients of the formulation and not injurious to the patient. Some examples of materials which can serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; (21) cyclodextrins such as Captisol®; and (22) other non-toxic compatible substances employed in pharmaceutical formulations.

In an embodiment, the nucleic acid based inhibitor is delivered to the CNS, e.g., the brain, *e.g.,* directly to the brain, *e.g.,* by intrathecal or intraventricular delivery. In an embodiment, the nucleic acid based inhibitor is delivered to the cerebrospinal fluid (CSF). The nucleic acid based inhibitor can also be delivered from an implantable device. In an embodiment, the nucleic acid-based inhibitor is delivered by infusion using, *e.g.,* a catheter, and optionally, a pump. In some embodiments, the pump is an intracerebral pump. In some embodiments, the formulation for CNS delivery comprises exosomes or liposomes.

Methods of introduction, e.g., to the CNS, may also be provided by rechargeable or biodegradable devices. Various slow release polymeric devices have been developed and tested in vivo in recent years for the controlled delivery of drugs, including proteinacious biopharmaceuticals. A variety of biocompatible polymers (including hydrogels), including both biodegradable and non-degradable polymers, can be used to form an implant for the sustained release of a compound at a particular target site.

The nucleic acid based inhibitor may be delivered directly to the eye by ocular tissue injection such as periocular, conjunctival, subtenon, intracameral, intravitreal, intraocular, subretinal, subconjunctival, retrobulbar, or intracanalicular injections; by direct application to the eye using a catheter or other placement device such as a retinal pellet, intraocular insert, suppository or an implant comprising a porous, non-porous, or gelatinous material; by topical ocular drops or ointments; or by a slow release device in the cul-de-sac or implanted adjacent to the sclera (transscleral) or in the sclera (intrascleral) or within the eye. Intracameral injection may be through the cornea into the anterior chamber to allow the agent to reach the trabecular meshwork. Intracanalicular injection may be into the venous collector channels draining Schlemm's canal or into Schlemm's canal.

For ophthalmic delivery, a nucleic acid based inhibitor may be combined with ophthalmologically acceptable preservatives, co-solvents, surfactants, viscosity enhancers, penetration enhancers, buffers, sodium chloride, or water to form an aqueous, sterile ophthalmic suspension or solution. Solution formulations may be prepared by dissolving the interfering RNA in a physiologically acceptable isotonic aqueous buffer. Further, the solution may include an acceptable surfactant to assist in dissolving the nucleic acid based inhibitor. Viscosity building agents, such as hydroxymethyl cellulose, hydroxyethyl cellulose, methylcellulose, polyvinylpyrrolidone, or the like may be added, to improve the retention of the inhibitor.

In order to prepare a sterile ophthalmic ointment formulation, the nucleic acid based TIA-1 inhibitor can be combined with a preservative in an appropriate vehicle, such as mineral oil, liquid lanolin, or white petrolatum. Sterile ophthalmic gel formulations may be prepared by suspending the nucleic acid based inhibitor in a hydrophilic base prepared from the combination of, for example, CARBOPOL-940 (BF Goodrich, Charlotte, N.C.), or the like, according to methods known in the art. VISCOAT (Alcon Laboratories, Inc., Fort Worth, Tex.) may be used for intraocular injection, for example. Other compositions may contain penetration enhancing agents such as cremephor and TWEEN 80 (polyoxyethylene sorbitan monolaureate, Sigma Aldrich, St. Louis, Mo.), in the event the nucleic acid based innhibitor is less penetrating in the eye.

In some embodiments, the nucleic acid based inhibitor is formulated as a liposomal formulation. The formulation can comprise, e.g., cationic lipid DOTMA (N-[1-{2,3-dioleyloxy}propyl]-N,N,N-triethylammonium chloride), stable nucleic acid-lipid particles (SNALP), Lipofectamine® 2000 (Thermo Fisher Scientific, Waltham, MA, USA), Lipofectamine® (Thermo Fisher Scientific, Waltham, MA, USA), Oligofectamine™ (Thermo Fisher Scientific, Waltham, MA, USA), LipoTrust™ (Hokkaido System Science Co, Ltd, Hokkaido, Japan), i-Fect™ (Neuromics, Edina, MN, USA), DLinDMA, DLin-KC2-DMA, DPPC, a PEG-lipid such as MPEG200-C-DMA, a neutral lipid (e.g., 1,2-Oleoyl-sn-Glycero-3-phosphocholine (DOPC) or 1,2-Dioleoyl-sn-Glycero-3-phosphoethanolamine (DOPE)), a cationic lipid such as 1-oleoyl-2-[6-[(7-nitro-2-1,3-benzoxadiazol-4-yl)amino]hexanoyl]-3-trimethylammonium propane (DOTAP), or any combination thereof. The liposome can include a cationic lipid, ionizable lipid, or both. A lipid, e.g., an ioniziable lipid, can include an amine head group, a linker group and one or more (e.g., 2) hydrophobic tails. In some embodiments, the liposome comprises PEG, e.g., lipid-anchored PEG. In some embodiments, the liposome comprises, e.g., at its core, 1,2-dioleoyl-sn-glycero-3-phosphatidylethanolamine (DOPE), 2-hydroxyethyl methacrylate (HEMA)-lysine-modified cholesterol, or both. In some embodiments, the liposome comprises cholesterol. In some embodiments, the liposome comprises a targeting moiety, e.g., a moiety that binds a surface protein on a target cell. The targeting moiety may comprise, e.g., lipoprotein ApoE, Retinol binding protein (RBP), or GalNAc.

In some embodiments, the nucleic acid based inhibitor is formulated with synthetic lipid-like materials (e.g., "lipidoids")

In some embodiments, the nucleic acid based inhibitor is formulated as nanoparticles, e.g., cyclodextrin polymer (CDP)-based nanoparticles. In some embodiments, the CDP-based nanoparticles are polycationic oligomers (n ≈ 5) synthesized by a step-growth polymerization between diamine-bearing cyclodextrin monomers and dimethyl suberimidate, yielding oligomers with amidine functional groups. The polymer termini can be capped with imidazole functional groups, e.g., to aid endosomal escape. The polymers can also comprise adamantane-PEG (AD-PEG), adamantane-PEG-transferrin (AD-PEG-Tf), or both. In some embodiments, the nanoparticles are about 20-200 nm. In some embodiments, the nanoparticles are large enough to avoid renal filtration but small enough to evade phagocytic clearance. In some embodiments, the nanoparticles comprise chitosan, cyclodextrin, polyethyleneimine (PEI), poly(lactic-co-glycolic) acid (PLGA), or dendrimers (heavily branched polymeric molecules). In some embodiments, the nanoparticles comprise a metallic substance, e.g., at the core. The metallic substance can be, e.g., iron oxide, iron cobalt, iron gold, or iron nickel. A nanoparticle with a metallic core can comprise a coating of sugars or other polymers, e.g., for linking the nucleic acid based inhibitor.

In some embodiments, the nucleic acid based inhibitor is formulated as oligonucleotide nanoparticles (ONPs). ONPs can comprise complementary DNA fragments designed to hybridize into predefined three-dimensional structures, such as a DNA tetrahedral structure. An ONP can comprise one or more (e.g., 2, 3, or more) folate ligands.

In some embodiments, the nucleic acid based inhibitor is formulated with polymeric micelles, e.g., comprising amphiphilic block copolymers.

In some embodiments, the nucleic acid based inhibitor is formulated as any of the compositions described in Kanasty et al., "Delivery materials for siRNA therapeutics," Vol. 12, Nov. 2013, p. 967 or Gavrilova et al., "Therapeutic siRna: Principles, challenges, and strategies" Yale Journal of Biology and Medicine 85 (2012), pp. 187-200, each of which is incorporated by reference in its entirety.

In some embodiments, the pharmaceutical composition comprises two or more, e.g., three, four, five, or more nucleic acid based inhibitors, e.g., nucleic acid based TIA-1 inhibitors. The TIA-1 inhibitors may, e.g., be directed against different regions of TIA-1, may comprise different chemical modifications, or may comprise different conjugate moieties.

Non-limiting examples of agents suitable for formulation with a nucleic acid based TIA-1 inhibitor described herein include: lipid nanoparticles (see for example Semple et al., 2010, Nat Biotechnol., February; 28(2): 172-6.); P-glycoprotein inhibitors (such as Ritinovir or Pluronic P85); biodegradable polymers, such as poly (DL-lactide-coglycolide) microspheres for sustained release delivery (Emerich, D F et al, 1999, Cell Transplant, 8, 47-58); and loaded nanoparticles, such as those made of polybutylcyanoacrylate. Other non-limiting examples of delivery strategies for a nucleic acid based TIA-1 inhibitor described herein include material described in Boado et al., 1998, J. Pharm. Sci., 87, 1308-1315; Tyler et al., 1999, FEBS Lett., 421, 280-284; Pardridge et al., 1995, PNAS USA., 92, 5592-5596; Boado, 1995, Adv. Drug Delivery Rev., 15, 73-107; Aldrian-Herrada et al., 1998, Nucleic Acids Res., 26, 4910-4916; and Tyler et al., 1999, PNAS USA., 96, 7053-7058.

Nucleic acid binding proteins, including double-stranded RNA binding domains, can been used to enhance delivery of nucleic acid based TIA-1 inhibitors into cells. (See, e.g., Eguchi et al. Nat. Biotech. 27:567-571 (2009)). Exemplary nucleic acid binding domains useful in the embodiments disclosed herein include, but are not limited to, those listed in U.S. Patent Application Publication No. US 2009/0093026.

A number of protein transduction domains/peptides may facilitate uptake of heterologous molecules linked to the transduction domains (e.g., cargo molecules such as nucleic acid based TIA-1 inhibitors described herein). Such peptide transduction domains (PTD's) may facilitate uptake through a process referred to as macropinocytosis. Macropinocytosis is a nonselective form of endocytosis. Exemplary peptide transduction domains (PTD's) may be derived from the Drosophila homeoprotein Antennapedia transcription protein (AntHD) (Joliot et al., New Biol. 3:1121-34, 1991; Joliot et al., Proc. Natl. Acad. Sci. USA, 88:1864-8, 1991; Le Roux et al., Proc. Natl. Acad. Sci. USA, 90:9120-4, 1993), the herpes simplex virus structural protein VP22 (Elliott and O'Hare, Cell 88:223-33, 1997), the HIV-1 transcriptional activator TAT protein (Green and Loewenstein, Cell 55:1179-1188, 1988; Frankel and Pabo, Cell 55:1189-1193, 1988), and the cationic N-terminal domain of prion proteins. Other exemplary peptide transduction domains are described in International Patent Application Publication No. WO 08/008,476. The peptide transduction domain may increase uptake of the biomolecule to which it is fused in a receptor independent fashion, may be capable of transducing a wide range of cell types, and may exhibit minimal or no toxicity (Nagahara et al., Nat. Med. 4:1449-52, 1998).

The nucleic acid based TIA-1 inhibitors described herein may be conjugated with a delivery vehicle, or otherwise delivered to target cells or tissues. In certain embodiments, the disclosure provides conjugates and/or complexes of nucleic acid based TIA-1 inhibitors. Such conjugates and/or complexes include ligand based and polymer based delivery modalities that may be used to facilitate delivery of nucleic acid based TIA-1 inhibitors into a biological system, such as a cell. The conjugates and complexes provided herein may impart therapeutic activity by transferring therapeutic compounds across cellular membranes, altering the pharmacokinetics, and/or modulating the localization of nucleic acid molecules described herein. Non-limiting examples of such conjugates are described in U.S. Publication Nos. US2008/0152661 A1 and US 2004/0162260 A1 (e.g., CDM-LBA, CDM-Pip-LBA, CDM-PEG, CDM-NAG, etc.) and U.S. patent application US 2003-0130186; and U.S. Pat. Nos. 7,833,992; 6,528,631; 6,335,434; 6,235,886; 6,153,737; 5,214,136; and 5,138,045.

In various embodiments, polyethylene glycol (PEG) may be covalently attached to nucleic acid based TIA-1 inhibitors described herein. The attached PEG may be any molecular weight, e.g. from about 100 to about 50,000 daltons (Da).

In some embodiments, the delivery method utilizes a transposon (e.g., a SBTS, PB, or pT2-based transposon).

In some embodiments, the nucleic acid based TIA-1 inhibitors described herein are administered (e.g., to the CNS, e.g., by lumbar puncture) about once every 1, 2, 3, 4, 5, or 6 months.

### Dosages

Actual dosage levels of nucleic acid based TIA-1 inhibitors may be varied so as to obtain an amount of the inhibitor that is effective to achieve the desired therapeutic response for a particular patient, composition, and mode of administration, without being toxic to the patient.

The selected dosage level will depend upon a variety of factors including the activity of the particular TIA-1 inhibitor employed, the route of administration, the time of administration, the rate of excretion of the particular TIA-1 inhibitor being employed, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

The amount of TIA-1 inhibitor that can be combined with a carrier material to produce a single dosage form will generally be that amount of the inhibitor that produces a therapeutic effect. Generally out of one hundred percent, this amount will range from about 0.1% to 99% of inhibitor, e.g., from about 5% to about 70%, e.g., from 10% to about 30%.

Toxicity and therapeutic efficacy can be determined by standard pharmaceutical procedures in cell cultures or experimental animals, *e.g*., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compositions that exhibit large therapeutic indices are preferred.

The data obtained from the cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized.

The therapeutically effective dose can be estimated initially from cell culture assays, e.g., cell culture assays described in Example 1. A dose may be formulated in animal models to achieve a circulating plasma concentration range that includes the IC₅₀ (i.e., the concentration of the therapeutic which achieves a half-maximal inhibition of symptoms) as determined in cell culture. Levels in plasma may be measured, for example, by high performance liquid chromatography. The effects of any particular dosage can be monitored by a suitable bioassay.

With respect to duration and frequency of treatment, it is typical for skilled clinicians to monitor subjects in order to determine when the treatment is providing therapeutic benefit, and to determine whether to increase or decrease dosage, increase or decrease administration frequency, discontinue treatment, resume treatment or make other alteration to treatment regimen. The dosing schedule can vary from once a week to daily depending on a number of clinical factors, such as the subject's sensitivity to the drugs. The desired dose can be administered at one time or divided into subdoses, *e.g.,* 2-4 subdoses and administered over a period of time, *e.g.,* at appropriate intervals through the day or other appropriate schedule. Such sub-doses can be administered as unit dosage forms. In some embodiments, administration is chronic, *e.g.,* one or more doses daily over a period of weeks or months. Examples of dosing schedules are administration daily, twice daily, three times daily or four or more times daily over a period of 1 week, 2 weeks, 3 weeks, 4 weeks, 1 month, 2 months, 3 months, 4 months, 5 months, or 6 months or more. In some embodiments, the nucleic acid based inhibitor is administered about once every 12, 18, 24, 30, or 36 months or more.

The present invention contemplates formulation of the TIA-1 inhibitors in any of the pharmaceutical compositions and preparations herein. Furthermore, the disclosure contemplates administration via any of the routes of administration herein. One of skill in the art can select the appropriate formulation and route of administration based on the condition being treated and the overall health, age, and size of the patient being treated.

### Indications and methods of treatment

In some embodiments, the patient comprises a TIA-1 mutation that increases the risk of a neurodegenerative disease and/or promotes stress granule formation. The mutation may be, e.g., E384K. See Hackman et al., "Welander distal myopathy is caused by a mutation in the RNA-binding protein TIA1" Ann. Neurol. 73:500-509(2013). Thus, in some embodiments, the TIA-1 nucleic acid-based inhibitor hybridizes to a nucleic acid encoding a E384K TIA-1 mutant. In some embodiments, the patient has aberrant tau protein function, e.g., dendritic mis-localization of microtubule associated protein tau.

In some embodiments, the patient has one or more mutation in TAR DNA binding protein 43 (TDP-43), fused in sarcoma/TLS (FUS), ataxin-2 (ATXN2), heterogeneous nuclear ribonucleoproteins A1/B2 (hnRNPA1/B2), optineurin, matrin-3, angiogenin and survival motor neuron 1 (SMN1). In some embodiments, the patient (e.g., a patient having ALS, FTD, or Spinocerebellar Ataxia) has a mutation in TAF15 or EWSR1. In some embodiments, the patient (e.g., a patient having a myopathy) has a mutation in TIA1 or VCP. In embodiments, the patient has a P301S mutation in 1N4R tau. In some embodiments, the patient (e.g., a patient having ALS or FTD) has a mutation in C9orf72, e.g., a hexanucleotide repeat expansion, e.g., more than about 20 or 30 repeats of the hexanucleotide. In some embodiments, the patient has an expansion of the trinucleotide repeats in fragile X mental retardation protein 1 (FMRP), an RBP.

In some embodiments, the stress granule comprises tar DNA binding protein-43 (TDP-43), T-cell intracellular antigen 1 (TIA-1), TIA1 cytotoxic granule-associated RNA binding protein-like 1 (TIAR, TIAL1), GTPase activating protein binding protein 1 (G3BP-1), GTPase activating protein binding protein 2 (G3BP-2), tris tetraprolin (TTP, ZFP36), fused in sarcoma (FUS), or fragile X mental retardation protein (FMRP, FMR1).

In some embodiments, the stress granule comprises tar DNA binding protein-43 (TDP-43), T-cell intracellular antigen 1 (TIA-1), TIA1 cytotoxic granule-associated RNA binding protein-like 1 (TIAR, TIAL1), GTPase activating protein binding protein 1 (G3BP-1), GTPase activating protein binding protein 2 (G3BP-2), fused in sarcoma (FUS), or fragile X mental retardation protein (FMRP, FMR1).

In some embodiments, the stress granule comprises tar DNA binding protein-43 (TDP-43), T-cell intracellular antigen 1 (TIA-1), TIA1 cytotoxic granule-associated RNA binding protein-like 1 (TIAR, TIAL1), GTPase activating protein binding protein 1 (G3BP-1), GTPase activating protein binding protein 2 (G3BP-2), or fused in sarcoma (FUS).

In some embodiments, the stress granule comprises tar DNA binding protein-43 (TDP-43).

In some embodiments, the stress granule comprises T-cell intracellular antigen 1 (TIA-1).

In some embodiments, the stress granule comprises TIA1 cytotoxic granule-associated RNA binding protein-like 1 (TIAR, TIAL1).

In some embodiments, the stress granule comprises GTPase activating protein binding protein 1 (G3BP-1).

In some embodiments, the stress granule comprises GTPase activating protein binding protein 2 (G3BP-2).

In some embodiments, the stress granule comprises tris tetraprolin (TTP, ZFP36).

In some embodiments, the stress granule comprises fused in sarcoma (FUS).

In some embodiments, the stress granule comprises fragile X mental retardation protein (FMRP, FMR1).

***Neurodegenerative diseases:*** Without wishing to be bound by a theory, nucleic acid based TIA-1 inhibitor described herein can be used to delay the progression of neurodegenerative illnesses where the pathology incorporates stress granules. Such illnesses include ALS and frontotemporal dementia (FTD). This group also includes Alzheimer's disease FTLD-U, Huntington's chorea, Creutzfeld-Jacob disease, and multisystem proteinopathy.

The term "neurodegenerative disease" as used herein, refers to a neurological disease characterized by loss or degeneration of neurons. The term "neurodegenerative disease" includes diseases caused by the involvement of genetic factors or the cell death (apoptosis) of neurons attributed to abnormal protein accumulation and so on. Additionally, neurodegenerative diseases include neurodegenerative movement disorders and neurodegenerative conditions relating to memory loss and/or dementia. Neurodegenerative diseases include tauopathies and α-synucleopathies. Exemplary neurodegenerative diseases include, but are not limited to, Alzheimer's disease, frontotemporal dementia (FTD), FTLD-U, FTD caused by mutations in the progranulin protein or tau protein, frontotemporal dementia with inclusion body myopathy (IBMPFD), frontotemporal dementia with motor neuron disease, amyotrophic lateral sclerosis (ALS), amyotrophic lateral sclerosis with dementia (ALSD), Huntington's disease (HD), Huntington's chorea, prion diseases (e.g., Creutzfeld-Jacob disease, bovine spongiform encephalopathy, Kuru, or scrapie), Lewy Body disease, diffuse Lewy body disease (DLBD), polyglutamine (polyQ)-repeat diseases, trinucleotide repeat diseases, cerebral degenerative diseases, presenile dementia, senile dementia, Parkinsonism linked to chromosome 17 (FTDP-17), progressive supranuclear palsy (PSP), progressive bulbar palsy (PBP), psuedobulbar palsy, spinal and bulbar muscular atrophy (SBMA), primary lateral sclerosis, Pick's disease, primary progressive aphasia, corticobasal dementia, HIV-associated dementia, Parkinson's disease, Parkinson's disease with dementia, dementia with Lewy bodies, Down's syndrome, multiple system atrophy, spinal muscular atrophy (SMA, e.g., SMA Type I (e.g., Werdnig-Hoffmann disease) SMA Type II, SMA Type III (e.g., Kugelberg-Welander disease), and congenital SMA with arthrogryposis), progressive spinobulbar muscular atrophy (e.g., Kennedy disease), post-polio syndrome (PPS), spinocerebellar ataxia, pantothenate kinase-associated neurodegeneration (PANK), spinal degenerative disease/motor neuron degenerative diseases, upper motor neuron disorder, lower motor neuron disorder, age-related disorders and dementias, Hallervorden-Spatz syndrome, Lytigo-bodig (amyotrophic lateral sclerosis-parkinsonism dementia), Guam-Parkinsonism dementia, hippocampal sclerosis, corticobasal degeneration, Alexander disease, Apler's disease, Krabbe's disease, neuroborreliosis, neurosyphilis, Sandhoff disease, Schilder's disease, Batten disease, Cockayne syndrome, Kearns-Sayre syndrome, Gerstmann-Straussler-Scheinker syndrome, hereditary spastic paraparesis, Leigh's syndrome, demyelinating diseases, epilepsy, tremors, depression, mania, anxiety and anxiety disorders, sleep disorders (e.g., narcolepsy, fatal familial insomnia), acute brain injuries (e.g., stroke, head injury) and autism.

As used herein, the term "α-synucleopathy" refers to a neurodegenerative disorder or disease involving aggregation of α-synuclein or abnormal α-synuclein in nerve cells in the brain (Ostrerova, N., et al. (1999) J Neurosci 19:5782:5791; Rideout, H.J., et al. (2004) J Biol Chem 279:46915-46920). α-Synucleopathies include, but are not limited to, Parkinson's disease, Parkinson's disease with dementia, dementia with Lewy bodies, Pick's disease, Down's syndrome, multiple system atrophy, amylotrophic lateral sclerosis (ALS), Hallervorden-Spatz syndrome, and the like.

As used herein, the term "tauopathy" refers to a neurodegenerative disease associated with the pathological aggregation of tau protein in the brain. Tauopathies include Alzheimer's disease, Pick's disease, corticobasal degeneration, Argyrophilic grain disease (AGD), progressive supranuclear palsy, Frontotemporal dementia, Frontotemporal lobar degeneration, or Pick's complex.

***Musculoskeletal diseases:*** Musculoskeletal diseases and disorders as defined herein are conditions that affect the muscles, ligaments, tendons, and joints, as well as the skeletal structures that support them. Without wishing to be bound by a theory, aberrant expression of certain proteins, such as the full-length isoform of DUX4, has been shown to inhibit protein turnover and increase the expression and aggregation of cytotoxic proteins (Homma, S. et al. Ann Clin Transl Neurol (2015) 2:151-166). Consequently, nucleic acid based TIA-1 inhibitor described herein may be used to prevent or treat a musculoskeletal disease, *e.g.,* a musculoskeletal disease that results in accumulation of stress granule proteins, *e.g*., in the nucleus, cytoplasm, or cell bodies of a muscle cell or motor neuron. Exemplary musculoskeletal diseases include muscular dystrophy, facioscapulohumeral muscular dystrophy (*e.g*., FSHD1 or FSHD2), Freidrich's ataxia, progressive muscular atrophy (PMA), mitochondrial encephalomyopathy (MELAS), multiple sclerosis, inclusion body myopathy, inclusion body myositis (*e.g*., sporadic inclusion body myositis), post-polio muscular atrophy (PPMA), motor neuron disease, myotonia, myotonic dystrophy, sacropenia, spasticity, multifocal motor neuropathy, inflammatory myopathies, paralysis, and other diseases or disorders relating to the aberrant expression of TIA-1 or tau and altered proteostasis. In addition, nucleic acid based TIA-1 inhibitor described herein may be used to prevent or treat symptoms caused by or relating to said musculoskeletal diseases, *e.g*., kyphosis, hypotonia, foot drop, motor dysfunctions, muscle weakness, muscle atrophy, neuron loss, muscle cramps, altered or aberrant gait, dystonias, astrocytosis (*e.g*., astrocytosis in the spinal cords), liver disease, inflammation, headache, pain (*e.g.,* back pain, neck pain, leg pain, inflammatory pain), and the like. In some embodiments, a musculoskeletal disease or a symptom of a musculoskeletal disease may overlap with a neurodegenerative disease or a symptom of a neurodegenerative disease.

***Retinal diseases:*** Retinal diseases and disorders as defined herein affect the retina and other parts of the eye and may contribute to impaired vision and blindness. Several retinal diseases are characterized by the accumulation of protein inclusions and stress granules within or between retinal cells and nearby tissues. In addition, retinal diseases may also be a symptom of or precursor to neurogenerative diseases, such as ALS and FTD (Ward, M.E., et al. (2014) J Exp Med 211(10): 1937). Therefore, use of compounds that may inhibit formation of protein inclusions and stress granules, including nucleic acid based TIA-1 inhibitor described herein, may play an important role in the prevention or treatment of retinal disease.

Exemplary retinal diseases include, but are not limited to, macular degeneration (*e.g*., age-related macular degeneration), diabetes retinopathy, histoplasmosis, macular hole, macular pucker, Bietti's crystalline dystrophy, retinal detachment, retinal thinning, retinoblastoma, retinopathy of prematurity, Usher's syndrome, vitreous detachment, Refsum disease, retinitis pigmentosa, onchocerciasis, choroideremia, Leber congenital amaurosis, retinoschisis (*e.g.,* juvenile retinoschisis), Stargardt disease, opthalmoplegia, and the like.

***Viral infections:*** Stress granules often form during viral illnesses, as viral infections often involve hijacking the cellular reproductive machinery toward production of viral proteins. In this case, inhibitors of stress granules can be useful for interfering with viral function. Other viruses appear to inhibit SG formation to prevent the cell from mobilizing a stress response. In such a case, an inducer of stress granules can interfere with viral activity and help combat viral infections (*e.g*., Salubrinal, a PERK inhibitor and stress granule inducer). Two viruses for which SG biology has been investigated include West Nile virus and respiratory syncytial virus (RSV) (Emara, M.E. and Brinton, M. A. (2007) Proc. Natl. Acad. Sci. USA 104(21): 9041-9046). Therefore, use of compounds that may inhibit formation of protein inclusions and stress granules, including nucleic acid based TIA-1 inhibitor described herein, may be useful for the prevention and/or treatment of a viral infection.

Exemplary viruses include, but are not limited to, West Nile virus, respiratory syncytial virus (RSV), Epstein-Barr virus (EBV), hepatitis A, B, C, and D viruses, herpes viruses, influenza viruses, chicken pox, avian flu viruses, smallpox, polio viruses, HIV, Ebola virus, and the like.

In some embodiments, the method of treatment comprises the administration of two or more, e.g., three, four, five, or more nucleic acid based inhibitors, e.g., nucleic acid based TIA-1 inhibitors. The TIA-1 inhibitors may, e.g., be directed against different regions of TIA-1, may comprise different chemical modifications, or may comprise different conjugate moieties.

### EXEMPLIFICATION

### Example 1: Microtubule Associated Protein Tau Regulates Stress Granule Biology

Dendritic mis-localization of microtubule associated protein tau is a hallmark of tauopathies, but the role of dendritic tau is poorly understood. This example reports a novel function for tau in regulating dendritic RNA granules. Tau accelerates stress granule (SG) formation and is required for normal interactions of the RNA binding protein TIA1 with proteins linked to RNA metabolism. Loss of tau abrogates interactions of TIA1 with RNA metabolism proteins, including ribosomal proteins and RNA binding proteins. Conversely, reducing TIA1 in vitro or in vivo inhibits pathology and toxicity caused by tau protein, while over-expressing TIA1 induces tau misfolding and stimulates neurodegeneration. The role of tau in translational biology identifies novel pharmacological interventions; preventing SG formation pharmacologically, or via TIA1 knockout, reduces tau granules and blocks tau-mediated neurodegeneration. Conversely, stimulating SG formation increases tau granules. These results present novel functions for tau, and point to new routes for pharmacotherapy of tauopathies.

### Introduction

RNA binding proteins (RBPs) are a class of about 800 proteins that function in the nucleus to regulate mRNA maturation, including splicing, RNA helicase activity, RNA polymerase elongation and nuclear export (Anderson and Kedersha, 2008). RBPs also function in the cytoplasm where they regulate RNA translation, trafficking, sequestration and degradation. RBP function is strongly regulated by the multiple signaling cascades integrated with RNA translation/protein synthesis, which will be referred to as "translational signaling". The cytoplasmic actions of RBPs play an important role in neurobiology because the large distance between the soma and synapse demands a proportionately large role of RBPs in the trafficking of mRNA transcripts (Liu-Yesucevitz et al., 2011).

Increasing evidence links neurological disease processes to dysfunction of neuronal RBPs, RNA granules and stress granules (SGs) (Liu-Yesucevitz et al., 2011; Ramaswami et al.; Vanderweyde et al., 2013; Wolozin, 2012). RBPs are the best characterized protein components that make up RNA granules. SGs are a particular type of RNA granule that accumulates during the translational response to stress. The term "SG" is used when describing RNA granules formed in the presence of an exogenous stressor (e.g., arsenite), or in experiments where multiple SG components are shown; other experiments will be described using the more general term "RNA granule". RBPs, such as T-cell intracellular antigen 1 (TIA1), contain prion-like, poly-glycine rich domains, which promote their physiological, reversible aggregation (Buchan and Parker, 2009; Gilks et al., 2004; Thomas et al., 2011). TIA1 is a core nucleating SG protein. Nucleation is followed by recruitment of secondary RBPs to form a mature SG, which is a key component of stress-induced translational suppression. SGs play a dynamic role in mRNA triage by sorting sequestered mRNAs for re-initiation, storage, or degradation.

Mutations in TAR DNA binding protein 43 (TDP-43), fused in sarcoma/TLS (FUS), ataxin-2, heterogeneous nuclear ribonucleoproteins A1/B2 (hnRNPA1/B2), optineurin, matrin-3, angiogenin and survival motor neuron 1 (SMN1) cause motor neuron diseases, including amyotrophic lateral sclerosis (ALS) (Li et al., 2013). Expansion of the trinucleotide repeats in another RBP, fragile X mental retardation protein 1 (FMRP), cause Fragile X Syndrome (Penagarikano et al., 2007). Many of the mutations in RBPs that are linked to disease appear to increase the tendency of these proteins to aggregate (Johnson et al., 2009; Kim et al., 2013; Kwiatkowski et al., 2009). Studies from our lab and others show that the mutations also increase RNA granule formation, leading to SGs that are larger and more abundant, as well as larger and slower transport granules (Alami et al., 2014; Colombrita et al., 2009; Liu-Yesucevitz et al., 2010b; Liu-Yesucevitz et al., 2014) (Patel et al., 2015). The deleterious effects of disease-linked mutations was recently highlighted by studies with recombinant FUS and hnRNAPA1. These proteins exhibit a normal abililty to cycle between solution and gel phases, forming liquid droplets. However, mutations in either protein impair the phase transition, leading to formation of stable amyloid-like fibrils (Patel et al., 2015) (Nott et al., 2015) (Molliex et al., 2015) (Lin et al., 2015).

SG proteins, including TIA1, co-localize with neuropathology in brain tissue of subjects with Alzheimer's disease (AD), frontotemporal dementia with parkinsonism (FTDP-17), frontotemporal lobar dementia (FTLD-TDP), ALS, Huntington's disease, Creutzfeld-Jacob disease, and spinomuscular atrophy, as well as in animal models of these diseases (Kedersha et al.; Liu-Yesucevitz et al., 2010b; Thomas et al., 2011; Vanderweyde et al., 2012; Wolozin, 2012). The biology of tau is intimately linked to TIA1, with the proteins accumulating concomitantly with each other over the disease course in brain tissue from subjects with human tauopathies as well as animal models of tauopathies (Vanderweyde et al., 2012).

Tau promotes SG formation and modulates the patterns of protein interactions of TIA1, a main SG component. The interaction between tau and TIA1 promotes tau misfolding and assembly at the site of SGs, and results in the degeneration of processes and stimulation of apoptotic markers in primary neurons. Reducing TIA1 inhibits tau misfolding and reduces synaptic loss both *in vitro* and *in vivo.* These results indicate that tau plays an important role in neuronal RBP biology, suggest a novel mechanism for mis-folding of tau and raise the possibility that the pathophysiology of tauopathies, such as AD, is associated with dysfunction of RBP biology.

### Results:

### Tau increases somatodendritic localization of TIA1

The distribution of TIA1 was examined in primary cultures of hippocampal neurons from WT and Tau -/- mice to investigate whether tau regulates the distribution of TIA1 (Fig. 1A). Primary hippocampal neurons from Tau -/- mice were transduced with TIA1-GFP ± AAV1 WT Tau-V5 or P301L Tau-V5. TIA1 exhibited a strong nuclear localization in neurons from tau^{-/-} mice, with few TIA1 granules (Fig. 1A). Expressing TIA1 plus tau dramatically increased the amount of somatodendritic TIA1, with the TIA1 exhibiting a strong granular character (Fig. 1A). Quantification shows that P301L tau significantly increased the size of TIA1 SGs compared to WT tau, but produced fewer SGs than WT tau (Fig. 1A-1C), which is strikingly similar to effects produced in neurons by disease-linked mutations in RBPs, such as TDP-43 (Liu-Yesucevitz et al., 2014).

The ability of tau to regulate TIA1 RNA granule formation suggests a biological role for tau in RNA granule trafficking, which was investigated using live cell imaging. Tau^{-/-} neurons (DIV 3) were transduced with AAV1-TIA1-mRFP (a monomeric form of RFP) ± AAV9-WT or P301L tau, and at DIV 21, the neurons were imaged. Compiled traces showing particle localization and tracks of TIA1 granules show decreased granule movement with tau expression (Fig. 1D-1G). Tau inhibited net displacement and velocity of TIA1 granules, with retrograde (-) movement inhibited somewhat more than anterograde (+) movement (Fig. 1H). Granule size was inversely correlated with granule velocity (Figs. 1D-G), with the trend particularly pronounced with P301L tau where the granule area vs velocity graph shows a distinct inflection at about 1.2 µm² (Fig. 1G). As with SG formation, this effect is strikingly similar to the relationship between size and granule movement among TDP-43 granules (Liu-Yesucevitz et al., 2014) (Alami et al., 2014).

### Tau increases SG number and size

Next the effects of tau on SG formation were investigated; HT22 cells were used, which are derived from mouse hippocampal neurons, to facilitate the studies under stress conditions. Similar to the observations of hippocampal neurons, expressing human tau (4R0N WT and P301L) increased SG formation in the HT22 cells (Fig. 1I). HT22 cells were transduced with human tau (4R0N WT and P301L) and TIA1, and then examined ±arsenite (0.5 mM, 30 min) to induce SGs, imaging the cells every minute for 20 min. Tau over-expression strongly increased RNA granule formation under basal and stressed conditions (Figs. 1I, 8A, 8B). Double labeling of these TIA1 granules demonstrated that they are bona-fide SGs because they are positive for 2 SG markers: TIA1 and PABP; in addition, co-treatment with 10 µg/ml cycloheximide prevented formation of the tau/SG complexes, while treatment with 25uM salubrinal enhanced SG levels (Figs. 1I, 8C-8F); salubrinal is a GADD34 inhibitor that increases and prolongs SG formation (Boyce et al., 2005).

The interaction between TIA1 and tau was also evident biochemically. Immunoprecipitations (IP) were performed to test whether TIA1 associates with tau. HT22 cells were transfected with WT tau (4R0N) and TIA1, and the human specific Tau13 antibody was used to IP the complex, followed by immunoblotting for TIA1; IP with anti-TIAl followed by immunoblotting with Tau13 was also performed, which produced compelling evidence indicating robust associations (Fig. 1J).

Because SGs are an important element of the stress response, and the data in Figs. 1A-1J indicate that tau enhances SG formation, the live cell imaging approach was applied to examine the effects of tau on stress granule dynamics. Expressing tau with TIA1-RFP strongly increased the rate of SG formation (Figs. 8G-8I). P301L tau accelerated the rate of SGs formation more than WT tau, with increased consolidation into larger SGs (Figs. 8G-8J). Thus, tau accelerates SG formation. Taken together, these studies point to an important role of tau in regulating trafficking and assembly of RNA granules, including SGs.

SGs are thought to reflect adaptation of protein synthesis to stress; typically the stress-induced changes in protein synthesis are associated with an overall reduction in protein synthesis. Analysis of cells over-expressing tau (4N0R, P301L) showed a decrease in total protein synthesis in response to the tau over-expression, which is consistent with the data above suggesting that tau promotes SGs and the translational stress response (data not shown).

### Tau regulates the interaction of TIA1 with its proteome

Proteomic studies revealed a surprising, novel role for tau in regulating the proteins that interact with TIA1. To investigate whether tau exerts control over TIA1 protein interactions, TIA1 was immunoprecipitated from cortical brain tissue of 10 month-old WT (C57BL/6J), tau^{-/-} and TIA1^{-/-} mice. The specificity of the TIA1 immunoprecipitation was verified by immunoblotting with anti-TIAl antibody (Fig. 2C), and the resulting TIA1 proteome was examined by mass spectroscopy. 163 proteins were identified that were present in TIA1 immunoprecipitates from WT or tau^{-/-} brains and absent in the TIA1 immunoprecipitates from TIA1^{-/-} brains (Table 1). Protein associations identified by proteomics were validated by repeat mass spectrometry on fresh samples, as well as by immunoblot for tau and 3 proteins that gave strong signals by mass spectrometry: RPL7, EWSR1, DDX5 (Fig. 2C). Gene ontology (GO) and functional (KEYWORD) terms showing statistically significant enrichment (False Discovery Rate <0.2) in the TIA1 associated proteome for either WT or tau^{-/-} cortex were determined using the Database for Annotation, Visualization and Integrated Discovery (DAVID) bioinformatics resource available through the NIH (Figs. 2A and 9A) (Huang da et al., 2009). A network was constructed to understand shared functional roles played by TIA1 binding proteins in each condition. In the network, lines connecting proteins indicate shared annotated functions (Fig. 2B). The size of the node corresponds to the degree of replications in the WT samples (Fig. 2B). As expected, immunoprecipitating TIA1 from WT mouse brain identified proteins that were enriched for RNA metabolism (Fig. 2A); proteins involved in mitochondrial function and vesicular/synaptic function also exhibited significant enrichment (Fig. 2A). The network of TIA1 binding proteins in WT mouse cortex includes multiple proteins linked to RNA metabolism, such as ribosomal proteins (e.g., RBL6, 7, 10A, 13 and 13A, MRPL46, RPS3 and 4X), translational regulatory proteins (EIF4A1, PABC1 and NACA), small nuclear ribonucleoproteins (SNRNP70 and SNRPB), heterogeneous nuclear ribonucleoproteins (HNRNPF, HNRNPR and HNRNPUL2, EWSR1 and SYNCRIP) and helicases DDX5 and 17 (Fig. 2B). Importantly, deletion of tau reduced interactions of TIA1 with many proteins in the network, including those associated with RNA metabolism (Figs. 2A and 2B). This network analysis of the TIA1 binding proteomes from the WT and tau^{-/-} mouse brain highlights the important role that tau plays in regulating proteins that interact with TIA1, with loss of tau abrogating interactions with multiple core TIA1 binding proteins including, EWSR1, RPL6, RPL7, MRPL46, RBM17, and SNRNP70 (Fig. 2B). The interaction of TIA1 with proteins, such as SIRT2, is novel and might point to regulatory interactions unique to the brain. The prominence of members of the U1 SNRNP family (SNRNP70, SNRPB and RBM17) in the TIA1 network is striking (Fig. 2B). Previous work identified strong accumulation of cytoplasmic SNRNP70 aggregates in the AD brain (Bai et al., 2013). The requirement of tau for inclusion of SNRNP70 in the TIA1 network points to the putative biological importance of tau in SNRNP70 function and raises the possibility that the accumulation of SNRPNP70 in the AD brain reflects the pathophysiology of tau. More generally, these results suggest that the presence of tau protein is required for normal association of TIA1 with the RNA metabolism machinery.

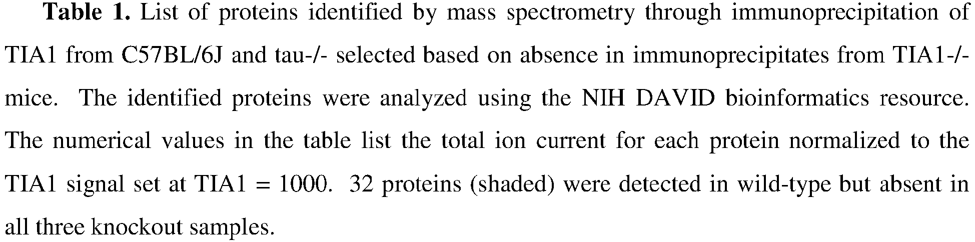

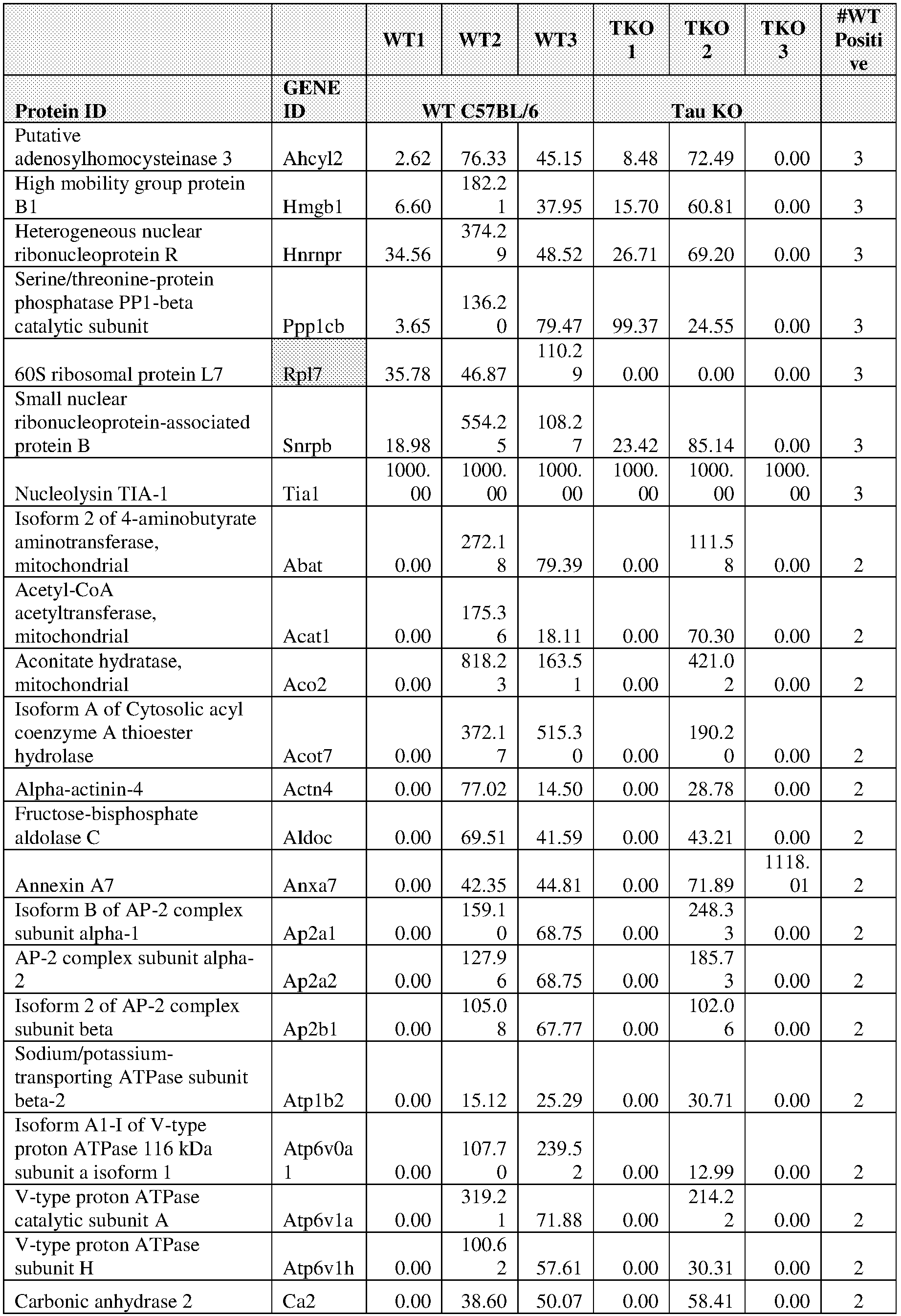

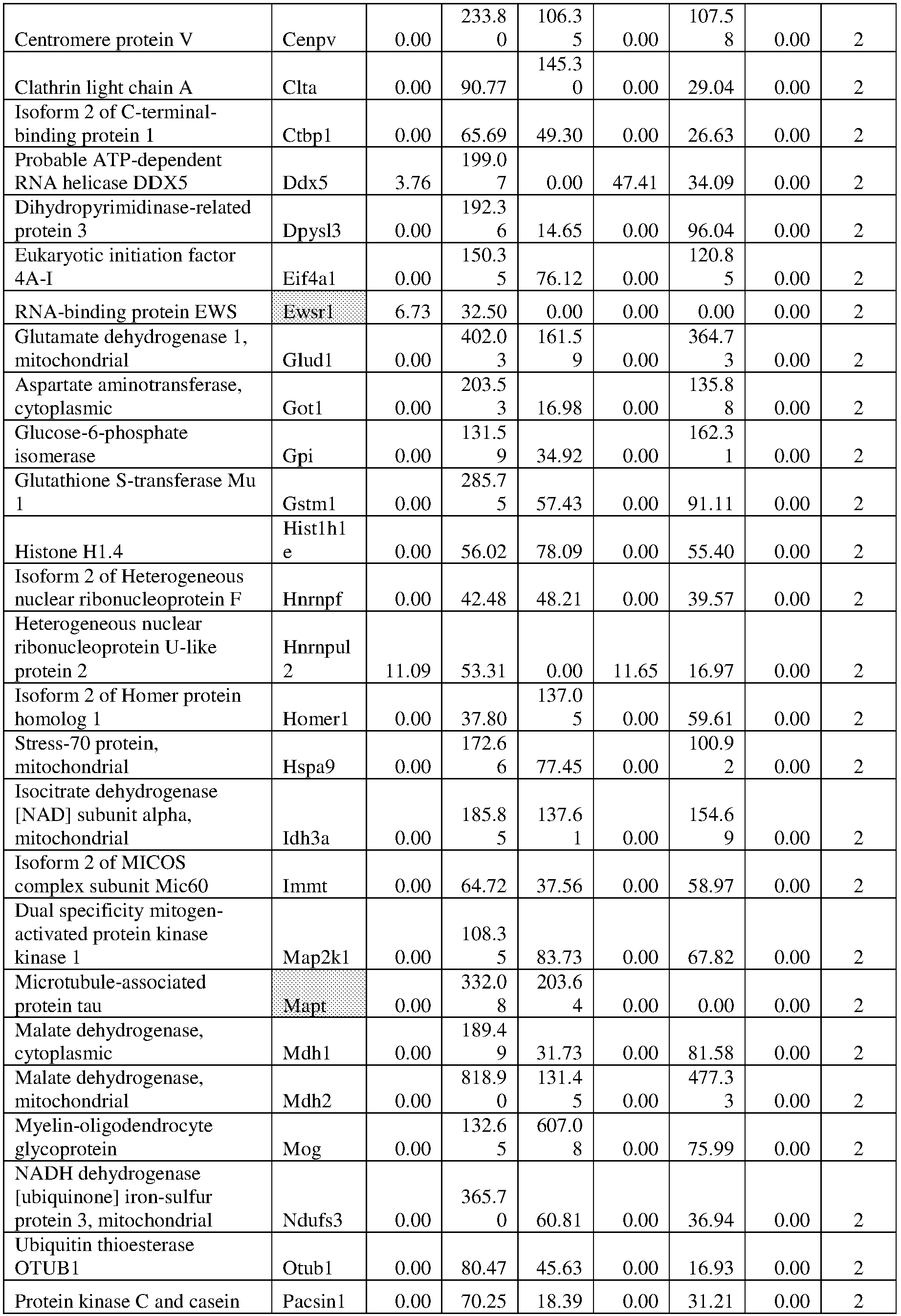

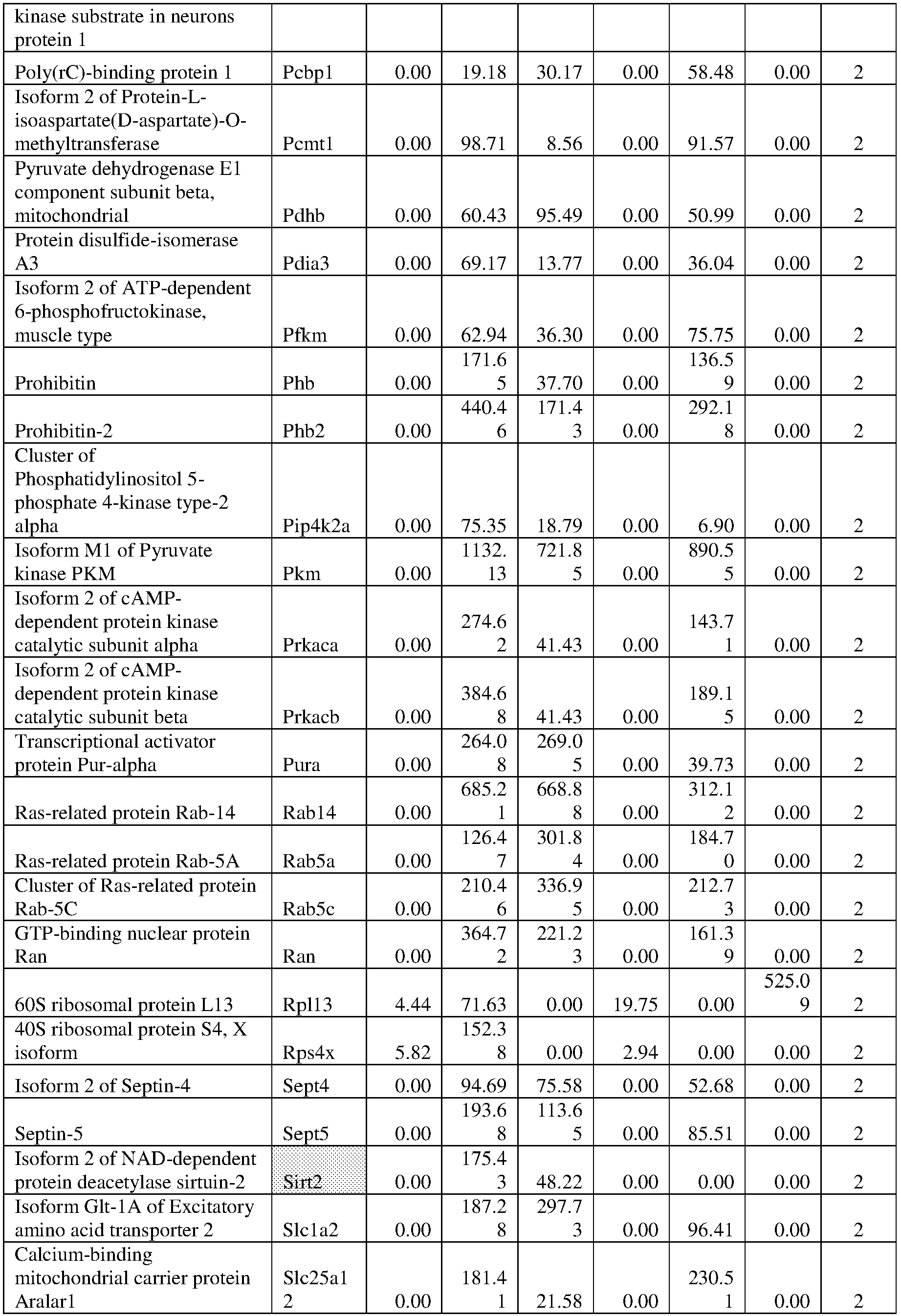

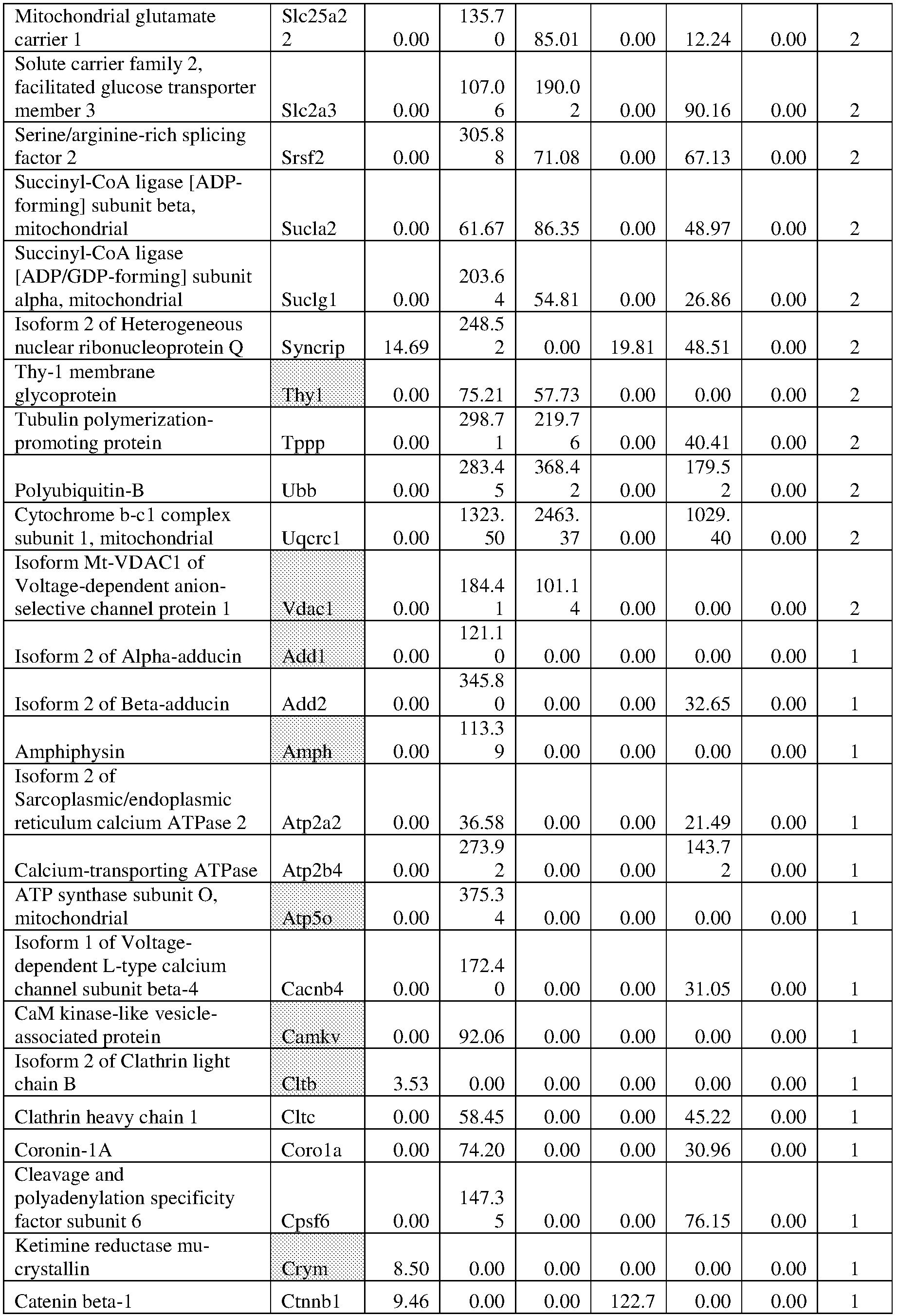

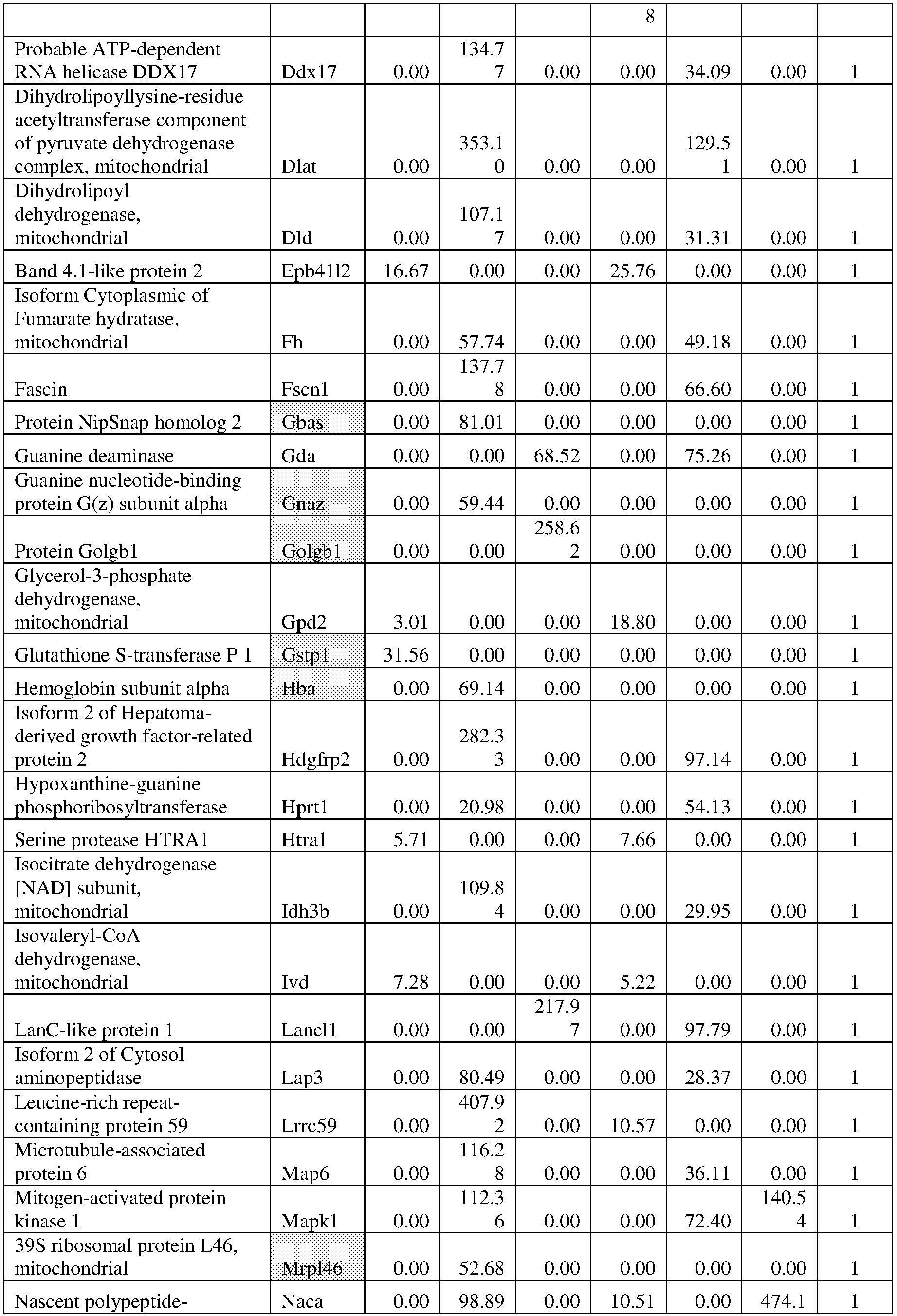

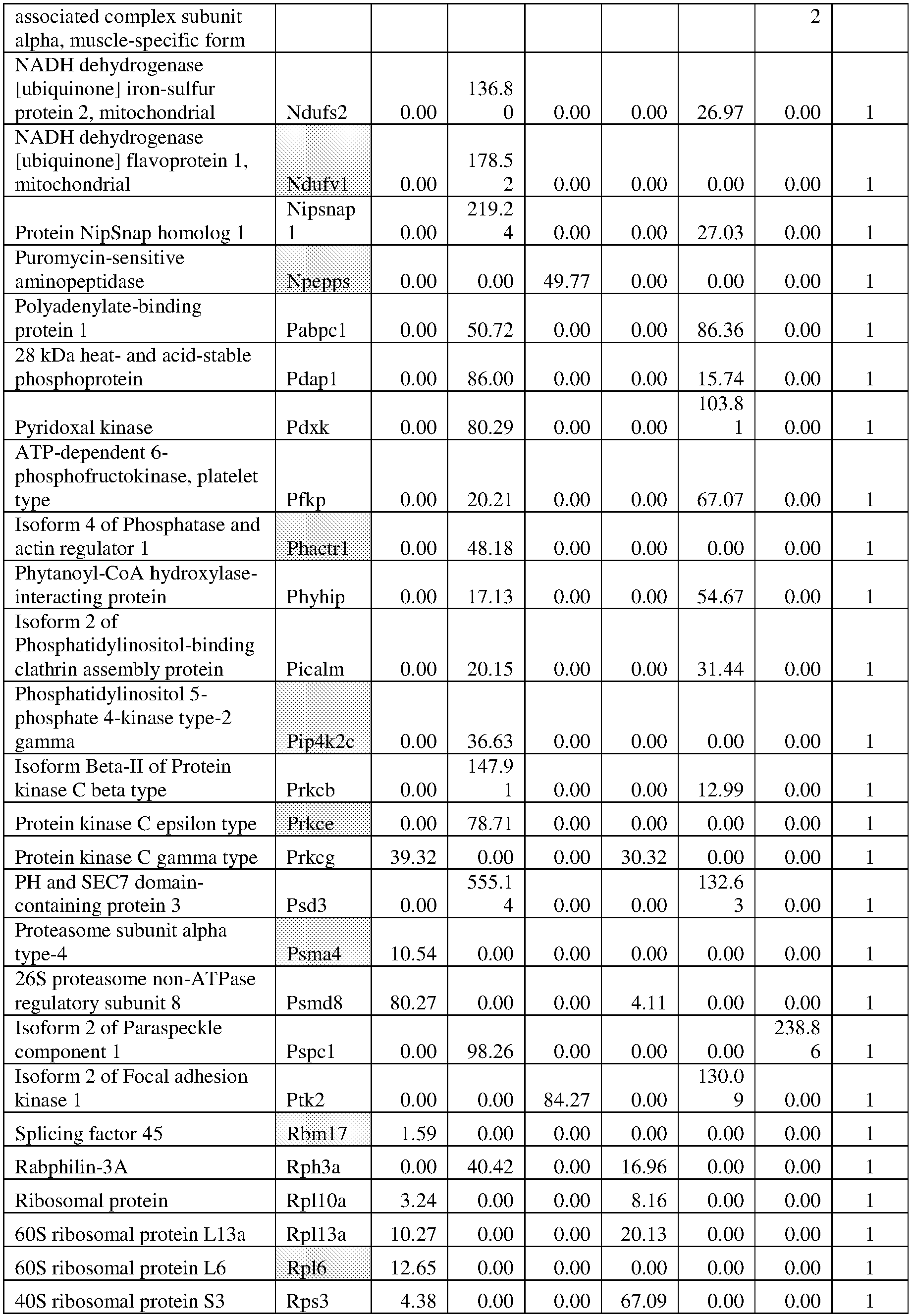

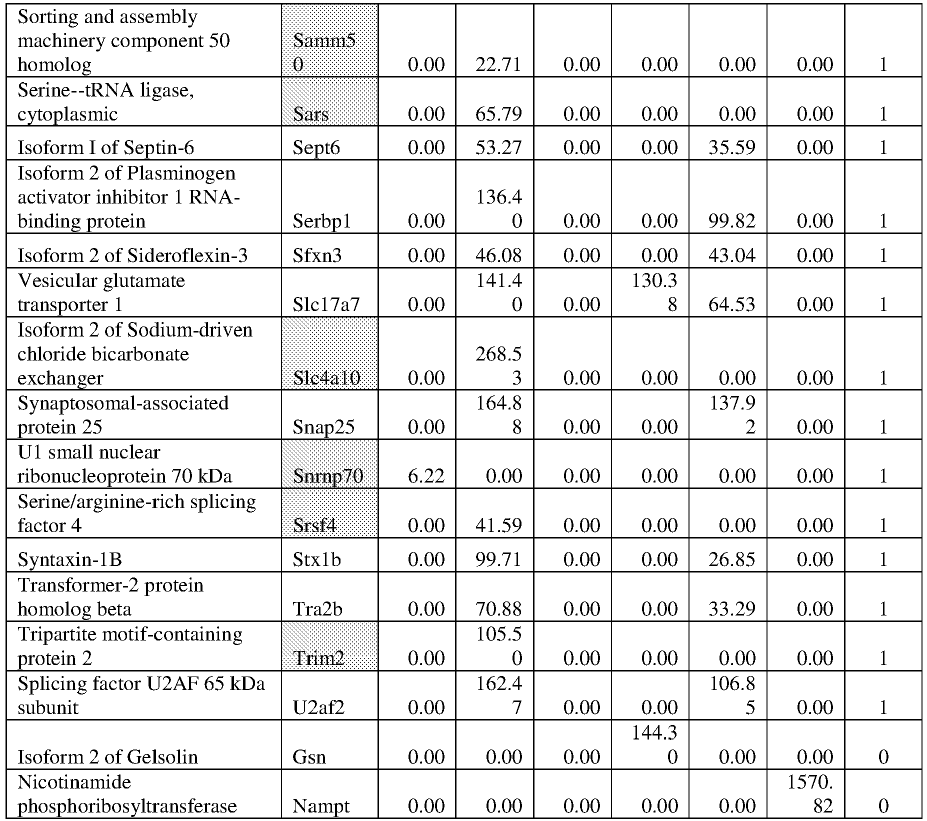

### RNA binding proteins in the TIA1 proteome co-localize with tau pathology in vivo

TIA1 co-localizes with tau pathology *in vivo.* The strong role of tau in the TIA1 binding proteome raised the possibility that TIA1-associated RBPs might co-localize with tau pathology in human tauopathies and mouse models of tauopathy. Brain tissue (frontal cortex) from 11 month old rTg4510 mice were labeled with antibodies for TIA1, EWSR1, hnRNPD, RPL7, TDP-43 and FUS (Figs. 2D and 9B); all samples were co-labeled with PHF1. Proteins present in the TIA1 network (TIA1, EWSR1, hnRNPD and RPL7) all co-localized with PHF1-positive tau pathology. The RPL7 reactivity was notable because it tended to localize to the outer rim of tau pathology (Fig. 2D). No co-localization with PHF-1 positive tau pathology was observed for TDP-43 or FUS, which are not part of the TIA1 binding network (Figs. 2D and 9B).

### TIA1 and tau interact biochemically

The association of tau with TIA1 was evident in primary neuronal cultures too. Hippocampal neurons from tau^{-/-} mice were grown in culture, and transduced with AAV9-WT or P301L tau ± AAV1-TIA1-mRFP (or mRFP) (Fig. 3A). IP with TIA1 indicated that total tau (Tau13, a human specific antibody that recognizes total tau), phospho-tau (PHF1, an antibody that recognizes tau phosphorylated at S396/404), and misfolded tau (MC1, a monoclonal antibody that detects a misfolded tau epitope that is associated with tauopathies) bound exogenous and endogenous TIA1 (Fig. 10A). Pre-treating the lysate with RNase, or use of a TIA1 construct lacking the 3 RNA recognition motifs abolished the association, indicating that tau associated with TIA1 through an RNA intermediate (Fig. 3B, data for RNase A treatment shown). The role of RNA intermediates supports the hypothesis that tau participates in RNA granule biology. Tau could interact with RNA directly but in a sequence independent manner, as suggested by prior studies, or in a sequence dependent manner through binding to a RNA binding protein (Kampers et al., 1996) (Wang et al., 2006).

Despite the dependence of an RNA intermediate for association of TIA1 with tau, imaging showed the two proteins in close apposition to one another. Super-resolution microscopy, which enables imaging at the nanometer scale, revealed an intimate physical proximity of TIA1 and tau in neurites. DIV21 mouse hippocampal tau^{-/-} neurons were transduced with AAV1-TIA1-mRFP and AAV9-WT or P301L tau, fixed and labeled with MC1. The results showed an intimate association between TIA1 and tau (data not shown). In the absence of tau, TIA1 exhibited reduced consolidation (data not shown). These data indicate that TIA1 increases the number and size of MC1⁺ granules in primary neurons, perhaps reflecting the ability of TIA1 to promote regulated protein aggregation, which is known to occur with TIA1⁺ SGs (Gilks et al., 2004). In this context, it is notable that both tau and TIA1 have large stretches of low-complexity sequences that can foster prion-like aggregation.

### TIA1 regulates tau levels

Comparison of levels of endogenous mouse tau in DIV21 primary cortical neurons from TIA^{-/-} vs. WT (C57B1/6J) demonstrated that endogenous TIA1 regulates levels of total tau protein. Immunoblots showed a lack of endogenous TIA1 (eTIA1) in the TIA1^{-/-} mice, and a corresponding increase in total tau levels compared to the control strain (Fig. 3C). Despite increasing levels of tau, reducing TIA1 decreased tau misfolding, as shown by MC1 levels. Knockdown of TIA1 with shTIA1 elicited a strong reduction in TIA1 levels, as demonstrated by immunoblot (Fig. 3D); note that the knockdown was done in HT22 cells because of the high efficiency of transduction. Next, primary hippocampal neurons were infected with WT human tau and transfected with shTIA1 or scrambled (shScr) to knock down TIA1. Analysis of MC1 reactivity at DIV 21 demonstrated that TIA1 knockdown elicited a robust decrease in MC1 levels (Figs. 3E and 3F). TIA1 knockdown also reduced formation of MC1 tau granules in HT22 cells (Figs. 10A-10F). The mechanism through which TIA1 regulates levels of tau protein appeared to have limited dependence on the proteasomal or autophagic systems because neither MG132 nor chloroquine prevented decreases in tau associated with TIA1 over-expression in HT22 cells (Fig. 10G).

### TIA1 increases the stability and insolubility of granular tau

Over-expressing TIA1 exhibited a robust reciprocal response, increasing levels of MC1 reactivity. Tau^{-/-} hippocampal neurons were transduced with WT or P301L tau AAV9 and TIA1-GFP or GFP lentivirus, and immunolabeled for MC1 tau and TIA1. Imaging demonstrated that neurons co-expressing tau and TIA1 displayed abundant MC1⁺ granules in processes that were not apparent in neurons expressing tau alone, and that co-expressing TIA1 increased the size of the granules (Figs. 4A-4C).

The ability of TIA1 to promote tau granules led to the hypothesis that TIA1 increased the stability of tau in granules. To test this hypothesis, photo-convertible tau (PC-Tau, WT 4N0R) construct was generated, that stably converts from cyan to green, transfected cortical neurons (DIV 5) with the PC-Tau ± TIA1, performed the photo-activation at DIV 21, and then imaged for 6 hrs. Neurons transfected with PC-Tau plus mCherry control exhibited green tau fluorescence present both diffusely and in granules. The tau fluorescence decreased steadily over 6 hrs down to a level of approximately 40% of the original fluorescence levels (Figs. 4D and 4E). In contrast, cortical neurons transfected with PC-Tau + TIA1 exhibited greatly reduced decay rates, with PC-Tau fluorescence remaining above 80% at 6 hrs (Figs. 4D and 4E). Expressing tau with TIA1 also caused a higher proportion of the tau to localize to granules, consistent with Figures 4A-4C. These data indicate that TIA1 stabilizes tau in granules.

The stabilization of tau in granules combined with the presence of misfolded, MC1⁺ tau in SGs raised the possibility that TIA1 increases formation of insoluble tau in granules. To test this hypothesis, HT22 cells were transfected with WT tau or P301L tau ± TIA1-RFP (Fig. 11A). TIA1 was immunoprecipitated; the TIA1 bound and unbound fractions were biochemically fractionated into sarkosyl soluble and insoluble fractions. Over-expressing TIA1 promoted the clearance of tau in all fractions except the insoluble-TIA-bound (Fig. 1A) where the amount of insoluble tau (WT and P301L) associated with TIA1 was increased (Figs. 11A and 11B). Thus these findings suggest that association with TIA1 promotes formation of insoluble tau, which is thought to be a key step in forming neurofibrillary tangles.

### Translational inhibitors and kinase inhibitors modulate SGs and tau granules in dendrites

The interaction between tau and TIA1 points to novel approaches for modulating formation of tau granules in neurons. SGs are regulated by translational inhibition. Cycloheximide prevents elongation which leaves ribosomes stalled on mRNA and inhibits SG formation, while puromycin causes premature translational termination which release of the 60S ribosomal subunit from the mRNA, promoting SG formation. Analysis of tau granules in neuronal cell lines demonstrated that cycloheximide prevented formation of tau granules (Figs. 1A, 8A, and 8B), while puromycin stimulated formation of tau granules (data not shown). It is hypothesized that tau granules might be regulated in a similar manner in neuronal dendrites.

Primary cultures of tau^{-/-} hippocampal neurons were transduced with AAV9-WT tau or P301L tau ± AAV1-mRFP or TIA1-mRFP (Figs. 5A-5G). Neurons expressing WT or P301L tau and mRFP control exhibited tau that was spread relatively diffusely along processes, with only weak granules evident (Fig. 5A). However, co-transducing WT or P301L tau with TIA1 resulted in processes with large granules positive for tau and TIA1, and little to no diffuse tau (Fig. 5A, arrows). Comparison of the effects of the two different translational inhibitors, puromycin and cycloheximide, highlighted the role of translational signaling. Neurons were treated at DIV21, immunolabeled for Tau13 and MAP2 and imaged (Figs. 5B and 5C). Treatment with puromycin yielded larger and more abundant tau granules that were particularly accentuated by TIA1/tau over-expression (Fig. 5B). Conversely, treatment with cycloheximide (10 µg/ml) yielded dendritic tau (and TIA1) that was spread diffusely with only small, less defined granules apparent (Fig. 5C). Thus, the localization and granule formation of tau and TIA1 are both modulated by translational signaling.

The role of tau in SG biology in neurons also suggests that kinases that regulate tau dynamics might also regulate tau-mediated SG formation in neurons. For instance, proline-directed kinases are known to phosphorylate tau, which leads to dissociation of tau from microtubules and increases the propensity of tau to aggregate (Lee et al., 2011; Matenia and Mandelkow, 2009). Chemical inhibitors of GSK3β, CDK5, p38, MARK and Fyn all significantly inhibited formation of granules positive for TIA1 and phospho-tau (P-S396/404, PHF1) (Figs. 5D and 5E). The strongest SG inhibition was observed with the p38 inhibitor, which is known to act downstream of each of these kinases (Roux and Blenis, 2004).

Use of phospho-mimetic tau constructs demonstrated a direct role for tau phosphorylation in modulating SG formation. Transfections were performed using phospho-mimetic (PMIM) or phospho-null (PNULL) tau constructs in which 14 sites exhibiting increased phosphorylation in AD were replaced with either aspartate or alanine (Hoover et al., 2010). HT22 cells were transfected with P301L, P301L PMIM, or P301L PNULL tau ± TIA1-RFP. Cells were treated ± 25 µM salubrinal and SG were imaged for endogenous TIA1 (Figs. 12A and 12C) or transfected TIA1-RFP (Figs. 12B and 12C). Imaging showed more SGs in the presence of phospho-mimetic tau, and fewer SGs in the presence of phospho-null tau (Figs. 12A-12C). Salubrinal treatment increased the number of SGs in all conditions; salubrinal inhibits the PP2A adapter protein, GADD34, which increases eIF2α phosphorylation and reduces mRNA translation (Boyce et al., 2005). These data point to direct phosphorylation of tau as a modulator of SG and tau granule formation in neurons.

The eukaryotic translation initiation factor eIF2α is regulated by phosphorylation. We investigated whether inhibiting PKR or PERK, two kinases that phosphorylate eIF2α, might also inhibit tau mediated SG formation. SGs were induced by transfection with TIA1 ± WT tau. After 24 hrs the cells were treated with inhibitors of PKR (C16, 1 µM, Sigma) or PERK (GSK2606414, 50 nM, EMD/Millipore), and then SG number was quantified after 24 hrs. PKR or PERK antagonists strongly inhibited tau-mediated SG formation (Figs. 5F and 5G). Thus, inhibiting translationally directed kinases also decreases tau-mediated SG formation.

### TIA1 decreases dendritic length of tau expressing primary hippocampal neurons

The results suggest a functional interaction between TIA1 and tau, with tau promoting formation of TIA1⁺ SGs, and TIA enhancing both tau catabolism and tau consolidation. The functional interactions between TIA1 and tau also extend to neurodegeneration, where these results show that TIA1 knockout inhibits tau-mediated degeneration, while TIA1 over-expression increases tau-mediated degeneration. Effects on degeneration were investigated. Hippocampal neurons (DIV3) from Tau -/- or TIA1 -/- mice were transduced with AAV9-WT or P301L tau ± AAV1-TIA1-mRFP or mRFP and imaged for MAP2 at DIV21 (Figs. 6A and 6B). Co-expressing TIA1 with tau significantly decreased dendritic length, but had no effect independent of tau or TIA1 (Figs. 6A-6C). P301L tau also caused toxicity on its own; neurons transduced with P301L tau exhibited significant dendritic shortening compared to neurons transduced with WT tau. However, TIA1 knockout prevented this toxicity. Hippocampal neurons (DIV3) from TIA1^{-/-} mice transduced with AAV9-P301L tau exhibited neurite lengths similar to neurons transduced with AAV9-WT tau (Fig. 6C). These data indicate that TIA1 expression is necessary for dendrite shortening associated with expression of P301L tau (Fig. 6C).

Further studies suggest that the modulation of dendritic length by TIA1 and tau is sensitive to translational signaling. Tau^{-/-} and TIA1^{-/-} primary hippocampal neurons were transduced with AAV9-WT tau or P301L tau ± AAV1-TIA1-mRFP or mRFP, and at DIV21 treated with translation inhibitors puromycin (5 µg/ml) or cycloheximide (10 µg/ml). Translation inhibition with cycloheximide did not affect dendrite length, while treatment with puromycin, which induces SGs, potentiated the decrease in dendritic length associated with TIA1/tau over-expression (Figs. 6D and 6E).

Induction of toxicity was also apparent using biochemical assays. Levels of synaptic and apoptotic markers were examined by immunoblot in WT primary cortical neurons transduced with AAV1-mRFP or TIA1-mRFP ± AAV9-WT tau or P301L tau. Markers examined included synaptophysin, PSD-95, caspase-3, and cleaved caspase-3. The data indicate a striking loss in the pre-synaptic marker synaptophysin in neurons co-transduced with tau and TIA1, indicating a corresponding loss of axonal terminals (Fig. 6F). Levels of cleaved caspase-3 were also elevated in TIA1 and tau co-transduced neurons indicating enhanced toxicity (Figs. 6F and 6H), which was potentiated by concurrent treatment with 25 µM salubrinal (Fig. 6H). Interestingly, changes in the post-synaptic marker PSD-95 levels were not prominent (Fig. 6F). Analysis of DNA fragmentation also showed that TIA1 increased apoptosis in tau expressing neurons (Fig. 6G). These data suggest that the interaction of TIA1 with tau can promote neurodegeneration under conditions where SG formation is enhanced, such as occurs with TIA1 over-expression or exposure to SG inducers.

### TIA1 haplo-insufficiency reduces tau-mediated degeneration

*In vivo* studies demonstrate that TIA1 is also required for tau mediated neurodegeneration. The role of TIA1 in tau-mediated neurodegeneration was investigated by crossing P301S Tau mice with TIA1 knockout mice. Markers of tau pathology and degeneration were examined at 2.5 months, when synaptic loss and tau hyperphosphorylation are first evident in the hippocampus. Synaptic loss was apparent in the dentate gyrus and CA1 regions of the 2.5 m P301S mice, as reported previously (Fig. 7A). Tau hyperphosphorylation was also apparent with the PHF1 antibody (Fig. 7B); some enhanced reactivity was also apparent with the MC1 and CP13 antibodies, but to a lesser degree (data now shown). Importantly, P301S tau / TIA1 +/mice showed no synaptic loss (Fig. 7A, synaptophysin panels) and no reactivity for the anti-tau antibodies PHF1, MC1 or CP13 (Fig. 7A and data not shown).

The loss of tau pathology was also apparent with biochemical fractionation. Hippocampal and cortical brain samples from the P301S crosses were biochemically fractionated and then immunoblotted. As expected, the P301S tau mice had abundant levels of total and phosphorylated tau in the sarkosyl insoluble fraction (Fig. 7B). However total and phosphorylated tau showed striking reductions in the P301S tau/TIA1^{+/-} mice, despite the absence of any change of total tau in the total lysate fraction (Fig. 7B). Taken together, these data indicate that haplo-insufficiency of TIA1 inhibits progression of tauopathy, indicating the importance of TIA1 in the disease process.

### Discussion

Tau is classically considered to function as a microtubule binding protein that plays an important role in axonal trafficking, however in tauopathies tau accumulates in the somatodendritic compartment where it forms protein aggregates. The cellular logic behind somatodendritic accumulation is poorly understood. These results suggest that the shift in tau localization to the somatodendritic compartment occurs to facilitate formation of SGs, which are RNA/protein complexes that are part of the translational stress response. SGs normally accumulate in the soma and dendrites as small insoluble macromolecular complexes in response to stress. In neurodegenerative diseases SGs become very large, and associate with pathological proteins, such as tau (in AD) and TDP-43 (in amyotrophic lateral sclerosis) (Vanderweyde et al., 2012) (Liu-Yesucevitz et al., 2010a). In moderation, this stress response is likely beneficial, but an over-active SG response causes a deleterious, degenerative response, such as that caused by over-expressing tau and either co-expressing with TIA1 or treating with puromycin.

TIA1 is known to be a protein involved in nuclear splicing, but recent studies also show that it is one of the core proteins that nucleates cytoplasmic SGs (Anderson and Kedersha, 2008). The network of proteins that associate with TIA1 in the brain includes 14 proteins that are very strongly linked from a functional perspective. This group of proteins includes RBPs typically associated with the spliceosome, (snRNP70, SNRPB, DDX5 and RBM17), RBPs associated with mRNA transport (HNRNPR and EWSR1) and multiple ribosomal proteins (e.g., RPL6, 7 10A, 13, 13A, RPS 3, 4X). The prominence of ribosomal proteins highlights the important role of RNA translation in this network. Loss of tau abrogates TIA1 binding to 5 out of 14 proteins in this core network, RPL6, RPL7, EWSR1, SNRNP70 and RBM17, which points to a role for tau in this translational and transport machinery (Fig. 3B). TIA1 shows reduced dendritic localization in tau^{-/-} neurons (Figs. 1A and 1B), which suggests altered interactions with trafficking proteins, but the mechanism for this altered localization remains to be determined. The interaction between tau and TIA1 parallels a recent study demonstrating that TIA1 interacts with tubulin to regulate microtubules in yeast (Li et al., 2014). The presence of novel TIA1 binding proteins whose binding is tau dependent, such as SIRT2 and clathrin (CLTB), points to interactions that might be more prominent in neurons or glia than in somatic cells.

The TIA1-binding proteome might differ between neurons and most peripheral cells because neurons must manage RNA biology in dendrites and synapses. RNA must be transported to the synapse, where RNA translation is tightly linked to synaptic activity through activity-dependent translation. This means that RBPs exert a much larger footprint on cellular activity outside of the nucleus in neurons (and possibly glia) than in somatic cells. The prominence of ribosomal proteins in the brain TIA1 network combined with the presence of RBPs important for RNA transport, such as HNRNPR, SYNCRIP and EWSR1, emphasizes an important, novel role for tau in regulating RNA transport and translation during stress. Under basal conditions, tau is present in dendrites only at low levels (Frandemiche et al., 2014). However, recent studies demonstrate that stress causes tau to localize to the somatodendritic compartment (Hoover et al., 2010; Stamer et al., 2002; Zempel et al., 2013); (Zempel and Mandelkow, 2014). Tau might function in this context to slow RNA granule transport and regulate the interaction of TIA1 with other SG proteins, which would facilitate SG formation and the translational stress response.

The immunohistochemical studies of the TIA1 proteome components complement existing studies to highlight an important role for the TIA1 proteome network in the pathophysiology of AD and other tauopathies. Each of the 5 RBPs examined co-localized with tau pathology. SNRNP70 also exhibits a strong tendency to aggregate, and has recently been shown to accumulate as an insoluble cytoplasmic aggregate in AD (Bai et al., 2013; Diner et al., 2014). Its aggregation is mediated by the low complexity prion-like domains shared in common with other RNA binding proteins (Diner et al., 2014); SNRNP70 is also a component of RNA granules, similar to other RBPs (Beaudoin et al., 2009; Buckingham and Liu, 2011). These results suggest that SNRNP70 interacts with TIA1 through a tau-mediated complex because loss of tau causes loss of SNRNP70 from the TIA1 network. The network also identifies RPL7 as another TIA1-interacting protein whose binding is also dependent on the presence of tau, and which has also been observed to be associated with tau pathology (Minjarez et al., 2013). These intersecting pieces of evidence suggest a model in which the accumulation of aggregated SNRNP70 and RPL7 in tauopathies might result from shared hyperactive SG pathways that also leads to the accumulation of aggregated tau.

TIA1 is a core-nucleating component of SGs. Deletion or knockdown of TIA1 reduces the ability of cells to form SGs. Our data indicate bidirectional regulation of tau and TIA1 because reducing TIA1 reduces tau misfolding and toxicity. P301L tau exhibits no toxicity in cultured neurons lacking TIA1. This observation was also strikingly apparent *in vivo,* where haplo-insufficiency reduces the accumulation of phosphorylated tau and prevents synaptic loss. The sensitivity of tau pathology to levels of TIA1 is particularly important because it suggests novel approaches to pharmacotherapy. Homozygous TIA1 deletion causes arthritis and immune dysfunction, but mice heterozygous for TIA1 do not exhibit a strong immunological phenotype, despite showing reduced neurodegeneration (Phillips et al., 2004). These results suggest that RBPs are an important element of the biological processes associated with tau pathology and degeneration.

Protein aggregation in neurodegenerative disease has been classically considered to result from dysfunctional protein misfolding. Chaperones prevent misfolding, but when proteins do misfold, the cell must either eliminate the pathological species or be damaged (Morimoto, 2011). However, SGs and other RNA granules exhibit reversible protein aggregation as part of a pathway that has important physiological functions (Vanderweyde et al., 2012) (Patel et al., 2015) (Nott et al., 2015) (Molliex et al., 2015) (Lin et al., 2015). RBPs exhibit an inherent ability to cycle between the liquid and solid state. In the cell, RBPs aggregate to form SGs this state transition, and disperse once the stress is gone. Although isolated RBPs can cycle between states, the process is much likely more highly regulated, and the cell contains a large number of biochemical pathways that can either induce or disperse these granules, and each of these pathways are potential targets of drug discovery. We show that proline-directed kinases, which are known to regulate the association of tau with microtubules, also regulate the tau-mediated SG pathway. Attention to the SG pathway highlights novel approaches to regulation. For instance, drug discovery efforts built around inhibiting SG formation have been successfully used to identify novel agents that prevent aggregation of TDP-43, which might be useful for therapy of ALS (Boyd et al., 2014) (Kim et al., 2014). Translational inhibitors provide another novel mechanism for regulation. The translational inhibitors, puromycin or cycloheximide, reciprocally induce or prevent tau-mediated SG formation, and also modulate the degeneration associated with over-expression of TIA1 with tau. Puromycin and cycloheximide are admittedly toxic, but we show that kinases regulating eIF2α phosphorylation, including PKR and PERK, regulate tau-mediated SG formation. These kinases might be particularly effective for tauopathies, such as AD, because they appear to inhibit disease processes at multiple levels, including preventing toxicity associated with β-amyloid (Ohno, 2014). Thus, the role of tau in RNA granule biology highlights the potential role of reversible protein aggregation in the pathophysiology of tauopathies, and presents a corresponding wide range of new avenues for pharmacotherapy of AD and other tauopathies.

### Materials and Methods

**Mice:** *Tau*^{-/-} *mice* were generated as described by Dawson and colleagues (Dawson et al., 2001). *TIA1*^{-/-} *mice* were generated as described by Piecyk et al. (Piecyk et al., 2000).

**Cell Culture:** HT22 cells were transfected using Lipofectamine (Invitrogen), incubated 24 hours, and treated after 24 hrs (25uM salubrinal, 5µg/ml puromycin, 10 µg/ml cycloheximide). At 48 hours the cells were fixed in 4% PFA.

Primary mouse P0 hippocampal cultures were grown for 21 days in Neurobasal medium supplemented with B-27 (Invitrogen).

**AAV Transduction:** At DIV2 neurons were transduced with AAV9 vectors at MOI 20 (mRFP, TIA1 shTIA1-GFP or shControl-GFP). At DIV7 neurons were transduced with AAV9-WT or P301L tau virus (MOI 20).

**Immunocytochemistry** was performed as described previously (Vanderweyde et al., 2012). Primary antibodies used were: Tau: CP-13, PHF-1, MC1 (1:150 each), and Tau13 (1:5,000). SGs: TIA1 (1:400, Santa Cruz). Neuronal marker: MAP2 (1:1000, Aves).

**Imaging:** Confocal microscopy was performed using a Carl Zeiss LSM 510.

**Image Analysis:** SG density and dendritic processes were quantified using Image J (using ImageJ plug-ins NeuronJ, tracing MAP2 positive processes for dendritic measurements). Granule movement and formation was quantified using Bitplane Imaris Track software (Imaris). **Photo-Conversion:** Photo-convertable WT human tau (PC-Tau) was generated by sub-cloning human 4N0R tau into the pPS-CFP2-C mammalian expression vector (Evrogen cat# FP801). Primary cortical cultures (E16) were transfected at DIV5 and aged to DIV18-23 prior to photo-conversion using a diode 405nm laser on a Zeiss LSM-710 Duo Scan microscope.

**Biochemical Fractionation:** HT22 cells were lysed in RIPA buffer with 1x Halt protease inhibitor cocktail (Thermo Scientific), 1x phosphatase inhibitor cocktail (PhosSTOP, Roche), and 1% Triton-X-100, sonicated and spun down to collect the supernatant. Lysates were first centrifuged for 1 hour at 100,000×g at 4°C, and the supernatants were collected as 1% Triton-X RIPA buffer soluble proteins the pellets re-sonicated and re-suspended in urea buffer.

**Sarkosyl Insoluble and Soluble Tau Fractions:** Supernatant (100 µl with 100 µg protein) in RIPA buffer plus 1% sarkosyl detergent was rotated at room temperature for 1 hour, then centrifuged 1hr at 100,000×g spin (room temperature). The supernatant collected and the sarkosyl pellet resuspended in sample buffer containing 100 mM DTT.

**Immunoblotting** was done with 15-well 4-12% Bis-Tris gels (Invitrogen), 1 hr blocking in 5% milk, 4°C overnight antibody incubation (1:7,500 Tau13, 1:500 PHF1, 1:500 TIA1 (Santa Cruz), 1:500, synaptophysin (Santa Cruz), 1:1,000 PSD-95 (NeuroMab), 1:1,000 caspase-3 or cleaved caspase 3 (Cell Signaling), or 1:10,000 actin (Millipore) in PBS. Secondary antibodies (Jackson) was incubated in 5% milk for 1 hr at RT. Developing was done using SuperSignal West Pico Substrate (Thermo).

**Immunoprecipitation:** Lysates (100-300 µg) were pre-cleared with rec-Protein G-Sepharose 4B Conjugate beads (Invitrogen), then IPs done using ON 4°C with 0.5 µl PHF-1 antibody, 1 µl Tau-5 antibody (Abcam), 1µL MC1 antibody, or 0.5µL of TIA1 antibody (Santa Cruz) or 1:200 HA antibody (Covance), followed by addition of 50 µl protein G rec-Protein G-Sepharose 4B Conjugate beads and incubation for 1 hour at 4°C. The beads were spun down and washed, boiled in SDS-sample buffer and blotted. See the supplemental methods for details on the IP methods used for the proteomic studies.

**Measurement of Protein Synthesis:** The procedure followed the SUnSET protocol in which each group of cells were treated with puromycin for 30 min and immunoblotted with the 12D10 antibody (Schmidt et al., 2009).

**Proteomics:** Quantitative proteomic analysis was performed using the total ion current (TIC) for proteins identified by LC-MS/MS normalized to the TIC level of TIA1 detected in each sample. The 163 proteins identified as unique to the WT and tau KO conditions were submitted to the DAVID Functional Clustering tool (Huang et al, 2008; Huang et al, 2009). Twelve resulting clusters with enrichment FDR < 0.05 were identified, and each of the 163 proteins was associated with the cluster(s) based on its membership in the clustered gene sets. A network was induced between proteins by counting the number of clusters shared between pairs of proteins, and visualized in the program Gephi 0.8.2.

**Cell Death studies:** Caspase 3/7 cleavage was quantified with the Caspase-Glo 3/7 Assay kit, Promega. Apoptosis detected DNA fragmentation (TiterTACS Colorimetric Apoptosis Detection kit, Trevigen).

### Supplemental Materials and Methods

*Tau*^{*-*/}*⁻ mice:* These tau knockout mice were originally generated as described by Dawson and colleagues (Dawson et al., 2001). Briefly, they created tau deficient mice by disrupting the MAPT gene. The engineered mice do not express the tau protein, appear physically normal and are able to reproduce. Embryonic hippocampal cultures from tau deficient mice show a significant delay in maturation as measured by axonal and neuritic extensions (Dawson et al., 2001). Both Tau-/- mice and control strain (B57B1/6) breeders were obtained from Jackson Laboratory and bred in the Boston University Laboratory Animal Science Center.

*TIA1*^{*-*/}*⁻ mice:* These TIA1 knockout mice were originally generated as described by Piecyk et al. (Piecyk et al., 2000). These mice are viable, though they develop mild arthritis and are more susceptible to endotoxin shock. These mice also exhibit altered polysome disassembly. Primary neurons from these mice were obtained in Paul Anderson's lab at Brigham and Women's Hospital, Boston, MA.

HT22 Culture and Transfection: HT22 cells were maintained in DMEM (4.5g glucose/L) media supplemented with 10% fetal bovine serum, 1x PenStrep (Invitrogen), 1x L-Glutamine (Invitrogen), and 1x non-essential amino acids. Cells were passaged twice a week 1:5. Cells were seeded on 18mm poly-D-lysine coated coverslips in 12-well plates (75,000/coverslip) one day prior to transfection in serum-free media (DMEM, 10% FBS). Cells were transfected at 1:2 ratio DNA:Lipofectamine (Invitrogen), delivering 2 µg total DNA per well. Lipofectamine in OptiMEM (Invitrogen) is incubated for 5 min prior to the addition of the DNA/OptiMEM. The transfection mixture is then allowed to incubate for 20 min at room temperature prior to being added to the cells. 500µL of media is removed and 500 µL of transfection mixture is added dropwise to each well. Cells were incubated 24 hours, after which the addition of 25µM salubrinal may be added to half of the plates and allowed to incubate for 24 hours. At 48 hours post-transfection, the cells were fixed in 4% PFA and stored in PBS.

shRNA Transfection: Transfection was done as stated above using Mission shRNA against TIA1 3'-UTR Clone 301302 (Sigma) or a scrambled shRNA control ± EGFP, WT Tau-EGFP, or P301L Tau-EGFP. Cells were either fixed in 4% PFA and stored in PBS or lysed in RIPA buffer for immunoblotting.

Primary Neuronal Culture: Primary hippocampal cultures were generated from P0 pups from C57B1/6 WT, TIA1 -/-, or tau -/- mice. The hippocampus was dissected out in Hank's Balanced Salt Solution buffered with HEPES and PenStrep, and dissociated with 0.125% trypsin for 15 min at 37°C, followed by trituration. Dissociated cells were plated on plates of coverslips pre-coated with Poly-D lysine (100µg/mL, Sigma). Neurons were grown for 21 days in Neurobasal medium supplemented with B-27 (Invitrogen)

Overexpression Transduction: At DIV3 neurons were transduced with AAV9-TIA1-mRFP or AAV9-mRFP virus (MOI 20). At DIV7 neurons were transduced with AAV1-WT tau-V5 or AAV1-P301L tau-V5 virus (MOI 20). Neurons were fixed for immunolabeling and confocal microscopy in 4% PFA, or lysed in via sonication in PBS with protease and phosphatase inhibitors.

Knockdown Transduction: At DIV3 neurons were transduced with AAV9-shTIA1-GFP or AAV9-shControl-GFP virus (MOI 20). At DIV7 neurons were transduced with AAV1-WT tau-V5 or AAV1-P301L tau-V5 virus (MOI 20). Neurons were fixed for immunolabeling and confocal microscopy in 4% PFA, or lysed in via sonication in PBS with protease and phosphatase inhibitors.

Tau kinase Inhibitor study: Human SH-SY5Y neuroblastoma cells were maintained in 50:50 DMEM/F-12 media supplemented with 10% FBS and 1% each of non-essential amino acids, L-glutamine, and penicillin/streptomycin. Cells were plated on 18 mm glass coverslips pre-coated with Poly-D Lysine and transfected 24 h later using Lipofectamine 2000 transfection reagent according to manufacturer's instructions (Life Technologies). 24 h after transfection, cells were treated with 10 µM Salubrinal (Santa Cruz Biotech, Cat#202332) or DMSO plus one of the following small molecule tau kinase inhibitors for 24 h: 5 µM GSK3β Inhibitor XXVI (EMD Millipore, Cat#361569), 5 µM Cdk 2/5 (EMD Millipore, Cat#219448), 250 nM p38 MAPK (Invitrogen, SB203580), 20 µM MARK/Par-1 (EMD Millipore, 39621), or 20 nM Fyn (Invitrogen, PP2, Cat#PHZ1223). Cells were fixed for 12 min in 4% paraformaldehyde and stored at 4°C in PBS. TIA1 and phosphorylated tau inclusions were labeled and analyzed as described in methods section for Immunocytochemistry (see below).

Cell Death studies: Caspase 3/7 cleavage was quantified with the Caspase-Glo 3/7 Assay kit, Promega. Apoptosis was analyzed by quantifying DNA fragmentation (TiterTACS Colorimetric Apoptosis Detection kit, Trevigen).

Immunocytochemistry: Coverslips were washed 3×5 min in PBS and washed with 0.1% Triton-X100 for 15 minutes. Coverslips were washed 3x 3minutes in PBS and blocked for 1 hr with 10% donkey serum in PBS. Incubation was performed with primary antibodies for tau and TIA1 overnight in 5% donkey serum/PBS. Primary antibodies used were used as follows: for tau: CP-13, PHF-1, MC1 (1:150, generously provided by Peter Davies, Albert Einstein College of Medicine), and Tau13 (1:5,000, generously provided by Skip Binder, Northwestern). For stress granules: TIA1 (1:400, Santa Cruz). For neuronal marker: MAP2 (1:1000, Avis).

### Immunohistochemistry:

### Microscopy

*Confocal Microscopy:* Microscopy was performed using a Carl Zeiss LSM 510 META confocal laser scanning microscope, carrying lasers at 405, 488, 543 and 633 nm. Images were captured using a 63X oil objective. LSM proprietary software was used for digital image analysis. Images were combined into figures using Adobe Photoshop software.

***Basic Microscopy:*** Quantitative and area analysis was done using Zeiss Axio Observer Z1 equipped with LED fluorescent illumination, digital camera (AxioCam MRm; Zeiss) and analyzed with Zeiss Axiovision software.

***Live Cell Microscopy:*** Live cell imaging was performed using an Olympus DSU spinning disk confocal capable carrying lasers at 405, 488, 543, and 633 nm, or a Zeiss Axio Observer Z1. Time course images were captured using a 63X oil objective, at 30s intervals up to 6 hours. Analysis was done using Imaris/Bitplane software.

***Photo-Conversion:*** Photo-convertible WT human tau (PA-Tau) was generated by sub-cloning human 0N4R tau into the pPS-CFP2-C mammalian expression vector (Evrogen cat# FP801). This expression vector encodes a cyan-to-green fluorescent protein under control of the CMV promoter, and has been optimized for high expression in mammalian cells. Primary cortical cultures were generated from E16 embryos from C57B1/6 WT mice, and cultured on MatTek glass-bottom dishes suitable for live-cell imaging. Neurons were transfected at DIV5 with PA-WT ± RFP-TIA1 or mCherry, using Lipofectamine-2000 (Invitrogen, per manufacturer's instructions), and aged to DIV18-23 prior to photo-conversion. Activation was optimized and performed using a diode 405nm laser on a Zeiss LSM-710 Duo Scan at 63X magnification, for efficient photo-conversion of tau with minimal photo-bleaching. Photo-conversion was performed using the 405nm laser powered at 20%. The samples were scanned first to make sure that no photo-conversion had occurred prior to laser treatment. To prevent photo-conversion during preliminary imaging, the laser was set with the 405nm laser powered at 9%, and imaged using a gain of 7-800. Next, the photo-conversion was accomplished with the 405nm laser powered at 20%, and samples scanned with 20 iterations of treatment, after which the samples were imaged again to confirm photo-conversion.

Following laser-activation, neurons were imaged on a Zeiss Axio Observer Z1, at 63X magnification, at 20min intervals over 6 hours. Quantification of tau fluorescence at the neuron soma was performed using ImageJ, by manually outlining the neuronal soma at Time=0 and analyzing fluorescence intensity within that ROI at each subsequent time-point. All values are expressed as the percentage of initial fluorescence intensity for a given neuron (ie: Time=0 was set to 100% independently, for each neuron).

***Image Analysis:** Granule count cells:* SG density was quantified using Image J software by calculating the number of puncta >1 mm² per 63x frame controlled for the number of cells. This was done by thresholding images, and using the analyze particle functionality. Quantifications of granule counts and area in primary neurons was done in ImageJ by using a free-hand tool to outline the neuron, thresholding the image, creating a mask over the nucleus, and using the particle analyzer tool.

*Colocalization:* To analyze the potential interaction between tau inclusions and stress granules, quantification of co-localization was done using the Carl Zeiss LSM software, and R-values for the degree of co-localization were recorded.

Granule count neurons: Quantifications of granule counts and area in primary neurons was done in ImageJ by using a free-hand tool to outline the neuron, thresholding the image, creating a mask over the nucleus, and using the particle analyzer tool.

*Dendrite length*: Measurements of dendritic processes were done using ImageJ plug-ins NeuronJ. Using the software to trace MAP2 positive processes of individual cells yields a pixel measurement that can then be back calculated to micrometers using the microscope scaling parameters.

*Live cell granule trafficking*: Measurements and analysis were done using Imaris Track on the Imaris/Bitplane software. Quantification of co-localization was done using the Carl Zeiss LSM software, and R-values for the degree of co-localization were recorded. Measurements of dendritic processes were done using ImageJ plug-ins NeuronJ, tracing MAP2 positive processes.

### Biochemical Fractionation

***Triton-X Fraction:*** HT22 cells were lysed in RIPA buffer (50 mM Tris-HCl, pH 8, 150 mM NaCl, 1% NP-40, 0.1% SDS, 0.5 mM sodium deoxycholate) with 1x Halt protease inhibitor cocktail (Thermo Scientific), 1x phosphatase inhibitor cocktail (PhosSTOP, Roche), and 1% Triton-X-100, and sonicated and spun down to collect the supernatant. Lysates were first centrifuged for 1 hour at 100,000×g 4°C, and the supernatants were collected as 1% Triton-X RIPA buffer soluble proteins. To prevent contamination caused by carrying over, the pellets were re-sonicated and re-centrifuged at 100,000×g for 30 min at 4°C. RIPA buffer-insoluble pellets were resuspended in sample buffer containing 100mM DTT. Soluble and insoluble proteins were analyzed by western blot.

***Sarkosyl Insoluble and Soluble Tau Fraction:*** To produce a fraction enriched for tau, 10 µL of a 10% solution of sarkosyl detergent was added to 100 µg of supernatant. Additional RIPA was used to produce a final volume of 100 µL. This sample was rotated at room temperature for 1 hour prior to a 1-hour centrifugation at 100,000×g spin at room temperature. The supernatant was collected in a separate tube and the sarkosyl pellet was resuspended in sample buffer containing 100 mM DTT.

Immunoblotting: Immunoblots were preformed using gradient PAGE on a 15-well 4-12% Bis-Tris gel (Invitrogen). Blocking was done in 5% milk for 1hr at RT. Primary antibody incubation was at 4°C overnight with 1:7,500 mouse monoclonal Tau13, 1:500 mouse monoclonal PHF1, 1:500 goat polyclonal TIA1 (Santa Cruz), 1:500 mouse monoclonal synaptophysin (Santa Cruz), 1:1,000 mouse monoclonal PSD-95 (NeuroMab), 1:1,000 rabbit polyclonal caspase-3 or cleaved caspase 3 (Cell Signaling), or 1:10,000 mouse monoclonal actin (Millipore) in PBS. Secondary antibody was incubated in 5% milk for 1 hr at RT. Developing was done using SuperSignal West Pico Substrate (Thermo).

Immunoprecipitation: Equal amounts of lysate were pre-cleared by rec-Protein G-Sepharose 4B Conjugate beads (Invitrogen) for 1 hour at 4°C. Samples were spun down and lysates were removed and 0.5 µl of PHF-1 antibody, 1 µl of Tau-5 antibody (Abcam), 1µL of MC1 antibody, or 0.5µL of TIA1 antibody (Santa Cruz) was added to each cell lysate (100-300 µg) and the samples were incubated overnight at 4°C on a rotating wheel. 50 µl of protein G rec-Protein G-Sepharose 4B Conjugate beads was added to the samples, then the samples were incubated for additional 1 hour at 4°C. The beads were spun down and washed three times in co-immunoprecipitation buffer. The beads were boiled at 95°C for 5 min in SDS-sample buffer. Proteins were then analyzed by western blot.

Immunoprecipitation with Fractionation: Equal amounts of lysate were pre-cleared by rec-Protein G-Sepharose 4B Conjugate beads (Invitrogen) for 1 hour at 4°C. Samples were spun down and lysates were removed and 0.5µL of TIA1 antibody (Santa Cruz) was added to each cell lysate (1000 µg) and the samples were incubated overnight at 4°C on a rotating wheel. 50 µl of protein G rec-Protein G-Sepharose 4B Conjugate beads was added to the samples, then the samples were incubated for additional 1 hour at 4°C. The beads were spun down and the non-bead bound portion was removed and sarkosyl stock buffer was added to form 1% and sonicated and centrifuged at 55,000g for 1 hour, the supernatant was taken and designated non-TIA1-bound soluble, and the pellet was washed once in RIPA buffer and centrifuged at 55,000g for 15 minutes. The pellet was resuspended in sample buffer with 100mM DTT and designated non-TIA1-bound insoluble. Concomitantly, the sepharose beads were washed three times in co-immunoprecipitation buffer. The beads were then rotated for 1 hour in 1% sarkosyl buffer and then spun down. The supernatant was taken as the TIA1-bound soluble fraction. The beads were boiled at 95°C for 5 min in SDS-sample buffer and this was designated the TIA1-bound insoluble fraction. Proteins were then analyzed by western blot.

Immunoprecipitation for Proteomics: 10 mo TIA1^{-/-}, Tau^{-/-}, and WT C57BL/6J (the genetic background) male mice were anesthetized in an isoflurane chamber and perfused with ice cold PBS. The brains were then dissected to separate the hindbrain, hippocampus, and cerebral cortex. Tissues were slowly frozen by submersion in methanol on dry ice and homogenized in RIPA lysis buffer (50 mM Tris-HCl, 150 mM NaCl, 1% Triton X-100, 0.1% SDS, 0.5% sodium deoxycholate, pH 6.8) supplemented with protease (Roche Cat#04693159001) and phosphatase inhibitor (Roche Cat#04906837001) cocktails. TIA-1 was immunoprecipitated from 2 mg cortex lysate using 10 µg goat anti-TIA-1 polyclonal antibody (Santa Cruz Biotech, Cat#sc-1751) immobilized on Pierce Direct IP columns according to manufacturer's instructions (Thermo Scientific, Cat#26148). The TIA-1 IP eluates were then separated on a Novex 4-12% Bis-Tris polyacrylamide gel (Life Technologies, Cat#NP0323) and stained with Simply Blue Coomassie G-250 SafeStain (Life Technologies, Cat#LC6060). Whole gel lanes were then excised and shipped to the UMass Mass Spectrometry and Proteomics facility for analysis by LC-MS/MS.

Proteomics, In Gel Digestion: Gel slices were cut into 1x1 mm pieces and placed in 1.5ml eppendorf tubes with 1ml of water for 30 min. The water was removed and 50ul of 250 mM ammonium bicarbonate was added. For reduction 20 µl of a 45 mM solution of 1, 4 dithiothreitol (DTT) was added and the samples were incubated at 50 C for 30 min. The samples were cooled to room temperature and then for alkylation 20 µl of a 100 mM iodoacetamide solution was added and allowed to react for 30 min. The gel slices were washed 2 X with 1 ml water aliquots. The water was removed and 1ml of 50:50 (50mM Ammonium Bicarbonate: Acetonitrile) was placed in each tube and samples were incubated at room temperature for 1hr. The solution was then removed and 200 ul of acetonitrile was added to each tube at which point the gels slices turned opaque white. The acetonitrile was removed and gel slices were further dried in a Speed Vac. Gel slices were rehydrated in 75 µl of 2ng/µl trypsin (Sigma) in 0.01% ProteaseMAX Surfactant (Promega): 50mM Ammonium Bicarbonate. Additional bicarbonate buffer was added to ensure complete submersion of the gel slices. Samples were incubated at 37C for 21hrs. The supernatant of each sample was then removed and placed in a separate 1.5 ml eppendorf tube. Gel slices were further dehydrated with 100 ul of 80:20 (Acetonitrile: 1% formic acid). The extract was combined with the supernatants of each sample. The samples were then dried down in a Speed Vac. Samples were dissolved in 25 µl of 5% Acetonitrile in 0.1% trifluroacetic acid prior to injection on LC/MS/MS.

LC/MS/MS on Q Exactive: A 3.0 µl aliquot was directly injected onto a custom packed 2cm x 100µm C18 Magic 5µm particle trap column. Labeled peptides were then eluted and sprayed from a custom packed emitter (75µm x 25cm C18 Magic 3µm particle) with a linear gradient from 95% solvent A (0.1% formic acid in water) to 35% solvent B (0.1% formic acid in Acetonitrile) in 90 minutes at a flow rate of 300 nanoliters per minute on a Waters Nano Acquity UPLC system. Data dependent acquisitions were performed on a Q Exactive mass spectrometer (Thermo Scientific) according to an experiment where full MS scans from 300-1750 m/z were acquired at a resolution of 70,000 followed by 10 MS/MS scans acquired under HCD fragmentation at a resolution of 17,500 with an isolation width of 1.6 Da. Raw data files were processed with Proteome Discoverer (version 1.4) prior to searching with Mascot Server (version 2.5) against the Uniprot database. Search parameters utilized were fully tryptic with 2 missed cleavages, parent mass tolerances of 10 ppm and fragment mass tolerances of 0.05 Da. A fixed modification of carbamidomethyl cysteine and variable modifications of acetyl (protein N-term), pyro glutamic for N-term glutamine, oxidation of methionine were considered. Search results were loaded into the Scaffold Viewer (Proteome Software, Inc.).

Proteomic Analysis: Quantitative proteomic analysis was performed using the total ion current (TIC) for proteins identified by LC-MS/MS normalized to the TIC level of TIA-1 detected in each sample. Proteins identified in the TIA-1 KO cortex tissue were considered as nonspecific binding proteins to the IP antibody and excluded from all analyses. The complete gene list of the proteins identified in each sample were then uploaded into the Database for Annotation, Visualization and Integrated Discovery (DAVID) resource available via the NIH website. Gene lists were analyzed using the KEYWORDS functional categories, GO gene ontology, INTERPRO protein domain, and KEGG pathways programs to identify gene categories that were enriched in each dataset. Categories with Benjamini corrected p values <0.05 were considered to be statistically significant. P values of statistically significant categories were then compared between samples to determine the fold enrichment for each category.

DAVID and network visualization analysis: The 163 proteins identified as unique to the WT and tau KO conditions were submitted to the DAVID Functional Clustering tool (Huang et al, 2008; Huang et al, 2009). Twelve resulting clusters with enrichment FDR < 0.05 were identified, and each of the 160 (163) proteins was associated to the cluster(s) based on its membership in the clustered gene sets. A network was created by adding a connection between protein pairs sharing annotation clusters. Edge weights were determined as the number of shared annotation clusters between protein pairs with thicker edges representing stronger functional associations between proteins (the smallest number of clusters shared between any two proteins was 1, and the largest was 8). The resulting network was visualized using the software Gephi 0.8.2 and arranged using the Force Atlas 2 layout algorithm. The network was generated using the python programming language (Python Software Foundation), and the networkx (Hagberg, 2008), numpy, and pandas python packages (Jones, 2001).

Statistical Analyses: Experiments with >2 groups are analyzed using ANOVAs with Tukeys post-hoc testing; the sample sizes are presented with each experiment. For studies of granule movement, a Mann-Whitney unpaired test with one-tailed p value was completed for comparing distances traveled, velocity analysis, and the directional percentages of granules within populations.

### Example 2: Reduction of the RNA binding protein TIA1 protects against tauopathy independent of tau aggregation

### Abstract

RNA binding proteins (RBPs) control the localization and utilization of transcripts by regulated aggregation, forming RNA granules that mediate mRNA transport, translation, and the stress response. RBP aggregation is a type of pathology in tauopathies such as Alzheimer's disease, and the interaction of tau with the T cell intracellular antigen 1 (TIA1) RNA granules promotes tau mediated neurodegeneration in primary hippocampal neurons. However, the role of RNA granules in the progression of tauopathies was unknown. This Example shows that reducing TIA1 *in vivo* alleviates neurodegeneration and prolongs survival in the PS19 mouse model of tauopathy. The protection associated with TIA1 reduction included increased binding of tau to microtubules, microtubule stabilization, improved spatial working memory, and reduced synaptic and neuronal loss. Remarkably, this protection occurred despite increased tau phosphorylation and neurofibrillary tangles. These findings suggest that tau aggregates via multiple pathways in tauopathies. Importantly, inhibition of RBP aggregation by TIA1 reduction elicits protection against an aggregation pathway with unique degenerative properties.

### Introduction

Misfolding and aggregation of microtubule associated protein tau (MAPT, or tau) is a defining pathological hallmark of tauopathies such as Alzheimer's disease (AD) and frontotemporal dementia (FTD). Tau is normally located in axons where it functions to bind and stabilize microtubules. In tauopathies, tau is missorted to the somatodendritic compartment where it misfolds and aggregates into insoluble neurofibrillary tangles (NFTs). Recent studies indicate that tau interacts with RNA binding proteins (RBPs) and regulates the translational stress response, in part by promoting formation of RNA-protein complexes, termed stress granules. However, the role of RBPs and stress granules in the progression of tauopathies is unknown.

RBPs are gaining increasing attention in the field of neurodegenerative disease due to their frequent involvement in disease pathology and genetics. The structure of RBPs is particularly striking because these proteins contain glycine-rich, low complexity (i.e. 'prion-like') domains that mediate reversible phase transition between soluble and liquid-droplet states. Self-aggregation of these low-complexity domains promotes regulated aggregation of RBPs and client mRNA transcripts to form RNA granules. While RBPs predominantly function in the nucleus to mediate RNA transcription, splicing, and maturation, their regulated aggregation into RNA granules also allows them to mediate diverse cytoplasmic functions including nuclear-cytoplasmic shuttling, mRNA transport, translational initiation/repression, and the translational stress response. Cytoplasmic RNA granules are especially important and abundant in neurons, which require extensive transport and local regulation of specialized mRNA in dendritic spines and axon terminals. Aggregation mediated by low complexity domains in RBPs appears to be important for their involvement in disease. Mutations in TDP-43, FUS, hnRNPA1/B2, TAF15, EWSR1, ATXN2, and other RBPs cause Amyotrophic Lateral Sclerosis (ALS), FTD, or Spinocerebellar Ataxia, while mutations in TIA1 and VCP (a protein that disaggregates stress granules) cause myopathies. Many of these disease-linked RBPs also represent the primary pathological aggregates in each of these diseases (i.e. TDP-43, FUS, TIA1). The aggregation process can be directly linked to the biochemical behavior of these RBPs because disease-linked mutations in FUS and hnRNP A1 are able to accelerate the phase transition of recombinant RBPs from soluble to liquid-droplet states, and can directly promote the irreversible transition from liquid-droplet to insoluble fibrils. Thus, dysfunction of RBPs appears to greatly influence the development of neurodegenerative disease.

Aggregation of RBPs is a pathological feature in tauopathies. RBPs, including the nucleating stress granule protein T cell intracellular antigen 1 (TIA1), co-localize with NFTs in patient tissues and progressively accumulate with aggregated tau in mouse models of disease (Tg4510, PS19). Importantly, the association of tau with stress granules containing TIA1 promotes tau misfolding and toxicity in cultured hippocampal neurons, while TIA1 knockdown or knockout inhibits tau misfolding and toxicity. This Example investigates whether reduction in endogenous TIA1 also protects against the progression of tauopathy in vivo, and demonstrates that reducing TIA1 is strongly neuroprotective despite a surprising age-related increase in tau aggregation.

### Results

### Heterozygous knockout of Tia1 reduces TIA1 expression in PS19 tauopathy mice

In order to investigate the role of endogenous TIA1 in the progression of tauopathy in vivo, the PS 19 transgenic mouse model of tauopathy was used. PS 19 mice overexpress human 1N4R tau with the P301S mutation associated with FTD under the control of the mouse prion promoter, which produces an age-dependent progressive tauopathy characterized by NFTs, neuronal loss, and premature mortality. PS19 mice were bred with Tial-/- or background strain (C57BL/6J) mice to generate tauopathy mice that express either 1 or 2 copies of the endogenous Tial allele, respectively, on the same mixed genetic background. To confirm reduction in endogenous TIA1 protein levels, PS19 mice were aged to 3 months and TIA1 protein expression was quantified by Western blot analysis. As expected, heterozygous knockout of Tial reduced TIA1 protein expression by more than 50% in PS19 mice brain tissue (data not shown). Further, the number of cytoplasmic TIA1 granules was reduced in PS19 with heterozygous deletion of Tial (data not shown). For the purposes of this Example, PS 19 mice with heterozygous or no deletion in the endogenous Tial gene are referred to as P301S TIA1+/- and P301S TIA1+/+, respectively.

### TIA1 reduction rescues synaptic and neuronal loss in PS19 mice

TIA1 reduction protects against tau toxicity in primary cultured hippocampal neurons. Thus, it was first investigated whether TIA1 reduction also impacted synaptic and neuronal degeneration in vivo. PS 19 mice exhibit progressive neuronal loss between 8 and 12 months of age that is preceded by presynaptic degeneration. This study focused on the CA3 region of the hippocampus (amongst others) due to the high density of both axon terminals and neuronal cell bodies, and because synaptic and neuronal loss in PS 19 mice is well characterized in this region. Compared to non-transgenic (WT Tau) mice, 6 month old P301S TIA1+/+ mice exhibited reduced levels of synaptophysin (SYP), a presynaptic terminal protein; this reduction was prevented in P301S TIA1+/- mice (Figs. 13A-13B). Total neuron numbers in the hippocampus (CA3) and lateral entorhinal cortex (LEnt) of P301S TIA1+/+ and P301S TIA1+/- mice were then quantified. The number of Nissl-positive neuronal cell bodies and NeuN (neuron specific nuclear protein)-positive nuclei were increased in P301S TIA1+/- mice compared to P301S TIA1+/+ mice in both CA3 and LEnt at 9 months of age (Figs. 13C-13D). The effect of TIA1 reduction on gross cortical atrophy was then assessed by measuring the length between the pial surface and the base of Layer II/III in the cortex of 9 month P301S TIA1+/+ and P301S TIA1+/- mice. P301S TIA1+/+ mice exhibited almost a 2-fold reduction in Layer II/III thickness, while P301S TIA1+/- mice were not significantly different from non-transgenic mice (Fig. 13E). Thus, the data suggests that TIA1 reduction protects against tau mediated neurodegeneration in vivo.

### TIA1 reduction promotes tau directed microtubule stabilization

To investigate whether neuroprotection associated with TIA1 reduction promoted normal tau functions, it was investigated whether TIA1 reduction improved microtubule (MT) stabilization. MT-bound (pellet, P) and unbound (supernatant, S) fractions were separated by centrifugation in RAB buffer, as described previously. As expected, tau progressively accumulated in the MT-unbound (S) fraction of P301S TIA1+/+ mice between 6 and 9 months of age, indicating detachment of tau from MTs (Fig. 14A). However, significantly less tau is detected in the MT-unbound (S) fraction of P301S TIA1+/- mice, resulting in a 3-fold increase in the ratio of MT-bound (P) to unbound (S) tau at 9 months of age (Figs. 14A-14B). Further, TIA1 reduction increased the ratio of acetylated to total α-tubulin (Fig. 14C), suggesting that the increased binding of tau to MTs also promotes MT stabilization.

### TIA1 reduction alleviates cognitive impairment and extends lifespan in PS19 mice

It was next determines whether TIA1 reduction improves the behavioral phenotype of PS19 mice. PS19 mice exhibit cognitive impairment in the Y maze spontaneous alternation task, as well as hyperactivity/anxiolysis in the Open Field (OF) and Elevated Plus Maze (EPM) tasks. In the Y maze, P301S TIA1+/+ mice exhibited reduced spontaneous alternation compared to non-transgenic mice (Fig. 14D), indicating an impairment in spatial working memory. Remarkably, TIA1 reduction rescued the performance of P301S TIA1+/- mice to normal working memory levels (Fig. 14D); TIA1 reduction had no effect in non-transgenic mice (Fig. 14D). Importantly, P301S TIA1+/- mice did not differ in their locomotor activity in the Y maze or OF tasks, excluding the possibility that their improved performance could be attributed to altered locomotor behavior (data not shown). Thus, TIA1 reduction ameliorates cognitive impairment in PS19 mice, while not altering the locomotor phenotype.

PS19 mice die prematurely due to motor ataxia and hindlimb paralysis, with a previously reported median survival of 9 months and 90% mortality by 12 months of age. The PS19 mice crossed to a C57BL/6J background (P301S TIA1+/+) recapitulate this lifespan phenotype (Fig. 14E). However, P301S TIA1+/- mice live 2.3 months longer on average, with 47% surviving to 12 months of age without motor symptoms (Fig. 14E). Thus, TIA1 reduction extends lifespan in PS 19 mice.

### TIA1 reduction elicits a biphasic change in tau phosphorylation

Aggregated tau in AD and FTD is abnormally hyperphosphorylated at a variety of disease-specific phospho-epitopes. Thus, the age-dependent accumulation of phosphorylated tau was compared in P301S TIA1+/+ and P301S TIA1+/- mice by immunohistochemistry (IHC). As expected, P301S TIA1+/+ mice accumulated detectable levels of CP13 (S202)- and AT8 (S202/T205)-phosphorylated tau in the hippocampus by 3 months of age (Fig. 15A). Little to no phosphorylated tau was observed in the cortex or cerebellum of P301S TIA1+/+ mice at 3 months of age, consistent with the expected progression of disease in PS19 mice. In contrast, P301S TIA1+/- mice exhibited reduced levels of CP13 and AT8 phosphorylated tau in the hippocampus (CA1, CA3) compared to P301S TIA1+/+ mice (Fig. 15A), suggesting that TIA1 reduction delays the onset of pathology in PS 19 mice.

It was then determined how TIA1 reduction affected tau phosphorylation at later stages of disease by measuring the levels of CP13- and PHF1-phosphorylated tau at 6 and 9 months of age. PHF1-positive tau selectively labels tau phosphorylated at a phospho-epitope (S396/S404) that is highly correlated with fibrillar tau aggregates. Surprisingly, TIA1 reduction led to increased CP13-positive phosphorylated tau levels at 6 and 9 months (Figs. 15A-15B), which was further confirmed by immunoblot analysis (data not shown). Further, the number of PHF1-positive neurons was increased in P301S TIA1+/- compared to P301S TIA1+/+ mice (Figs. 15D-15E). Despite the changes in phosphorylated tau, the level of total tau in P301S TIA1+/- mice was not significantly different from P301S TIA1+/+ mice (data not shown). Further, while almost 20% of PHF1-positive inclusions co-localized with cytoplasmic TIA1 in P301S TIA1+/+ LEnt cortex, less than 5% of PHF1 inclusions contained TIA1 in P301S TIA1+/- mice (Figs. 15F-15G). TIA1 was largely restricted to the nucleus in P301S TIA1+/- mice, even in PHF1-positive neurons (Fig. 15F). Thus, while TIA1 reduction initially decreased the level of phosphorylated tau pathology, P301S TIA1+/- mice accumulated greater levels of phosphorylated tau at later stages of disease.

### TIA1 reduction accelerates neurofibrillary tangle accumulation

The surprising finding that TIA1 reduction increases tau phosphorylation raised the possibility that TIA1 reduction also impacts neurofibrillary tangles (NFTs). In PS19 mice, mature NFTs can be visualized by colorimetric and fluorescent dyes that bind insoluble beta-pleated fibrils. Both the Gallyas Silver stain and Thioflavin S (ThioS) histological methods were used to analyze the NFT burden in 9 month P301S TIA1+/+ and P301S TIA1+/- mice. As expected, 9 month old P301S TIA1+/+ mice developed widespread Gallyas-positive and ThioS-positive tangles throughout the hippocampus and temporal cortex (Figs. 16A, 16B, 16C, and 16D). Interestingly, TIA1 reduction accelerated the number of Gallyas Silver-positive neurons in both the frontal (primary motor area, M1) and temporal (LEnt) cortices (Fig. 16B); TIA1 reduction also increased the level of ThioS fluorescence (Fig. 16D). The number of Gallyas Silver-positive tangles was not affected in the CA3 region of the hippocampus, perhaps reflecting the advanced level of pathology in CA3 relative to other brain areas in this model (Fig. 16B). However, despite equal number of tangles in CA3 at 9 months of age, there was no relationship between tangle burden and neuronal loss in either P301S TIA1+/+ or P301S TIA1+/- mice (r² = 0.07; Fig. 16E). Thus, the neuroprotection associated with TIA1 reduction in PS 19 mice occurs independent of NFTs, consistent with studies suggesting that NFTs are not the cause of neuronal loss in tauopathies.

### TIA1 reduction alters the biochemical and structural properties of tau aggregation

The surprising disconnect between brain pathology and disease phenotype in P301S TIA1+/- mice led to the hypothesis that TIA1 reduction altered either the structural or biochemical properties of tau aggregates, producing tau species that are less toxic. This possibility seemed especially likely given that different strains of tau aggregates exhibit distinct patterns of propagation and pathophysiology when injected into tauopathy mice. Further, biochemical fractionation studies using the Hsaio method have shown that separation of TBS-extractable (S1), sarkosyl-soluble (S3), and sarkosyl-insoluble (P3) fractions yield tau aggregates with distinct structural properties. Notably, the insoluble material (S1p) from the TBS-extractable fraction contains a 64-kD tau species that is highly correlated with the extent of neurodegeneration in rTg4510 mice. Thus, brain homogenates from the cortex of 6 and 9 month old P301S TIA1+/+ and P301S TIA1+/- mice were biochemically fractionated. As expected, tau progressively accumulated in both the S1p and P3 insoluble fractions of P301S TIA1+/+ mice (Figs. 17A-17B). Interestingly, TIA1 reduction selectively decreased the level of tau detected in the S1p fraction, while concomitantly increasing the level of tau in the P3 fraction (Figs. 17A, 17B, and 17C). Additional RBPs associated with TIA1-positive stress granules were also reduced in the S1p fraction, including PABP and DDX6, while not changed in the S3 (not shown) or P3 fractions (Fig. 17D). This result suggests that RBP aggregation mediated by TIA1 promotes formation of TBS-extractable tau aggregates, and that inhibiting this pathway shifts tau aggregation towards alternative pathways.

### DISCUSSION

Tau, which is normally an axonal protein, mislocalizes to the somatodendritic compartment in response to stress to regulate mitochondrial transport and the translational stress response. Tau interacts with various aggregation-prone RNA binding proteins as part of this process, including the stress granule core nucleating protein TIA1. Importantly, the association of tau with RNA granules stimulates tau misfolding and toxicity in primary neurons. This study shows that reducing RNA granule formation in vivo also protects against the progression of tauopathy in PS 19 mice.

The results show that reducing TIA1 improves many aspects of the disease phenotype, including reduced neuronal loss, increased binding of tau to microtubules, improved cognition, and extended lifespan. Surprisingly, this behavioral protection occurred despite an age-dependent increase in neurofibrillary tangles. This finding highlights studies suggesting that neurofibrillary tangles are not directly responsible for neurodegeneration in tauopathies. Further, this work suggests that RBP aggregation promotes the formation of toxic tau aggregates with degenerative properties unique from neurofibrillary tangles. This study demonstrates a striking dependence of these tau aggregates on TIA1 since heterozygous knockout of Tial selectively cleared this strain of tau aggregate at the expense of continued accumulation NFTs and sarkosyl-insoluble tau. These results complement tau propagation studies demonstrating that different strains of tau aggregates produce distinct patterns of tau pathophysiology and toxicity in vivo.

For decades, the majority of experimental therapeutics have targeted the clearance of insoluble aggregates from the brains of patients with neurodegenerative disorders, including tauopathies. Despite the ability of many of these compounds to effectively reduce amyloid fibrils and neurofibrillary tangles, none to date have offered a disease-modifying benefit to patients. These results highlight the need for therapeutics that target specific tau species or modulate biological pathways with particularly toxic outcomes. The findings from this study suggest that reducing RBP aggregation is a viable therapeutic strategy for the treatment of tauopathies. However, despite the striking ability of TIA1 reduction to protect against tauopathy in PS 19 mice, the relative contributions of altered tau aggregation and putative neuroprotective changes in other biological pathways remains to be determined.

### EQUIVALENTS

It will be recognized that one or more features of any embodiments disclosed herein may be combined and/or rearranged to produce further embodiments.

Those skilled in the art will recognize, or be able to ascertain using no more than routine experimentation, many equivalents to the specific embodiments of the invention described herein.

Although the invention has been described and illustrated in the foregoing illustrative embodiments, it is understood that the present disclosure has been made only by way of example, and that numerous changes in the details of implementation of the invention can be made without departing from the spirit and scope of the invention, which is limited only by the claims that follow. Features of the disclosed embodiments can be combined and/or rearranged in various ways within the scope and spirit of the invention to produce further embodiments. Those skilled in the art will recognize, or be able to ascertain, using no more than routine experimentation, numerous equivalents to the specific embodiments described specifically in this disclosure.

## Claims

1. A nucleic acid inhibitor of an mRNA encoding TIA-1 for use in treating a neurodegenerative disease and/or disorder, wherein the inhibitor reduces the level or translation of the mRNA.

2. The nucleic acid inhibitor for use as claimed in claim 1, comprising a nucleic acid sequence, which;
is capable of hybridizing with a first region of a TIA-1 nucleic acid sequence of SEQ ID NO: 1 that overlaps by at least 1, 2, 3, 4, 5, 6, 7, 8, 9, 10, 11, 12, 13, 14, 15, 16, 17, 18, 19, or 20 nucleotides with a second region comprising nucleotides 1526-1545 of SEQ ID NO: 1;
is capable of hybridizing with a first region of a TIA-1 nucleic acid sequence of SEQ ID NO: 1 that is within at least 0, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 200, 300, 400, 500, 600, 700, 800, 900, or 1000 nucleotides of a second region comprising nucleotides 1526-1545 of SEQ ID NO: 1, or;
comprises a sequence of SEQ ID NO: 4, or a sequence having no more than 1, 2, 3, 4, 5, 6, 7, 8, 9, or 10 substitutions, insertions, or deletions relative to SEQ ID NO: 4.

3. The nucleic acid inhibitor for use as claimed in claim 1, comprising a sequence of SEQ ID NO: 2, or a sequence having no more than 1, 2, 3, 4, or 5 substitutions, insertions, or deletions relative to SEQ ID NO: 2.

4. The nucleic acid inhibitor for use as claimed in claim 3, further comprising a sequence capable of hybridizing to SEQ ID NO: 2, preferably wherein the sequence capable of hybridizing to SEQ ID NO: 2 is a sequence having no more than 1, 2, 3, 4, or 5 substitutions, insertions, or deletions relative to SEQ ID NO: 3.

5. The nucleic acid inhibitor for use as claimed in any of the preceding claims, comprising a double stranded RNA (dsRNA), a siRNA, an shRNA, an antisense RNA, a microRNA (miRNA), long interfering dsRNA (liRNA), an aptamer, or a ribozyme.

6. The nucleic acid inhibitor for use as claimed in any of the preceding claims, comprising a double stranded region, preferably wherein the double stranded region is about 16, 17, 18, 19, 20, 21, 22, 23, 24, 25, 26, 27, 28, 29, or 30 base pairs in length, wherein the nucleic acid inhibitor for use comprises one or two overhanging ends of 1, 2, or 3 nucleotides, preferably wherein the nucleic acid inhibitor for use comprises one or two blunt ends.

7. The nucleic acid inhibitor for use as claimed in any of claims 1-3, which is single stranded.

8. The nucleic acid inhibitor for use as claimed in any of the preceding claims, comprising at least one chemical modification, preferably wherein the nucleic acid inhibitor for use comprises one or more chemical modifications selected from: phosphorothioate internucleotide linkages, 2'-deoxyribonucleotides, 2'-O-methyl ribonucleotides, 2'-deoxy-2'-fluoro ribonucleotides, universal base nucleotides, acyclic nucleotides, 5-C-methyl nucleotides, and terminal glyceryl and inverted deoxy abasic residues.

9. The nucleic acid inhibitor for use as claimed in any of the preceding claims, comprising one or more conjugate moieties, wherein the conjugate moiety is attached to a nucleic acid via one or more cleavable bonds, preferably a phosphodiester linkage.

10. The nucleic acid inhibitor for use as claimed in any of the preceding claims, further comprising administering a second anti-neurodegenerative agent or therapy to the subject, preferably wherein the subject is a mammal, e.g., a human.

11. The nucleic acid inhibitor for use as claimed in any of the preceding claims, wherein the neurodegenerative disorder comprises a tauopathy.

12. The nucleic acid inhibitor for use as claimed in any of claims 1 to 10, wherein the neurodegenerative disorder is selected from progressive supranuclear palsy (PSP), corticobasal degeneration (CBD), multiple system atrophy (MSA), Huntington's disease, Creutzfeld-Jacob disease, spinomuscular atrophy, and a motor neuron disease, e.g., amyotrophic lateral sclerosis (ALS).

13. The nucleic acid inhibitor for use as claimed in claim 11, wherein the tauopathy is selected from: Alzheimer's disease, frontotemporal dementias (FTD) comprising preferably Pick's disease or frontotemporal dementia with parkinsonism (FTDP-17), progressive supranuclear palsy (PSP), corticobasal degeneration (CBD), and multisystems atrophy (MSA).

14. The nucleic acid inhibitor for use as claimed in any of the preceding claims, wherein total tau levels do not decrease, preferably wherein tau levels increase, preferably wherein TIA-1 RNA granules comprise tau protein, e.g., misfolded tau protein, e.g., tau protein comprising an epitope recognized by the antibody MC1, more preferably wherein decreasing the stability or increasing the solubility of TIA-1 RNA granules.

15. A nucleic acid inhibitor which targets mRNA encoding TIA-1 for use in treating a neurodegenerative disease and/or disorder, the use comprising reducing a number or size of TIA-1 RNA granules, inhibiting TIA-1 RNA granule formation, or reducing the number of cells that are positive for TIA-1 RNA granules, compared to an untreated sample or subject, or compared to the expected course of disease without treatment and comprising contacting a cell with the nucleic acid inhibitor which targets mRNA encoding TIA-1.

16. A nucleic acid inhibitor which targets mRNA encoding TIA-1 for use in treating a neurodegenerative disease and/or disorder, the use comprising prolonging cell survival and comprising contacting the cell with the nucleic acid inhibitor which targets mRNA encoding TIA-1.

17. A nucleic acid inhibitor which targets mRNA encoding TIA-1 for use in treating a neurodegenerative disease and/or disorder, the use comprising inhibiting tau misfolding and comprising contacting a cell having misfolded tau with the nucleic acid inhibitor which targets mRNA encoding TIA-1.

18. A kit comprising a nucleic acid inhibitor for use as claimed in any of claims 1-9 and instructions for using the nucleic acid inhibitor for treating a neurodegenerative disease.

19. A vector encoding a nucleic acid inhibitor for use as claimed in any of claims 1-9, preferably wherein the vector further comprises one or more of a promoter, selectable marker, and polyadenylation site, preferably wherein the vector is a viral vector, e.g., an AAV vector, more preferably wherein the vector comprises or encodes a plurality of the nucleic acid inhibitor for use.

20. A pharmaceutical composition comprising a nucleic acid inhibitor for use as claimed in any of claims 1-9 and one or more pharmaceutically acceptable excipient.

## Patentansprüche

1. Nukleinsäureinhibitor einer TIA-1 codierenden mRNA zur Verwendung bei der Behandlung einer neurodegenerativen Krankheit und/oder Störung, wobei der Inhibitor den Spiegel oder die Translation der mRNA reduziert.

2. Nukleinsäureinhibitor zur Verwendung nach Anspruch 1, umfassend eine Nukleinsäuresequenz, die
mit einer ersten Region einer TIA-I-Nukleinsäuresequenz unter SEQ ID NO: 1 hybridisieren kann, die zu wenigstens 1, 2, 3, 4, 5, 6, 7, 8, 9, 10, 11, 12, 13, 14, 15, 16, 17, 18, 19 oder 20 Nukleotiden mit einer zweiten Region, die Nukleotide 1526-1545 von SEQ ID NO: 1 umfasst, überlappt;
mit einer ersten Region einer TIA-I-Nukleinsäuresequenz unter SEQ ID NO: 1 hybridisieren kann, die wenigstens 0, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 200, 300, 400, 500, 600, 700, 800, 900 oder 1000 Nukleotide von einer zweiten Region, die Nukleotide 1526-1545 von SEQ ID NO: 1 umfasst, entfernt ist, oder;
eine Sequenz unter SEQ ID NO: 4 oder eine Sequenz mit nicht mehr als 1, 2, 3, 4, 5, 6, 7, 8, 9 oder 10 Substitutionen, Insertionen oder Deletionen relativ zu SEQ ID NO: 4 umfasst.

3. Nukleinsäureinhibitor zur Verwendung nach Anspruch 1, umfassend eine Sequenz unter SEQ ID NO: 2 oder eine Sequenz mit nicht mehr als 1, 2, 3, 4 oder 5 Substitutionen, Insertionen oder Deletionen relativ zu SEQ ID NO: 2.

4. Nukleinsäureinhibitor zur Verwendung nach Anspruch 3, ferner umfassend eine Sequenz, die an SEQ ID NO: 2 hybridisieren kann, vorzugsweise wobei es sich bei der Sequenz, die an SEQ ID NO: 2 hybridisieren kann, um eine Sequenz mit nicht mehr als 1, 2, 3, 4 oder 5 Substitutionen, Insertionen oder Deletionen relativ zu SEQ ID NO: 3 handelt.

5. Nukleinsäureinhibitor zur Verwendung nach einem der vorhergehenden Ansprüche, umfassend eine Doppelstrang-RNA (dsRNA), eine siRNA, eine shRNA, eine Antisense-RNA, eine microRNA (miRNA), liRNA (long interfering dsRNA), ein Aptamer oder ein Ribozym.

6. Nukleinsäureinhibitor zur Verwendung nach einem der vorhergehenden Ansprüche, umfassend eine Doppelstrangregion, vorzugsweise wobei die Doppelstrangregion eine Länge von etwa 16, 17, 18, 19, 20, 21, 22, 23, 24, 25, 26, 27, 28, 29 oder 30 Basenpaaren aufweist, wobei der Nukleinsäureinhibitor zur Verwendung ein oder zwei überhängende Enden mit 1, 2 oder 3 Nukleotiden umfasst, vorzugsweise wobei der Nukleinsäureinhibitor zur Verwendung ein oder zwei stumpfe Enden umfasst.

7. Nukleinsäureinhibitor zur Verwendung nach einem der Ansprüche 1-3, der als Einzelstrang vorliegt.

8. Nukleinsäureinhibitor zur Verwendung nach einem der vorhergehenden Ansprüche, umfassend wenigstens eine chemische Modifikation, vorzugsweise wobei der Nukleinsäureinhibitor zur Verwendung eine oder mehrere chemische Modifikationen umfasst, die ausgewählt sind aus: Phosphorothioat-Internukleotidverknüpfungen, 2'-Desoxyribonukleotiden, 2'-O-Methylribonukleotiden, 2'-Desoxy-2'-fluorribonukleotiden, Universalbasennukleotiden, acyclischen Nukleotiden, 5-C-Methylnukleotiden und terminalen Glyceryl- und invertierten abasischen Desoxyresten.

9. Nukleinsäureinhibitor zur Verwendung nach einem der vorhergehenden Ansprüche, umfassend eine oder mehrere Konjugatgruppierungen, wobei die Konjugatgruppierung an eine Nukleinsäure über eine oder mehrere spaltbare Bindungen, vorzugsweise eine Phosphodiester-Verknüpfung, gebunden ist.

10. Nukleinsäureinhibitor zur Verwendung nach einem der vorhergehenden Ansprüche, ferner umfassend Verabreichen eines bzw. einer zweiten antineurodegenerativen Mittels bzw. Therapie an das Individuum, vorzugsweise wobei es sich bei dem Individuum um einen Säuger, z. B. einen Menschen, handelt.

11. Nukleinsäureinhibitor zur Verwendung nach einem der vorhergehenden Ansprüche, wobei die neurodegenerative Störung eine Tauopathie umfasst.

12. Nukleinsäureinhibitor zur Verwendung nach einem der Ansprüche 1 bis 10, wobei die neurodegenerative Störung ausgewählt ist aus progressiver supranukleärer Paralyse (PSP), corticobasaler Degeneration (CBD), multipler Systematrophie (MSA), Morbus Huntington, Creutzfeldt-Jakob-Krankheit, spinaler Muskelatrophie und einer Motoneuron-Krankheit, z. B. amyotropher Lateralsklerose (ALS).

13. Nukleinsäureinhibitor zur Verwendung nach Anspruch 11, wobei die Tauopathie ausgewählt ist aus: Morbus Alzheimer, frontotemporalen Demenzen (FTD), die bevorzugt Pick-Krankheit oder frontotemporale Demenz mit Parkinsonismus (FTDP-17) umfassen, progressiver supranukleärer Paralyse (PSP), corticobasaler Degeneration (CBD) und Multisystematrophie (MSA).

14. Nukleinsäureinhibitor zur Verwendung nach einem der vorhergehenden Ansprüche, wobei die Tau-Gesamtspiegel nicht sinken, vorzugsweise wobei Tau-Spiegel steigen, vorzugsweise wobei TIA-1-RNA-Granula Tau-Protein umfassen, z. B. fehlgefaltetes Tau-Protein, z. B. Tau-Protein, das ein vom Antikörper MC1 erkanntes Epitop umfasst, stärker bevorzugt wobei die Stabilität von TIA-1-RNA-Granula abnimmt und ihre Löslichkeit zunimmt.

15. Nukleinsäureinhibitor, der auf TIA-1 codierende mRNA zielt, zur Verwendung bei der Behandlung einer neurodegenerativen Krankheit und/oder Störung, wobei die Verwendung Verringern einer Zahl oder Größe von TIA-1-RNA-Granula, Hemmen der Ausbildung von TIA-1-RNA-Granula oder Reduzieren der Anzahl an Zellen, die positiv für TIA-1-RNA-Granula sind, im Vergleich zu einer unbehandelten Probe bzw. einem nicht behandelten Individuum oder im Vergleich zum erwarteten Verlauf der Krankheit ohne Behandlung und Inkontaktbringen einer Zelle mit dem auf TIA-1 codierende mRNA zielenden Nukleinsäureinhibitor umfasst.

16. Nukleinsäureinhibitor, der auf TIA-1 codierende mRNA zielt, zur Verwendung bei der Behandlung einer neurodegenerativen Krankheit und/oder Störung, wobei die Verwendung Verlängern des Zellüberlebens und Inkontaktbringen der Zelle mit dem auf TIA-1 codierende mRNA zielenden Nukleinsäureinhibitor umfasst.

17. Nukleinsäureinhibitor, der auf TIA-1 codierende mRNA zielt, zur Verwendung bei der Behandlung einer neurodegenerativen Krankheit und/oder Störung, wobei die Verwendung Hemmen von Tau-Fehlfaltung und Inkontaktbringen einer Zelle, die fehlgefaltetes Tau hat, mit dem auf TIA-1 codierende mRNA zielenden Nukleinsäureinhibitor umfasst.

18. Kit, umfassend einen Nukleinsäureinhibitor zur Verwendung nach einem der Ansprüche 1-9 und Anweisungen zur Verwendung des Nukleinsäureinhibitors zur Behandlung einer neurodegenerativen Krankheit.

19. Vektor, codierend einen Nukleinsäureinhibitor zur Verwendung nach einem der Ansprüche 1-9, vorzugsweise wobei der Vektor ferner ein bzw. eine oder mehrere von einem Promotor, selektierbaren Marker und Polyadenylierungsstelle umfasst, vorzugsweise wobei es sich bei dem Vektor um einen Virusvektor, z. B. einen AAV-Vektor, handelt, stärker bevorzugt wobei der Vektor mehrere des Nukleinsäureinhibitors zur Verwendung umfasst oder codiert.

20. Pharmazeutische Zusammensetzung, umfassend einen Nukleinsäureinhibitor zur Verwendung nach einem der Ansprüche 1-9 und einen oder mehrere pharmazeutisch unbedenkliche Exzipienten.

## Revendications

1. Inhibiteur d'acide nucléique d'un ARNm codant pour TIA-1 pour utilisation dans le traitement d'une maladie et/ou un trouble neurodégénératif, l'inhibiteur réduisant le taux ou la traduction de l'ARNm.

2. Inhibiteur d'acide nucléique pour utilisation selon la revendication 1, comprenant une séquence d'acide nucléique, qui ;
est capable de s'hybrider avec une première région d'une séquence d'acide nucléique TIA-1 de SEQ ID NO : 1 qui se chevauche d'au moins 1, 2, 3, 4, 5, 6, 7, 8, 9, 10, 11, 12, 13, 14, 15, 16, 17, 18, 19 ou 20 nucléotides avec une deuxième région comprenant les nucléotides 1526 à 1545 de SEQ ID NO : 1 ;
est capable de s'hybrider avec une première région d'une séquence d'acide nucléique TIA-1 de SEQ ID NO : 1 qui est à au moins 0, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 200, 300, 400, 500, 600, 700, 800, 900 ou 1000 nucléotides d'une deuxième région comprenant les nucléotides 1526 à 1545 de SEQ ID NO : 1, ou ;
comprend une séquence de SEQ ID NO : 4, ou une séquence ayant pas plus de 1, 2, 3, 4, 5, 6, 7, 8, 9 ou 10 substitutions, insertions ou délétions par rapport à SEQ ID NO : 4.

3. Inhibiteur d'acide nucléique pour utilisation selon la revendication 1, comprenant une séquence de SEQ ID NO : 2, ou une séquence ayant pas plus de 1, 2, 3, 4 ou 5 substitutions, insertions ou délétions par rapport à SEQ ID NO : 2.

4. Inhibiteur d'acide nucléique pour utilisation selon la revendication 3, comprenant en outre une séquence capable de s'hybrider à SEQ ID NO : 2, la séquence capable de s'hybrider à SEQ ID NO : 2 étant de préférence une séquence ayant pas plus de 1, 2, 3, 4 ou 5 substitutions, insertions ou délétions par rapport à SEQ ID NO : 3.

5. Inhibiteur d'acide nucléique pour utilisation selon l'une quelconque des revendications précédentes, comprenant un ARN double brin (ARNdb), un ARNsi, un ARNsh, un ARN antisens, un microARN (ARNmi), un ARNdb interfèrent long (ARNli), un aptamère ou un ribozyme.

6. Inhibiteur d'acide nucléique pour utilisation selon l'une quelconque des revendications précédentes, comprenant une région double brin, la région double brin étant de préférence d'environ 16, 17, 18, 19, 20, 21, 22, 23, 24, 25, 26, 27, 28, 29 ou 30 paires de bases de longueur, l'inhibiteur d'acide nucléique pour utilisation comprenant une ou deux extrémités saillantes de 1, 2 ou 3 nucléotides, l'inhibiteur d'acide nucléique pour utilisation comprenant de préférence une ou deux extrémités franches.

7. Inhibiteur d'acide nucléique pour utilisation selon l'une quelconque des revendications 1 à 3, qui est simple brin.

8. Inhibiteur d'acide nucléique pour utilisation selon l'une quelconque des revendications précédentes, comprenant au moins une modification chimique, l'inhibiteur d'acide nucléique pour utilisation comprenant de préférence une ou plusieurs modifications chimiques choisies parmi : des liaisons internucléosidiques phosphorothioate, des 2'-désoxyribonucléotides, des 2'-O-méthyl-ribonucléotides, des 2'-désoxy-2'-fluoro-ribonucléotides, des nucléotides de base universelle, des nucléotides acycliques, des 5-C-méthyl-nucléotides, et des résidus désoxy-abasiques à glycéryle terminal et inversés.

9. Inhibiteur d'acide nucléique pour utilisation selon l'une quelconque des revendications précédentes, comprenant un ou plusieurs fragments conjugués, dans lequel le fragment conjugué est lié à un acide nucléique par l'intermédiaire d'une ou plusieurs liaisons clivables, de préférence une liaison phosphodiester.

10. Inhibiteur d'acide nucléique pour utilisation selon l'une quelconque des revendications précédentes, comprenant en outre l'administration d'un deuxième agent ou d'une deuxième thérapie antineurodégénératif au sujet, le sujet étant de préférence un mammifère, par exemple, un humain.

11. Inhibiteur d'acide nucléique pour utilisation selon l'une quelconque des revendications précédentes, le trouble neurodégénératif comprenant une tauopathie.

12. Inhibiteur d'acide nucléique pour utilisation selon l'une quelconque des revendications 1 à 10, le trouble neurodégénératif étant choisi parmi la paralysie supranucléaire progressive (PSP), la dégénérescence corticobasale (DCB), l'atrophie multisystématisée (AMS), la maladie de Huntington, la maladie de Creutzfeld-Jacob, l'atrophie spinomusculaire et une maladie des motoneurones, par exemple, la sclérose latérale amyotrophique (SLA).

13. Inhibiteur d'acide nucléique pour utilisation selon la revendication 11, la tauopathie étant choisie parmi : la maladie d'Alzheimer, des démences frontotemporales (DFT) comprenant de préférence la maladie de Pick ou une démence frontotemporale avec parkinsonisme (FTDP-17), la paralysie supranucléaire progressive (PSP), la dégénérescence corticobasale (DCB) et l'atrophie multisystématisée (AMS).

14. Inhibiteur d'acide nucléique pour utilisation selon l'une quelconque des revendications précédentes, les taux totaux de tau ne diminuant pas, les taux de taux de préférence augmentant, des granules d'ARN TIA-1 comprenant de préférence la protéine tau, par exemple, une protéine tau mal repliée, par exemple, la protéine tau comprenant un épitope reconnu par l'anticorps MC1, plus préférablement diminuant la stabilité ou augmentant la solubilité de granules d'ARN TIA-1.

15. Inhibiteur d'acide nucléique qui cible un ARNm codant pour TIA-1 pour utilisation dans le traitement d'une maladie et/ou un trouble neurodégénératif, l'utilisation comprenant la réduction d'un nombre ou de la taille de granules d'ARN TIA-1, l'inhibition de la formation de granules d'ARN TIA-1, ou la réduction du nombre de cellules qui sont positives pour des granules d'ARN TIA-1, par rapport à un échantillon ou sujet non traité, ou par rapport à l'évolution prévue de la maladie sans traitement et comprenant la mise en contact d'une cellule avec l'inhibiteur d'acide nucléique qui cible un ARNm codant pour TIA-1.

16. Inhibiteur d'acide nucléique qui cible un ARNm codant pour TIA-1 pour utilisation dans le traitement d'une maladie et/ou un trouble neurodégénératif, l'utilisation comprenant la prolongation de la survie cellulaire et comprenant la mise en contact de la cellule avec l'inhibiteur d'acide nucléique qui cible un ARNm codant pour TIA-1.

17. Inhibiteur d'acide nucléique qui cible un ARNm codant pour TIA-1 pour utilisation dans le traitement d'une maladie et/ou un trouble neurodégénératif, l'utilisation comprenant l'inhibition d'un mauvais repliement de tau et comprenant la mise en contact d'une cellule ayant tau mal replié avec l'inhibiteur d'acide nucléique qui cible un ARNm codant pour TIA-1.

18. Kit comprenant un inhibiteur d'acide nucléique pour utilisation selon l'une quelconque des revendications 1 à 9 et des instructions pour utiliser l'inhibiteur d'acide nucléique pour traiter une maladie neurodégénérative.

19. Vecteur codant pour un inhibiteur d'acide nucléique pour utilisation selon l'une quelconque des revendications 1 à 9, le vecteur comprenant de préférence en outre l'un ou plusieurs parmi un promoteur, un marqueur sélectionnable, et un site de polyadénylation, le vecteur étant de préférence un vecteur viral, par exemple, un vecteur AAV, le vecteur comprenant ou codant de préférence pour une pluralité de l'inhibiteur d'acide nucléique pour utilisation.

20. Composition pharmaceutique comprenant un inhibiteur d'acide nucléique pour utilisation selon l'une quelconque des revendications 1 à 9 et un ou plusieurs excipients pharmaceutiquement acceptables.
